# EUROPEAN PATENT APPLICATION

(11) **EP 3 901 155 A1**
(43) Date of publication of application: **27.10.2021**
(21) Application number: 20170326.1
(22) Date of filing: 20.04.2020
(51) Int. Cl.: C07D 487/04, A61K 31/5517, A61P 25/00, A61P 43/00

(54) **NEW BENZODIAZEPINE DERIVATIVES AS GABA A GAMMA1 PAM**

(71) Applicant: F. Hoffmann-La Roche AG, 4070 Basel (CH)
(72) Inventor: The designation of the inventor has not yet been filed
(74) Representative: Bernard, Guillaume

(57) **Abstract**

The invention provides novel compounds having the general formula (I) 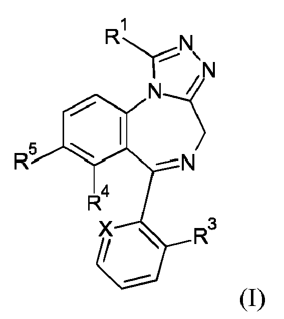 wherein R¹, R³, R⁴, R⁵ and X are as described herein, compositions including the compounds and methods of using the compounds.

## Description

The present invention relates to organic compounds useful for therapy or prophylaxis in a mammal, and in particular to GABA_{A} γ1 receptor positive allosteric modulators (PAMs) for the treatment or prophylaxis of GABA_{A} γ1 receptor related diseases and diseases or conditions which can be treated by the modulation of GABA_{A} γ1 receptor activity, such autism spectrum disorders (ASD) targeting core symptoms and associated comorbidities including anxiety and irritability, Angelman syndrome, Rett syndrome, Prader-Willi syndrome, fragile-X disorder, schizophrenia including psychosis, cognitive impairment and negative symptoms, tardive dyskinesia, anxiety, separation anxiety disorder, selective mutism, specific phobia, social anxiety disorder (social phobia), panic disorder, agoraphobia, generalized anxiety disorder, substance/medication-induced anxiety disorder, disruptive, impulse-control and conduct disorders, Tourette's syndrome (TS), obsessive-compulsive disorder (OCD), acute stress disorder, post-traumatic stress disorder (PTSD), attention deficit hyperactivity disorder (ADHD), sleep disorders including narcolepsy-cataplexy, neurodegenerative conditions including Parkinson's disease (PD), Huntington's chorea, Alzheimer's disease (AD), mild cognitive impairment (MCI) dementia, behavioral and psychological symptoms (BPS) in neurodegenerative conditions, multi-infarct dementia, psychosis and aggression, eating disorders including anorexia nervosa, bulimia nervosa, binge eating disorder, depression and related conditions including treatment-resistant depression (TRD), chronic apathy, anhedonia, chronic fatigue, seasonal affective disorder, postpartum depression, drowsiness, sexual dysfunction, bipolar disorders, epilepsy and pain.

The present invention provides a novel compound of formula (I) 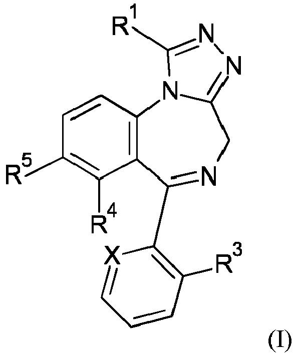 wherein
- R¹: is selected from
i) H,
ii) C₁₋₆-alkyl,
iii) C₁₋₆-alkoxy,
iv) C₁₋₆-alkoxy-C₁₋₆-alkyl,
v) hydroxy,
vi) hydroxy-C₁₋₆-alkyl,
vii) C₃₋₈-cycloalkyl optionally substituted by R⁷, R⁸ and R⁹,
viii) amino-C₁₋₆-alkyl
ix) heteroaryl optionally substituted by R⁷, R⁸ and R⁹, and
x) heterocycloalkyl optionally substituted by R⁷, R⁸ and R⁹;
- R³: is selected from
i) Cl, and
ii) F;
- X: is selected from
i) CR⁶, and
ii) N;
- R⁶: is selected from
i) H,
ii) Cl, and
iii) F;
- R⁴: is selected from
i) Br, and
ii) Cl;
- R⁵: is selected from
i) C₁₋₆-alkyl,
ii) C₁₋₆-alkoxy,
iii) halogen,
iv) halo-C₁₋₆-alkyl,
v) cyano, and
vi) C₃₋₈-cycloalkyl;
- R⁷, R⁸ and R⁹: are independently selected from
i) C₁₋₆-alkyl, and
ii) C₁₋₆-alkoxy;
or pharmaceutically acceptable salts.

Receptors for the major inhibitory neurotransmitter, gamma-aminobutyric acid (GABA), are divided into two main classes: (1) GABA_{A} receptors, which are members of the ligand-gated ion channel superfamily and (2) GABA_{B} receptors, which are members of the G-protein linked receptor family. The GABA_{A} receptor complex which is a membrane-bound heteropentameric protein polymer is composed principally of a, β and y subunits. GABA_{A} receptors are ligand-gated chloride channels and the principal mediators of inhibitory neurotransmission in the human brain.

There are 19 genes encoding for GABA_{A} receptor subunits that assemble as pentamers with the most common stoichiometry being two a, two β and one y subunit. GABA_{A} subunit combinations give rise to functional, circuit, and behavioral specificity (Sieghart, 2006; Vithlani et al., 2011). GABA_{A} receptors containing the γ1 subunit (GABA_{A} γ1) are of particular interest due to their enriched expression in the limbic system (Seeburg et al., 1990; Pirker et al., 2000; Esmaeili et al., 2008; Durisic et al., 2017; Sequeira et al., 2019) and unique physiological and pharmacological properties (Mohler et al., 1996; Wingrove et al., 1997; Sieghart et al., 2005). The GABA_{A} γ1 subunit-containing receptors, while less abundant (around 5-10% of total expression of GABA_{A} receptors in the brain) than y2 subunit-containing receptors exhibit an enriched brain mRNA and protein distribution in key brain areas such as extended amygdala (central, medial, and bed nucleus of the stria terminalis), lateral septum, hypothalamus, and pallidum/nigra. These structures form the interconnected core of a subcortical limbic circuit regulating motivated social and affective behaviors. In abnormal or disease conditions, hyper-recruitment of this circuit promotes anxiety, arousal, aggression, fear and defense while inhibiting foraging and social interactions (Goossens et al., 2007; Hofmann et al., 2011; Fox et al., 2012; Martin-Santos et al., 2014; Anderson et al., 2014; Calhoon et al., 2015).

Hyperactivity in limbic cortical regions (known to form a coordinated functional network with extended amygdala/ hypothalamus regions) which are key areas for processing of social and emotionally relevant stimuli, is the common hallmark of a variety of psychiatric, neurological, neurodevelopmental, neurodegenerative, mood, motivational and metabolic disorders. In such a disease state, and given the characteristic anatomical distribution of the γ1 subunit-containing GABA_{A} receptors, a GABA_{A} γ1 positive allosteric modulator (PAM) may be an effective treatment as a symptomatic or disease-modifying agent.

Multiple lines of evidence suggest that an imbalance between excitatory/inhibitory (E/I) neurotransmission arising from dysfunction of GABAergic signaling system, the main inhibitory neurotransmitter system in the brain, to be at the core of the pathogenesis a variety of CNS disorders. Given the distribution and function of GABA_{A} γ1 subunit-containing receptors in the CNS, they are very attractive targets for restoring levels of inhibition within key brain circuits and consequently the E/I balance in these conditions.

Therefore compounds described herein and their pharmaceutically acceptable salts and esters can be used, alone or in combination with other drugs, as disease-modifying or as symptomatic agents for the treatment or prevention of acute neurological disorders, chronic neurological disorders, cognitive disorders, autism spectrum disorders (ASD), Angelman syndrome, Rett syndrome, Prader-Willi syndrome, fragile-X disorder, schizophrenia, tardive dyskinesia, anxiety, social anxiety disorder (social phobia), panic disorder, agoraphobia, generalized anxiety disorder, disruptive, impulse-control and conduct disorders, Tourette's syndrome (TS), obsessive-compulsive disorder (OCD), acute stress disorder, post-traumatic stress disorder (PTSD), attention deficit hyperactivity disorder (ADHD), sleep disorders, Parkinson's disease (PD), Huntington's chorea, Alzheimer's disease (AD), mild cognitive impairment (MCI), dementia, behavioral and psychological symptoms (BPS) in neurodegenerative conditions, multi-infarct dementia, agitation, psychosis, substance-induced psychotic disorder, aggression, eating disorders, depression, chronic apathy, anhedonia, chronic fatigue, seasonal affective disorder, postpartum depression, drowsiness, sexual dysfunction, bipolar disorders, epilepsy and pain.

The most preferred indications in accordance with the present invention are anxiety, targeting social anxiety disorder (social phobia) and generalized anxiety disorder, and autism spectrum disorder (ASD), targeting core symptoms and associated comorbidities including anxiety and irritability.

ASD is a complex, heterogeneous neurodevelopmental disorder characterized by impairments in two core domains: impairments in social interaction and communication, and presence of repetitive or restricted behaviors, interests, or activities (American Psychiatric Association 2013).

No approved pharmacological treatment exists for core symptoms of social deficits and restricted/repetitive behaviour of ASD, while only inadequate therapeutic options are available for most of ASD's affective and physiological co-morbidities. As a result, this disorder continues to be an area of high unmet medical need. Current approved treatments for associated symptoms of ASD are limited to the antipsychotics (Risperidone and Aripiprazole) indicated for the treatment of irritability associated with ASD symptoms. Emerging evidence suggests that the GABAergic system, the main inhibitory neurotransmitter system in the brain, plays a key role in the pathophysiology of ASD (Dhossche et al., 2002; Pizzarelli and Cherubini, 2011; Robertson et al., 2016).

Both genetic and imaging studies using positron emission tomography study (PET) and magnetic resonance spectroscopy (MRS) suggest alterations in GABAergic signaling in ASD. The gene encoding GABA_{A} γ1: GABRG1 is located on chromosome 4 (mouse Chr.5) in a cluster with genes encoding α2, α4 and β1 GABA_{A} receptor subunits. Rare CNVs, including inversion of chromosome 4p12 disrupting GABRG1 have been observed in autistic siblings (Horike et al., 2006), as well as GABRG1 loss in one case of ADHD. Mutations in 4p12 gene cluster have been linked to increased risk of anxiety, substance abuse and eating disorders - providing a link between GABRG1/4p12 and affective dysfunction. MRS studies found altered GABA levels in ASD (Gaetz et al., 2014; Rojas et al., 2014) and in particular some recent studies showed reduced GABA and altered somatosensory function in children with ASD and (Puts et al., 2016; Robertson et al., 2016). In line with these observations, a reduced number of inhibitory interneurons were found from postmortem tissues of ASD and TS patients (Rapanelli et al., 2017). Furthermore, reduced GABA synthesizing enzymes, glutamic acid decarboxylase (GAD) 65 and 67 were found in parietal and cerebellar cortices of patients with autism (Fatemi et al., 2002). Strong evidence in humans points to specific dysfunction in ASD of the limbic cortical regions known to form a coordinated functional network with GABA_{A} γ1 subunit-containing extended amygdala/ hypothalamus regions. These areas: Cortical/lateral amygdala, Insula, PFC, and Cingulate are recognized key for processing of social and emotionally relevant stimuli. While subcortical subnuclei that form specific partnerships with these areas, coordinating behavioural outcomes, are often difficult to study due to spatial resolution limitations, many lines of evidence point to hyper-recruitment of these cortical- to sub cortical connections in ASD. Moreover, recent high resolution studies provide a clear link between extended amygdala activity /functional connectivity and emotional state (Kleinhans et al., 2009, 2016; Swartz et al., 2013; Nordahl et al., 2016; Ehrlich et al., 2017; Avino et al., 2018; Ibrahim et al., 2019). Targeting such highly specific limbic subcortical regions, which exhibit substantial molecular and cellular diversity compared to the neocortex, will create a precision entry point for safe and specific therapeutic modulation of ASD-affected socio-affective circuits, while avoiding broad modulation of global brain state. Enhancement of GABA_{A} receptor activity by non-selective BZDs have been shown to ameliorate behavioral deficits in mouse models of ASD, however very narrow therapeutic margins were observed due to sedation mediated by the GABA_{A} α1γ2 subtype (Han et al., 2012, 2014; Soto et al., 2013). These findings support the notion that rebalancing of GABAergic transmission *via* GABA_{A} γ1 receptors can improve symptoms in ASD without the side effects of non-selective benzodiazepines.

Objects of the present invention are compounds of formula (I) and their pharmaceutically acceptable salts and esters, the preparation of the above mentioned compounds, medicaments containing them and their manufacture as well as the use of the above mentioned compounds in the treatment or prevention of diseases related to GABA_{A} γ1 receptor dysfunction and diseases or conditions which can be treated by the enhancement of GABA_{A} γ1 receptor activity, such as autism spectrum disorders (ASD), Angelman syndrome, Rett syndrome, Prader-Willi syndrome, fragile-X disorder, schizophrenia, tardive dyskinesia, anxiety, separation anxiety disorder, selective mutism, specific phobia, social anxiety disorder, panic disorder, agoraphobia, generalized anxiety disorder, substance/medication-induced anxiety disorder, disruptive, impulse-control and conduct disorders, Tourette's syndrome (TS), obsessive-compulsive disorder (OCD), acute stress disorder, post-traumatic stress disorder (PTSD), attention deficit hyperactivity disorder (ADHD), sleep disorders including narcolepsy-cataplexy, neurodegenerative conditions including Parkinson's disease (PD), Huntington's chorea, Alzheimer's disease (AD), mild cognitive impairment (MCI) dementia, behavioral and psychological symptoms (BPS) in neurodegenerative conditions, multi-infarct dementia, psychosis and aggression, eating disorders including anorexia nervosa, bullimia nervosa, binge eating disorder, depression and related conditions including treatment-resistant depression (TRD), chronic apathy, anhedonia, chronic fatigue, seasonal affective disorder, postpartum depression, drowsiness, sexual dysfunction, bipolar disorders, epilepsy and pain.

Compounds of the present invention are selective GABA_{A} γ1 receptor positive allosteric modulators (PAMs) as they selectively enhance the function of γ1-containing GABA_{A} receptors by increasing GABAergic currents (influx of chloride) at a given concentration (*e.g.* EC₂₀) of gamma amino butyric acid (GABA). The compounds of the present invention have high PAM efficacy and binding selectivity for the γ1-containing subtypes (α5γ1, α2γ1, α1γ1) relative to the γ2-containing subtypes (*e.g.* α1γ2, α2γ2, α3γ2 and α5γ2). As such, compounds of the present invention are strongly differentiated from classical benzodiazepine drugs such as Alprazolam, Triazolam, Estazolam, Midazolam which are selective for the y2-containing GABA_{A} subtypes and possess low affinity for the γ1-containing subtypes. Compatible with the γ1-subtypes brain distribution, selective GABA_{A} γ1 PAMs will restore GABAergic signaling in key brain regions (e.g. extended amygdala: central, medial, and bed nucleus of the stria terminalis, lateral septum, hypothalamus, and pallidum/nigra) without the side-effects of non-selective GABA_{A} modulators (e.g. benzodiazepines).

The term "amino" denotes a -NH₂ group.

The term "amino-C₁₋₆-alkyl" denotes an C₁₋₆-alkyl group wherein one of the hydrogen atoms of the C₁₋₆-alkyl group has been replaced by an amino group. Examples of amino-C₁₋₆-alkyl include aminomethyl, amionethyl, aminopropyl, aminomethylpropyl, aminomethylethyl and aminobutyl. Particular example includes aminomethyl.

The term "C₁₋₆-alkoxy" denotes a group of the formula -O-R', wherein R' is an C₁₋₆-alkyl group. Examples of C₁₋₆-alkoxy groups include methoxy, ethoxy, n-propoxy, isopropoxy, n-butoxy, isobutoxy and tert-butoxy. Particular examples are methoxy and ethoxy. More particular example is methoxy.

The term "C₁₋₆-alkoxy-C₁₋₆-alkyl" denotes an C₁₋₆-alkyl group wherein at least one of the hydrogen atoms of the C₁₋₆-alkyl group has been replaced by an C₁₋₆-alkoxy group. Exemplary C₁₋₆-alkoxy-C₁₋₆-alkyl groups include methoxymethyl, ethoxymethyl, methoxymethyl, ethoxyethyl, methoxypropyl and ethoxypropyl. Particular example includes methoxyethyl.

The term "C₁₋₆-alkyl" denotes a monovalent linear or branched saturated hydrocarbon group of 1 to 6 carbon atoms. Examples of C₁₋₆-alkyl include methyl, ethyl, n-propyl, isopropyl, n-butyl, iso-butyl, sec-butyl, tert-butyl and pentyl. Particular C₁₋₆-alkyl groups are methyl and ethyl. More particular example is methyl.

The term "C₃₋₈-cycloalkyl" denotes a monovalent saturated monocyclic or bicyclic hydrocarbon group of 3 to 8 ring carbon atoms. Bicyclic means a ring system consisting of two saturated carbocycles having one or two carbon atoms in common. Examples of monocyclic C₃₋₈-cycloalkyl are cyclopropyl, cyclobutanyl, cyclopentyl, cyclohexyl or cycloheptyl. Example of bicyclic C₃₋₈-cycloalkyl is spiro[3.3]heptanyl. Particular monocyclic C₃₋₈-cycloalkyl groups are cyclopropyl and cyclobutanyl. More particular monocyclic C₃₋₈-cycloalkyl group include cyclopropyl.

The term "cyano" denotes a -CN group.

The term "halo-C₁₋₆-alkyl" denotes an C₁₋₆-alkyl group wherein at least one of the hydrogen atoms of the C₁₋₆-alkyl group has been replaced by the same or different halogen atoms. The term "perhalo-C₁₋₆-alkyl-C₁₋₆-alkyl" denotes an-C₁₋₆-alkyl-C₁₋₆-alkyl group where all hydrogen atoms of the alkyl group have been replaced by the same or different halogen atoms. Examples of halo-C₁₋₆-alkyl include fluoromethyl, difluoromethyl, trifluoromethyl, fluoroethyl, difluoroethyl and trifluoroethyl. Particular halo-C₁₋₆-alkyl groups include trifluoromethyl and difluoroethyl. More particular halo-C₁₋₆-alkyl groups are difluoromethyl and trifluoromethyl.

The term "halogen" and "halo" are used interchangeably herein and denote fluoro, chloro, bromo or iodo. Particular halogens include fluoro and chloro.

The term "heteroaryl" denotes a monovalent aromatic heterocyclic mono- or bicyclic ring system of 5 to 12 ring atoms, comprising 1, 2, 3 or 4 heteroatoms selected from N, O and S, the remaining ring atoms being carbon. Examples of heteroaryl group include pyrrolyl, furanyl, thienyl, imidazolyl, oxazolyl, thiazolyl, triazolyl, oxadiazolyl, thiadiazolyl, tetrazolyl, pyridinyl, pyrazinyl, pyrazolyl, pyridazinyl, pyrimidinyl, triazinyl, azepinyl, diazepinyl, isoxazolyl, benzofuranyl, isothiazolyl, benzothienyl, indolyl, isoindolyl, isobenzofuranyl, benzimidazolyl, benzoxazolyl, benzoisoxazolyl, benzothiazolyl, benzoisothiazolyl, benzooxadiazolyl, benzothiadiazolyl, benzotriazolyl, purinyl, quinolinyl, isoquinolinyl, quinazolinyl and quinoxalinyl. Particular heteroaryl groups include pyridinyl, pyrazolyl, pyrimidinyl, pyridazinyl and isoxazolyl. More particular heteroaryl groups are pyrazolyl, pyrimidinyl and pyridazinyl.

The term "heterocycloalkyl" denotes a monovalent saturated or partly unsaturated mono- or bicyclic ring system of 4 to 11 ring atoms, comprising 1, 2, or 3 ring heteroatoms selected from N, O and S, the remaining ring atoms being carbon. Bicyclic means consisting of two cycles having one or two ring atoms in common. Examples for monocyclic saturated heterocycloalkyl are 4,5-dihydro-oxazolyl, oxetanyl, azetidinyl, pyrrolidinyl, 2-oxo-pyrrolidin-3-yl, tetrahydrofuranyl, tetrahydrothiophenyl, pyrazolidinyl, imidazolidinyl, oxazolidinyl, isoxazolidinyl, thiazolidinyl, piperidinyl, tetrahydropyranyl, tetrahydrothiopyranyl, piperazinyl, morpholinyl, thiomorpholinyl, 1,1-dioxo-thiomorpholin-4-yl, azepanyl, diazepanyl, homopiperazinyl, or oxazepanyl. Examples for bicyclic saturated heterocycloalkyl are oxabicyclo[2.2.1]heptanyl, oxaspiro[3.3]heptanyl, 8-aza-bicyclo[3.2.1]octyl, quinuclidinyl, 8-oxa-3-aza-bicyclo[3.2.1]octyl, 9-aza-bicyclo[3.3.1]nonyl, 3-oxa-9-aza-bicyclo[3.3.1]nonyl, or 3-thia-9-aza-bicyclo[3.3.1]nonyl. Examples for partly unsaturated heterocycloalkyl are dihydrofuryl, imidazolinyl, dihydro-oxazolyl, tetrahydro-pyridinyl, or dihydropyranyl. Particular heterocycloalkyl is tetrahydropyranyl.

The term "hydroxy" denotes a -OH group.

The term "hydroxy-C₁₋₆-alkyl" denotes an C₁₋₆-alkyl group wherein one of the hydrogen atoms of the C₁₋₆-alkyl group has been replaced by a hydroxy group. Examples of hydroxy-C₁₋₆-alkyl include hydroxymethyl, hydroxyethyl, hydroxypropyl, hydroxymethylpropyl hydroxymethyl ethyl and hydroxybutyl. Particular example includes hydroxymethyl.

The term "pharmaceutically acceptable salts" refers to those salts which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid and the like, in particular hydrochloric acid, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, *N*-acetylcystein and the like. In addition, these salts may be prepared by addition of an inorganic base or an organic base to the free acid. Salts derived from an inorganic base include, but are not limited to, the sodium, potassium, lithium, ammonium, calcium, magnesium salts and the like. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, *N*-ethylpiperidine, piperidine, polyimine resins and the like. Particular pharmaceutically acceptable salts of compounds of formula (I) are the hydrochloride salts, methanesulfonic acid salts and citric acid salts.

"Pharmaceutically acceptable esters" means that compounds of general formula (I) may be derivatised at functional groups to provide derivatives which are capable of conversion back to the parent compounds in vivo. Examples of such compounds include physiologically acceptable and metabolically labile ester derivatives, such as methoxymethyl esters, methylthiomethyl esters and pivaloyloxymethyl esters. Additionally, any physiologically acceptable equivalents of the compounds of general formula (I), similar to the metabolically labile esters, which are capable of producing the parent compounds of general formula (I) in vivo, are within the scope of this invention.

The term "protecting group" (PG) denotes a group which selectively blocks a reactive site in a multifunctional compound such that a chemical reaction can be carried out selectively at another unprotected reactive site in the meaning conventionally associated with it in synthetic chemistry. Protecting groups can be removed at the appropriate point. Exemplary protecting groups are amino-protecting groups, carboxy-protecting groups or hydroxy-protecting groups. Particular protecting groups are the tert-butoxycarbonyl (Boc), benzyloxycarbonyl (Cbz), fluorenylmethoxycarbonyl (Fmoc) and benzyl (Bn) groups. Further particular protecting groups are the tert-butoxycarbonyl (Boc) and the fluorenylmethoxycarbonyl (Fmoc) groups. More particular protecting group is the tert-butoxycarbonyl (Boc) group.

The abbreviation uM means microMolar and is equivalent to the symbol µM.

The abbreviation uL means microliter and is equivalent to the symbol µL.

The abbreviation ug means microgram and is equivalent to the symbol µg.

The compounds of formula (I) can contain several asymmetric centers and can be present in the form of optically pure enantiomers, mixtures of enantiomers such as, for example, racemates, optically pure diastereoisomers, mixtures of diastereoisomers, diastereoisomeric racemates or mixtures of diastereoisomeric racemates.

According to the Cahn-Ingold-Prelog Convention the asymmetric carbon atom can be of the "R" or "S" configuration.

Also an embodiment of the present invention is a compound according to formula (I) as described herein and pharmaceutically acceptable salts or esters thereof, in particular compounds according to formula (I) as described herein and pharmaceutically acceptable salts thereof, more particularly compounds according to formula (I) as described herein.

A particular embodiment of the present invention provides a compound according to formula (I) as described herein, wherein
- R¹: is selected from
iii) H,
iv) C₁₋₆-alkyl,
v) C₁₋₆-alkoxy,
vi) C₁₋₆-alkoxy-C₁₋₆-alkyl,
vii) hydroxy,
viii) hydroxy-C₁₋₆-alkyl,
ix) C₃₋₈-cycloalkyl optionally substituted by R⁷, R⁸ and R⁹,
x) amino-C₁₋₆-alkyl
xi) pyrazolyl optionally substituted by R⁷, R⁸ and R⁹,
xii) pyridinyl optionally substituted by R⁷, R⁸ and R⁹,
xiii) pyrimidinyl optionally substituted by R⁷, R⁸ and R⁹,
xiv) pyridazinyl optionally substituted by R⁷, R⁸ and R⁹, and
xv) tetrahydropyranyl optionally substituted by R⁷, R⁸ and R⁹;
- R³: is selected from
i) Cl, and
ii) F;
- X: is selected from
i) CR⁶, and
ii) N;
- R⁶: is selected from
i) H,
ii) Cl, and
iii) F;
- R⁴: is selected from
i) Br, and
ii) Cl;
- R⁵: is selected from
i) C₁₋₆-alkyl,
ii) C₁₋₆-alkoxy,
iii) halogen,
iv) halo-C₁₋₆-alkyl,
v) C₃₋₈-cycloalkyl;
- R⁷, R⁸ and R⁹: are independently selected from
i) C₁₋₆-alkyl, and
ii) C₁₋₆-alkoxy;
or pharmaceutically acceptable salts.

A more particular embodiment of the present invention provides a compound of formula (I) according to claim 1, wherein
- R¹: is selected from
iii) H,
iv) C₁₋₆-alkyl,
v) hydroxy,
vi) hydroxy-C₁₋₆-alkyl,
vii) C₃₋₈-cycloalkyl optionally substituted by R⁷, R⁸ and R⁹,
viii) pyrazolyl optionally substituted by R⁷, R⁸ and R⁹,
ix) pyrimidinyl optionally substituted by R⁷, R⁸ and R⁹,
x) pyridazinyl optionally substituted by R⁷, R⁸ and R⁹, and
xi) tetrahydropyranyl optionally substituted by R⁷, R⁸ and R⁹;
- R³: is F;
- X: is selected from
i) CR⁶, and
ii) N;
- R⁶: is selected from
i) H, and
ii) F;
- R⁴: is selected from
i) Br, and
ii) Cl;
- R⁵: is selected from
i) C₁₋₆-alkyl,
ii) halogen, and
iii) halo-C₁₋₆-alkyl;
- R⁷, R⁸ and R⁹: are independently selected from C₁₋₆-alkyl,
or pharmaceutically acceptable salts.

A furthermore particular embodiment of the present invention provides a compound according to formula (I) as described herein,
- R¹: is C₁₋₆-alkyl;
- R³: is F;
- X: is CR⁶;
- R⁶: is F;
- R⁴: is Cl;
- R⁵: is halo-C₁₋₆-alkyl;
or pharmaceutically acceptable salts.

Another particular embodiment of the present invention provides a compound according to formula (I) as described herein, wherein R¹ is selected from
- i): C₃₋₈-cycloalkyl optionally substituted by R⁷, R⁸ and R⁹,
- ii): amino-C₁₋₆-alkyl
- iii): pyrazolyl optionally substituted by R⁷, R⁸ and R⁹,
- iv): pyridinyl optionally substituted by R⁷, R⁸ and R⁹,
- v): pyrimidinyl optionally substituted by R⁷, R⁸ and R⁹,
- vi): pyridazinyl optionally substituted by R⁷, R⁸ and R⁹, and
- vii): tetrahydropyranyl optionally substituted by R⁷, R⁸ and R⁹;

Another particular embodiment of the present invention provides a compound according to formula (I) as described herein, wherein R¹ is selected from
- i): H,
- ii): C₁₋₆-alkyl,
- iii): H,
- iv): C₁₋₆-alkyl,
- v): hydroxy,
- vi): hydroxy-C₁₋₆-alkyl,
- vii): C₃₋₈-cycloalkyl optionally substituted by R⁷, R⁸ and R⁹,
- viii): pyrazolyl optionally substituted by R⁷, R⁸ and R⁹,
- ix): pyrimidinyl optionally substituted by R⁷, R⁸ and R⁹,
- x): pyridazinyl optionally substituted by R⁷, R⁸ and R⁹, and
- xi): tetrahydropyranyl optionally substituted by R⁷, R⁸ and R⁹;

Another particular embodiment of the present invention provides a compound according to formula (I) as described herein, wherein R¹ is C₁₋₆-alkyl.

Another particular embodiment of the present invention provides a compound according to formula (I) as described herein, wherein R³ is F.

Another particular embodiment of the present invention provides a compound according to formula (I) as described herein, wherein X is CR⁶.

Another particular embodiment of the present invention provides a compound according to formula (I) as described herein, wherein R⁶ is F.

Another particular embodiment of the present invention provides a compound according to formula (I) as described herein, wherein R⁴ is Cl.

Another particular embodiment of the present invention provides a compound according to formula (I) as described herein, wherein R⁵ is selected from
- i): C₁₋₆-alkyl,
- ii): C₁₋₆-alkoxy,
- iii): halogen,
- iv): halo-C₁₋₆-alkyl,
- v): C₃₋₈-cycloalkyl;

Another particular embodiment of the present invention provides a compound according to formula (I) as described herein, wherein R⁵ is selected from
- i): C₁₋₆-alkyl,
- ii): halogen, and
- iii): halo-C₁₋₆-alkyl;

Another particular embodiment of the present invention provides a compound according to formula (I) as described herein, wherein R⁵ is halo-C₁₋₆-alkyl.

Another particular embodiment of the present invention provides a compound according to formula (I) as described herein, wherein R⁷, R⁸ and R⁹ are independently selected from C₁₋₆-alkyl.

Particular examples of a compound of formula (I) as described herein are selected from
8-bromo-7-chloro-6-(2,6-difluorophenyl)-1-methyl-4H-[1,2,4]triazolo[4,3-a][1,4]benzodiazepine;
8-bromo-7-chloro-1-cyclopropyl-6-(2-fluorophenyl)-4H-[1,2,4]triazolo[4,3-a][1,4]benzodiazepine;
8-bromo-7-chloro-6-(2-fluorophenyl)-1-pyridazin-3-yl-4H-[1,2,4]triazolo[4,3-a][1,4]benzodiazepine;
7,8-dichloro-6-(2-fluorophenyl)-1-methyl-4H-[1,2,4]triazolo[4,3-a][1,4]benzodiazepine; 7,8-dichloro-6-(2,6-difluorophenyl)-1-methyl-4H-[1,2,4]triazolo[4,3-a][1,4]benzodiazepine;
7-chloro-8-cyclopropyl-6-(2,6-difluorophenyl)-1-methyl-4H-[1,2,4]triazolo[4,3-a][1,4]benzodiazepine;
8-bromo-7-chloro-6-(2,6-difluorophenyl)-4H-[1,2,4]triazolo[4,3-a][1,4]benzodiazepine;
8-bromo-7-chloro-6-(2-fluorophenyl)-4H-[1,2,4]triazolo[4,3-a][1,4]benzodiazepine;
7,8-dichloro-6-(2,6-difluorophenyl)-2,4-dihydro-[1,2,4]triazolo[4,3-a][1,4]benzodiazepin-1-one;
7,8-dichloro-6-(2-fluorophenyl)-1-pyridazin-3-yl-4H-[1,2,4]triazolo[4,3-a][1,4]benzodiazepine;
7,8-dichloro-6-(2,6-difluorophenyl)-1-tetrahydropyran-4-yl-4H-[1,2,4]triazolo[4,3-a][1,4]benzodiazepine;
7,8-dichloro-6-(2,6-difluorophenyl)-1-pyrimidin-4-yl-4H-[1,2,4]triazolo[4,3-a][1,4]benzodiazepine;
8-bromo-7-chloro-6-(2,6-difluorophenyl)-2,4-dihydro-[1,2,4]triazolo[4,3-a][1,4]benzodiazepin-1-one;
7,8-dichloro-6-(2,6-difluorophenyl)-1-pyridazin-3-yl-4H-[1,2,4]triazolo[4,3-a][1,4]benzodiazepine;
7,8-dichloro-6-(2,6-difluorophenyl)-4H-[1,2,4]triazolo[4,3-a][1,4]benzodiazepine;
7-chloro-6-(2,6-difluorophenyl)-8-methyl-2,4-dihydro-[1,2,4]triazolo[4,3-a][1,4]benzodiazepin-1-one;
7-chloro-6-(2,6-difluorophenyl)-8-methyl-4H-[1,2,4]triazolo[4,3-a][1,4]benzodiazepine;
7,8-dichloro-6-(2-fluorophenyl)-1-pyrimidin-4-yl-4H-[1,2,4]triazolo[4,3-a][1,4]benzodiazepine;
7,8-dichloro-6-(2,6-difluorophenyl)-1-(2-methoxyethyl)-4H-[1,2,4]triazolo[4,3-a][1,4]benzodiazepine;
7-chloro-6-(2,6-difluorophenyl)-1,8-dimethyl-4H-[1,2,4]triazolo[4,3-a][1,4]benzodiazepine;
7-chloro-8-(1,1-difluoroethyl)-6-(2,6-difluorophenyl)-4H-[1,2,4]triazolo[4,3-a][1,4]benzodiazepine;
7-chloro-8-(1,1-difluoroethyl)-6-(2,6-difluorophenyl)-1-methyl-4H-[1,2,4]triazolo[4,3-a][1,4]benzodiazepine;
7-chloro-8-(difluoromethyl)-6-(2,6-difluorophenyl)-4H-[1,2,4]triazolo[4,3-a][1,4]benzodiazepine;
8-bromo-7-chloro-6-(3-fluoro-2-pyridyl)-1-methyl-4H-[1,2,4]triazolo[4,3-a][1,4]benzodiazepine;
7-chloro-8-(difluoromethyl)-6-(2,6-difluorophenyl)-1-methyl-4H-[1,2,4]triazolo[4,3-a][1,4]benzodiazepine;
8-bromo-7-chloro-6-(2-fluorophenyl)-2,4-dihydro-[1,2,4]triazolo[4,3-a][1,4]benzodiazepin-1-one;
7-chloro-6-(2-fluorophenyl)-1-pyridazin-3-yl-8-(trifluoromethyl)-4H-[1,2,4]triazolo[4,3-a][1,4]benzodiazepine;
7,8-dichloro-6-(2,6-difluorophenyl)-1-(1-methylpyrazol-4-yl)-4H-[1,2,4]triazolo[4,3-a][1,4]benzodiazepine;
8-bromo-7-chloro-6-(3 -fluoro-2-pyridyl)-4H-[1,2,4]triazolo[4,3-a][1,4]benzodiazepine;
[8-bromo-7-chloro-6-(2,6-difluorophenyl)-4H-[1,2,4]triazolo[4,3-a][1,4]benzodiazepin-1-yl]methanol;
7-chloro-6-(2,6-difluorophenyl)-8-iodo-4H-[1,2,4]triazolo[4,3-a][1,4]benzodiazepine;
[8-bromo-7-chloro-6-(2,6-difluorophenyl)-4H-[1,2,4]triazolo[4,3-a][1,4]benzodiazepin-1-yl]methanamine hydrochloride;
7-chloro-6-(2,6-difluorophenyl)-8-(trifluoromethyl)-4H-[1,2,4]triazolo[4,3-a][1,4]benzodiazepine;
7-chloro-6-(2,6-difluorophenyl)-1-pyridazin-3-yl-8-(trifluoromethyl)-4H-[1,2,4]triazolo[4,3-a][1,4]benzodiazepine;
7-chloro-6-(2,6-difluorophenyl)-8-iodo-1-pyridazin-3-yl-4H-[1,2,4]triazolo[4,3-a][1,4]benzodiazepine;
7-chloro-6-(2,6-difluorophenyl)-1-methyl-8-(trifluoromethyl)-4H-[1,2,4]triazolo[4,3-a][1,4]benzodiazepine;
7-chloro-6-(2,6-difluorophenyl)-8-iodo-1-methyl-4H-[1,2,4]triazolo[4,3-a][1,4]benzodiazepine;
7,8-dibromo-6-(2,6-difluorophenyl)-1-pyridazin-3-yl-4H-[1,2,4]triazolo[4,3-a][1,4]benzodiazepine;
7,8-dibromo-6-(2,6-difluorophenyl)-1-pyrimidin-4-yl-4H-[1,2,4]triazolo[4,3-a][1,4]benzodiazepine;
8-bromo-7-chloro-6-(3-chloro-2-pyridyl)-1-pyrimidin-4-yl-4H-[1,2,4]triazolo[4,3-a][1,4]benzodiazepine;
8-bromo-7-chloro-6-(3-fluoro-2-pyridyl)-1-pyridazin-3-yl-4H-[1,2,4]triazolo[4,3-a][1,4]benzodiazepine;
8-bromo-7-chloro-6-(3-fluoro-2-pyridyl)-1-pyrimidin-4-yl-4H-[1,2,4]triazolo[4,3-a][1,4]benzodiazepine;
8-bromo-7-chloro-6-(3-chloro-2-pyridyl)-1-pyridazin-3-yl-4H-[1,2,4]triazolo[4,3-a][1,4]benzodiazepine;
7-chloro-6-(2,6-difluorophenyl)-8-methyl-1-pyridazin-3-yl-4H-[1,2,4]triazolo[4,3-a][1,4]benzodiazepine;
7-chloro-6-(2,6-difluorophenyl)-8-methyl-1-pyrimidin-4-yl-4H-[1,2,4]triazolo[4,3-a][1,4]benzodiazepine;
8-bromo-7-chloro-6-(2-chloro-6-fluoro-phenyl)-1-methyl-4H-[1,2,4]triazolo[4,3-a][1,4]benzodiazepine;
7,8-dichloro-6-(3-fluoro-2-pyridyl)-1-pyridazin-3-yl-4H-[1,2,4]triazolo[4,3-a][1,4]benzodiazepine;
7,8-dichloro-6-(3-fluoro-2-pyridyl)-1-pyrimidin-4-yl-4H-[1,2,4]triazolo[4,3-a][1,4]benzodiazepine;
7-chloro-6-(2,6-difluorophenyl)-8-methoxy-1-pyridazin-3-yl-4H-[1,2,4]triazolo[4,3-a][1,4]benzodiazepine;
7,8-dichloro-6-(3-fluoro-2-pyridyl)-1-(1-methylpyrazol-4-yl)-4H-[1,2,4]triazolo[4,3-a][1,4]benzodiazepine;
7,8-dichloro-6-(3-fluoro-2-pyridyl)-2,4-dihydro-[1,2,4]triazolo[4,3-a][1,4]benzodiazepin-1-one;
7,8-dichloro-6-(3-fluoro-2-pyridyl)-4H-[1,2,4]triazolo[4,3-a][1,4]benzodiazepine;
7-chloro-6-(2,6-difluorophenyl)-8-methoxy-1-pyrimidin-4-yl-4H-[1,2,4]triazolo[4,3-a][1,4]benzodiazepine;
7-chloro-6-(2,6-difluorophenyl)-8-methoxy-1-(3-pyridyl)-4H-[1,2,4]triazolo[4,3-a][1,4]benzodiazepine;
7,8-dichloro-1-cyclopropyl-6-(3-fluoro-2-pyridyl)-4H-[1,2,4]triazolo[4,3-a][1,4]benzodiazepine;
7-chloro-6-(3-fluoro-2-pyridyl)-1-pyrimidin-4-yl-8-(trifluoromethyl)-4H-[1,2,4]triazolo[4,3-a][1,4]benzodiazepine;
7-chloro-6-(3-fluoro-2-pyridyl)-1-(1-methylpyrazol-4-yl)-8-(trifluoromethyl)-4H-[1,2,4]triazolo[4,3-a] [1,4]benzodiazepine;
7,8-dichloro-6-(3-fluoro-2-pyridyl)-1-methyl-4H-[1,2,4]triazolo[4,3-a][1,4]benzodiazepine;
7-chloro-6-(3-fluoro-2-pyridyl)-1-pyridazin-3-yl-8-(trifluoromethyl)-4H-[1,2,4]triazolo[4,3-a][1,4]benzodiazepine;
8-bromo-7-chloro-1-cyclopropyl-6-(3-fluoro-2-pyridyl)-4H-[1,2,4]triazolo[4,3-a][1,4]benzodiazepine;
7-chloro-6-(3-fluoro-2-pyridyl)-1-methyl-8-(trifluoromethyl)-4H-[1,2,4]triazolo[4,3-a][1,4]benzodiazepine;
7-chloro-1-cyclopropyl-6-(2,6-difluorophenyl)-8-methoxy-4H-[1,2,4]triazolo[4,3-a][1,4]benzodiazepine;
7,8-dichloro-6-(2,6-difluorophenyl)-1-methoxy-4H-[1,2,4]triazolo[4,3-a][1,4]benzodiazepine;
*trans*-7,8-dichloro-6-(2,6-difluorophenyl)-1-(3-methoxycyclobutyl)-4H-[1,2,4]triazolo[4,3-a][1,4]benzodiazepine;
8-bromo-7-chloro-6-(2,6-difluorophenyl)-1-pyridazin-3-yl-4H-[1,2,4]triazolo[4,3-a][1,4]benzodiazepine;
or pharmaceutically acceptable salts thereof.

Furthermore particular examples of a compound of formula (I) as described herein are selected from
7-chloro-6-(2,6-difluorophenyl)-1-methyl-8-(trifluoromethyl)-4H-[1,2,4]triazolo[4,3-a][1,4]benzodiazepine;
7-chloro-8-(1,1-difluoroethyl)-6-(2,6-difluorophenyl)-1-methyl-4H-[1,2,4]triazolo[4,3-a][1,4]benzodiazepine;
or pharmaceutically acceptable salts thereof.

Even more particular examples of a compound of formula (I) as described herein are selected from
7-chloro-6-(2,6-difluorophenyl)-1-methyl-8-(trifluoromethyl)-4H-[1,2,4]triazolo[4,3-a][1,4]benzodiazepine;
or pharmaceutically acceptable salts thereof.

Processes for the manufacture of a compound of formula (I) as described herein are also an object of the invention.

The preparation of compounds of formula (I) of the present invention may be carried out in sequential or convergent synthetic routes. Syntheses of the invention are shown in the following general schemes. The skills required for carrying out the reactions and purifications of the resulting products are known to those skilled in the art. The substituents and indices used in the following description of the processes have the significance given herein before unless indicated to the contrary.

In more detail, the compounds of formula (I) can be manufactured by the methods given below, by the methods given in the examples or by analogous methods. Appropriate reaction conditions for the individual reaction steps are known to a person skilled in the art. The reaction sequence is not limited to the one displayed in schemes 1 - 3, however, depending on the starting materials and their respective reactivity the sequence of reaction steps can be freely altered. Starting materials are either commercially available or can be prepared by methods analogous to the methods given below, by methods described in references cited in the description or in the examples, or by methods known in the art.

The present compounds of formula (I) and their pharmaceutically acceptable salts can be prepared by a process described below (Scheme 1).

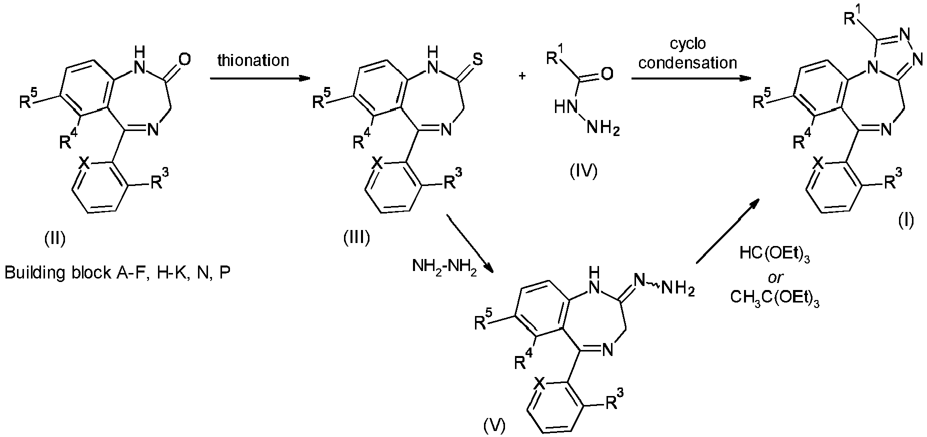

### Scheme 1: synthesis of benzodiazepines (I) wherein all definitions are as described above and in the claims

According to Scheme 1, a compound of formula (I) can be prepared in two steps starting from lactame building blocks (A-F, H-K, N, P) of formula (II). Following thionation reaction using Lawesson's reagent or P₂S₅, lactames (II) are converted to corresponding thiolactames (III). Their reaction with hydrazides (IV) *via* a Pellizzari type process yields 1,2,4-triazoles of general formula (I). In alternative, 1,2,4-triazoles (I) can be obtained by reaction between thiolactames (II) and hydrazine to form hydrazones (V) followed by treatment with triethyl orthoacetate or triethyl orthoformate.

In certain embodiments of the invention where R¹ is hydroxyl (OH), benzodiazepines of formula (I) can be obtained in two steps according to the process described in Scheme 2. It is widely accepted that 3-hydroxy-1,2,4-triazoles are existing as two tautomeric forms and in this invention they will be represented exclusively in their most stable form (triazolones). To this end, hydrazones (V) can be reacted with 1,1'-carbonyldiimidazole (CDI) to yield triazolones of formula (I) (Scheme 2).

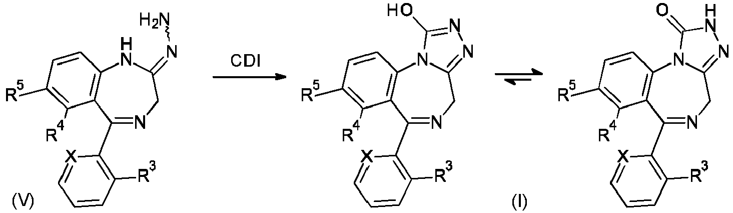

### Scheme 2: synthesis of benzodiazepines (I) wherein R¹ is hydroxyl; all other definitions are as described above and in the claims

The synthesis of building blocks (A-F, H-K, N, P) of formula (II) is highlighted in Scheme 3. Commercially available 2-amino-6-chlorobenzoic acid or 2-amino-6-bromobenzoic acid can be heated in acetic anhydride to form 5-chloro-2-methyl-3,1-benzoxazin-4-one and 5-bromo-2-methyl-3,1-benzoxazin-4-one, respectively. Grignard or organolithium reagents of formula (VI) (prepared by metalation reaction from corresponding aryl bromide or *via* kinetic deprotonation) can be reacted with benzoxazin-4-ones (electrophiles) at controlled temperatures to provide ketones of formula (VII). Following *N*-acetamide hydrolysis under acidic conditions (HCl), compounds of formula (VII) are converted into anilines of formula (VIII). Conveniently, at this junction, the halogen at R⁵ can be installed by treatment with *N*-chlorosuccinimide (NCS), *N-*bromosuccinimide (NBS) or *N*-iodosuccinimide (NIS) to yield intermediates of formula (IX). Final thermal cyclisation reaction with ethyl 2-aminoacetate hydrochloride in pyridine yields the desired benzodiazepines (II), presumably via formation of imine intermediate (X).

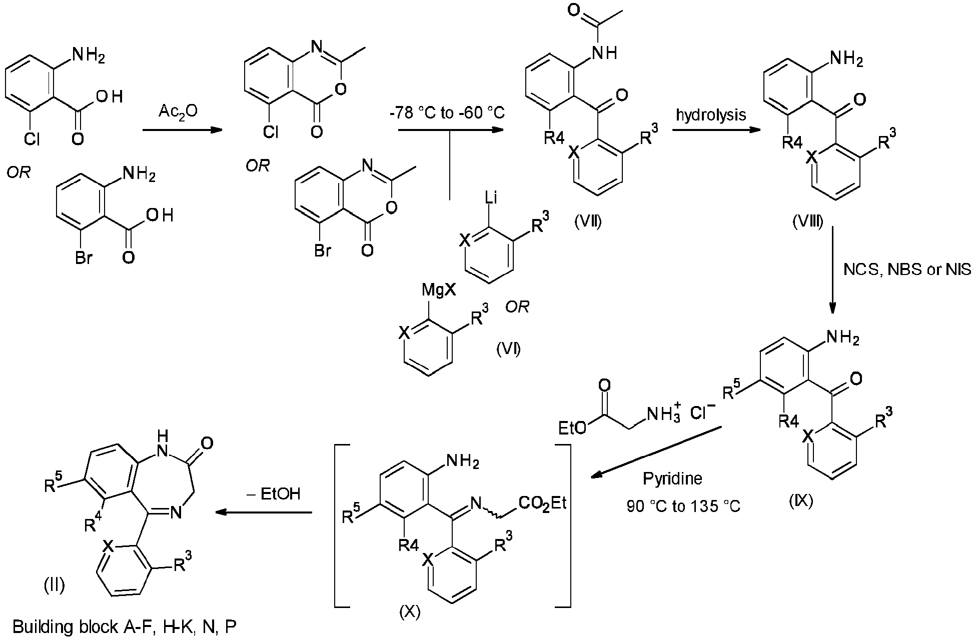

### Scheme 3: synthesis of building blocks (A-F, H-K, N, P) wherein R⁵ is Cl, Br or I and all other definitions are as described above and in the claims

Also an embodiment of the present invention is a process to prepare a compound of formula (I) as defined above comprising the reaction of a compound of formula (III) with a compound of formula (IV) in a solvent, particularly an alcohol, such as butan-1-ol, and at temperature between room temperature and reflux of solvent, particularly at reflux of solvent. 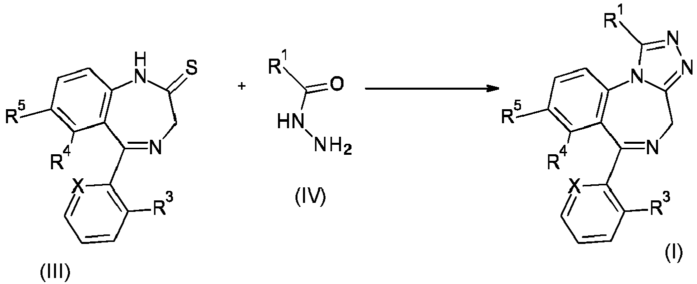 wherein R¹, R³, R⁴ and R⁵ are as defined herein.

Also an object of the present invention is a compound according to formula (I), more particularly compounds of formula (I), as described herein for use as a therapeutically active substance.

Likewise an object of the present invention is a pharmaceutical composition comprising a compound according to formula (I), more particularly compounds of formula (I), as described herein and a therapeutically inert carrier.

A particular embodiment of the present invention is a compound according to formula (I) or pharmaceutically acceptable salts thereof, as described herein for use in the treatment or prophylaxis, more particularly for use the treatment, of Alzheimer's disease, mild cognitive impairment (MCI), age-related cognitive decline, negative and/or cognitive symptoms associated with schizophrenia, bipolar disorders, autism spectrum disorder (ASD), Angelman syndrome, Rett syndrome, Prader-Willi syndrome, epilepsy, post-traumatic stress disorder (PTSD), amyotrophic lateral sclerosis (ALS), fragile-X disorder, more particularly autism spectrum disorder (ASD), Angelman syndrome, Alzheimer's disease, negative and/or cognitive symptoms associated with schizophrenia and post-traumatic stress disorder (PTSD).

The present invention also relates to the use of a compound according to formula (I) or pharmaceutically acceptable salts thereof, more particularly compounds of formula (I), as described herein for the preparation of a medicament for the treatment or prophylaxis, more particularly the treatment, of Alzheimer's disease, mild cognitive impairment (MCI), age-related cognitive decline, negative and/or cognitive symptoms associated with schizophrenia, bipolar disorders, autism spectrum disorder (ASD), Angelman syndrome, Rett syndrome, Prader-Willi syndrome, epilepsy, post-traumatic stress disorder (PTSD), amyotrophic lateral sclerosis (ALS), fragile-X disorder, more particularly autism spectrum disorder (ASD), Angelman syndrome, Alzheimer's disease, negative and/or cognitive symptoms associated with schizophrenia and post-traumatic stress disorder (PTSD).

Also an object of the invention is a method for the treatment or prophylaxis, more particularly the treatment, of Alzheimer's disease, mild cognitive impairment (MCI), age-related cognitive decline, negative and/or cognitive symptoms associated with schizophrenia, bipolar disorders, autism spectrum disorder (ASD), Angelman syndrome, Rett syndrome, Prader-Willi syndrome, epilepsy, post-traumatic stress disorder (PTSD), amyotrophic lateral sclerosis (ALS), fragile-X disorder, more particularly autism spectrum disorder (ASD), Angelman syndrome, Alzheimer's disease, negative and/or cognitive symptoms associated with schizophrenia and post-traumatic stress disorder (PTSD), which method comprises administering an effective amount of a compound according to formula (I), more particularly compounds of formula (I), as described herein.

Also an embodiment of the present invention are compounds of formula (I), more particularly compounds of formula (I), as described herein, when manufactured according to any one of the described processes.

### Assay procedures

### Membrane preparation and binding assay for γ1-containing GABA_{A} subtypes

The affinity of compounds at GABA_{A} γ1 subunit-containing receptors was measured by competition for [³H]RO7239181 (67.3 Ci/mmol; Roche) binding to membranes from HEK293F cells (ThermoFisher R79007) expressing human (transiently transfected) receptors of composition α5β2γ1, α2β2γ1, α1β2γ1. For better protein expression of the α2 subunit-containing receptors, the 28 amino acid long signal peptide (Met1 to Ala28)of the human GABA_{A} α2 subunit was substituted by the 31 amino acid long signal peptide (Met1 to Ser31) of human GABA_{A} α5 subunit.

Harvested pellets from HEK293F cells expressing the different GABA_{A} receptor subtypes were resuspended in Mannitol Buffer pH 7.2-7.4 (Mannitol 0.29M, Triethylamine 10mM, Acetic acid 10mM, EDTA 1mM plus protease inhibitors (20 tablets Complete, Roche Diagnostics Cat. No. 05 056 489 001 per liter)), washed two times and then resuspended at 1:10 to 1:15 dilution in the same buffer. Cell disruption was performed by stirring the suspension in a Parr vessel #4637 at 435 psi for 15 minutes, and then the suspensions were centrifuged at 1000xg for 15 minutes at 4°C (Beckman Avanti J-HC; rotor JS-4.2). The supernatant (S1) was transferred in a 21 Schott flask and the pellet (P1) was resuspended with Mannitol Buffer up to 175ml. The resuspended pellet was transferred into a 250ml Corning centrifugal beaker and centrifuged at 1500xg for 10 minutes at 4°C (Beckman Avanti J-HC; rotor JS-4.2). The supernatant (S1) was then transferred in the 21 Schott flask and the pellet was discarded. The supernatants (S1) were centrifuged in 500ml Beckman polypropylene centrifugal beaker at 15'000xg for 30 minutes at 4°C (Beckman Avanti J-20 XP; rotor JLA-10.500). The pellet (P2) was resuspended with Mannitol Buffer 1:1 and frozen at -80°C. The supernatant (S2) was centrifuged in 100 ml Beckman polypropylene centrifugal tubes at 48000xg for 50 minutes at 4°C (Beckman Avanti J-20 XP; rotor JA-18). The supernatant (S3) was discarded and the pellet (P3) was resuspended with 1:1 Mannitol Buffer. The P2 and P3 protein concentration was determined with the BIORAD Standard assay method with bovine serum albumin as standard and measured on the NANO-Drop 1000. The membrane suspension was aliquots (500µlper tube) and stored at -80°C until required.

Membrane homogenates were resuspended and polytronised (Polytron PT1200E Kinematica AG) in Potassium Phosphate 10mM, KCl 100mM binding buffer at pH 7.4 to a final assay concentration determined with a previous experiment.

Radioligand binding assays were carried out in a volume of 200 µL (96-well plates) which contained 100 µL of cell membranes, [³H]RO7239181 at a concentration of 1.5 nM (α5β2γ1) or 20-30 nM (α1β2γ1, α2β2γ1) and the test compound in the range of [0.3-10000] × 10⁻⁹ M. Nonspecific binding was defined by 10 × 10⁻⁶ (α5β2γ1) and 30 × 10⁻⁶ M RO7235136 and typically represented less than 5% (α5β2γ1) and less than 20% (α1β2γ1, α2β2γ1) of the total binding. Assays were incubated to equilibrium for 1 hour at 4 °C and then, membranes were filtered onto unifilter (96-well white microplate with bonded GF/C filters preincubated 20-50 minutes in 0.3% Polyethylenimine) with a Filtermate 196 harvester (Packard BioScience) and washed 4 times with cold Potassium Phosphate 10mM pH 7.4, KCl 100mM binding buffer. After drying, filter-retained radioactivity was detected by liquid scintillation counting. Kᵢ values were calculated using Excel-Fit (Microsoft) and are the means of two determinations.

The compounds of the accompanying examples were tested in the above described assays, and the preferred compounds were found to possess a Kᵢ value for the displacement of [³H]RO7239181 from GABA_{A} γ1 subunit-containing receptors (e.g. α5β2γ1, α2β2γ1, α1β2γ1) of 100 nM or less. Most preferred are compounds with a Ki (nM) < 50. Representative test results, obtained by the above described assay measuring binding affinity to HEK293 cells expressing human (h) receptors, are shown in the Table 1.

### Preparation of [³H]RO7239181, 6-chloro-5-(2,6-difluorophenyl)-7-methyl-1-(tritritiomethyl)-3H-1,4-benzodiazepin-2-one

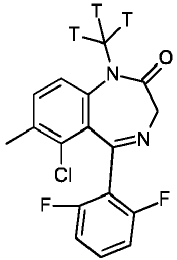

### a) 6-Chloro-5-(2,6-difluorophenyl)-7-methyl-1,3 -dihydro-1,4-benzodiazepin-2-one

A microwave tube was charged with 7-bromo-6-chloro-5-(2,6-difluorophenyl)-1,3-dihydro-1,4-benzodiazepin-2-one (building block A (see infra), 450 mg, 1.17 mmol), trimethylboroxine (205 mg, 228 µL, 1.63 mmol), potassium carbonate (242 mg, 1.75 mmol) and tetrakis(triphenylphosphine)palladium (0) (67.4 mg, 58.4 µmol). Degassed 1,4-dioxane (8.1 mL) and H₂O (2.7 ml) were added then the vial was capped. The suspension was reacted in microwave at 130 °C for 30 min to give complete conversion. The mixture was evaporated, treated with sat. aq. NaHCO₃ (20 mL) and extracted with EtOAc (2 x 20 mL). The organic layers were dried over Na₂SO₄, filtered and solvents were evaporated. The residue was purified by flash chromatography (silica gel, 40 g, eluted with CH₂Cl₂/EtOAc in heptane 10% to 40% to 70%) to give the title compound (344 mg, 92%) as light yellow solid. MS (ESI): 321.1 ([M+H]⁺).

### b) 6-Chloro-5-(2,6-difluorophenyl)-7-methyl-1-(tritritiomethyl)-3H-1,4-benzodiazepin-2-one

To a solution of [³H]methyl nosylate (1.85 GBq, 50 mCi, 0.61 µmol) in THF (200 µL) were added the *N*-desmethyl precursor 6-chloro-5-(2,6-difluorophenyl)-7-methyl-1,3-dihydro-1,4-benzodiazepin-2-one (0.43 mg, 1.34 µmol) dissolved in THF (200 µL) and 10 equivalents of sodium *tert*-butylate (0.5 M in THF, 13.4 µmol). After stirring for 4 h at room temperature the reaction mixture was treated with H₂O, evaporated, and the crude product was purified by HPLC (X-Terra Prep RP-18, 10 x 150 mm, MeCN/H₂O (containing 5% of MeCN) 40:60, 4 ml/min, 230 nm). The pure tritium-labeled compound was isolated by solid phase extraction (Sep-Pak Plus C18) and eluted from the cartridge as ethanolic solution to yield 1.6 GBq (43.2 mCi) of the target compound in > 99% radio-chemical purity and a specific activity of 2.49 TBq/mmol (67.3 Ci/mmol) as determined by mass spectrometry (MS). The identity of the labeled compound was confirmed by HPLC (by co-injecting the unlabeled reference standard) and by MS.
MS: m/z = 335 [M(H)+H]⁺ (16%), 337 [M(³H)+H]⁺ (0%), 339 [M(³H₂)+H]⁺ (16%), 341 [M(³H₃)+H]⁺ (68%).

### Membrane preparation and binding assay for y2-containing GABA_{A} subtypes

The affinity of compounds at GABA_{A} y2 subunit-containing receptors was measured by competition for [³H]Flumazenil (81.1 Ci/mmol; Roche) binding to HEK293F cells expressing human (transiently transfected) receptors of composition α1β3γ2.

Harvested pellets from HEK293F cells expressing the different GABA_{A} y2 receptor subtypes were resuspended in Mannitol Buffer pH 7,2 -7,4 and processed as described above for the cells expressing the GABA_{A} γ1 subunit-containing receptors.

Radioligand binding assays were carried out in a volume of 200 µL (96-well plates) which contained 100 µL of cell membranes, [³H]Flumazenil at a concentration of 1 nM and the test compound in the range of 0.1 × 10⁻⁹ to 30 × 10⁻⁶ M. Nonspecific binding was defined by 10⁻⁵ M Diazepam and typically represented less than 5% of the total binding. Assays were incubated to equilibrium for 1 hour at 4 °C and harvested onto GF/C uni-filters (Packard) by filtration using a Packard harvester and washing with ice-cold wash buffer (50 mM Tris; pH 7.5). After drying, filter-retained radioactivity was detected by liquid scintillation counting. Kᵢ values were calculated using Excel-Fit (Microsoft) and are the means of two determinations.

The compounds of the accompanying examples were tested in the above described assay, and the preferred compounds were found to possess large Kᵢ value for displacement of [³H]Flumazenil from the α1β3γ2 subtype of the human GABA_{A} receptor of 100 nM or above. Most preferred are compounds with a Kᵢ α1β3γ2 (nM) > 300. In a preferred embodiment the compounds of the invention are binding selective for the γ1 subunit-containing GABA_{A} receptors relative to y2 subunit-containing GABA_{A} receptors. In particular, compounds of the present invention have γ2/γ1 selectivity ratio defined as "Kᵢ α1β3γ2 (nM) / Kᵢ α2β2γ1 (nM)" above 10-fold, or LogSel defined as "Log[Ki α1β3γ2 (nM) / Kᵢ α2β2γ1 (nM)]" above 1. Representative test results, obtained by the above described assay measuring binding affinity to HEK293 cells expressing human (h) receptors, are shown in the Table 1 below.

**Table 1**

| **Example** | **Ki h-GABA_{A} α5β2γ1 (nM)** | **Ki h-GABA_{A} α2β2γ1 (nM)** | **Ki h-GABA_{A} α1β2γ1 (nM)** | **Ki h-GABA_{A} α1β3γ2 (nM)** | **γ2/γ1 Selectivity Ratio** | **LogSel** |
|---|---|---|---|---|---|---|
| **1** | 1.1 | 8.0 | 11.3 | 146.2 | 18.3 | 1.26 |
| **2** | 5.7 | 47.9 | 77.5 | 485.6 | 10.1 | 1.01 |
| **3** | 1.5 | 8.8 | 26.1 | 452.9 | 51.2 | 1.71 |
| **4** | 5.4 | 65.8 | 102.9 | 794.9 | 12.1 | 1.08 |
| **5** | 0.8 | 6.6 | 8.2 | 137.3 | 20.8 | 1.32 |
| **6** | 12.7 | 93.3 | 236.7 | 964.7 | 10.3 | 1.01 |
| **7** | 0.4 | 8.5 | 9.1 | 205.0 | 24.2 | 1.38 |
| **8** | 2.3 | 47.6 | 43.8 | 576.4 | 12.1 | 1.08 |
| **9** | 0.7 | 8.0 | 7.3 | 396.4 | 49.7 | 1.70 |
| **10** | 1.4 | 9.5 | 25.8 | 645.3 | 67.7 | 1.83 |
| **11** | 8.3 | 49.1 | 60.3 | 690.3 | 14.1 | 1.15 |
| **12** | 2.3 | 10.4 | 17.6 | 490.1 | 47.3 | 1.67 |
| **13** | 1.1 | 6.2 | 6.2 | 331.3 | 53.5 | 1.73 |
| **14** | 0.8 | 3.4 | 5.2 | 298.6 | 86.6 | 1.94 |
| **15** | 0.9 | 8.7 | 11.5 | 248.7 | 28.7 | 1.46 |
| **16** | 1.8 | 26.5 | 22.7 | 665.5 | 25.1 | 1.40 |
| **17** | 1.3 | 19.8 | 47.7 | 492.7 | 24.9 | 1.40 |
| **18** | 2.6 | 21.6 | 54.1 | 864.5 | 40.1 | 1.60 |
| **19** | 2.5 | 26.0 | 43.9 | 829.4 | 31.9 | 1.50 |
| **20** | 2.9 | 23.8 | 37.1 | 289.2 | 12.2 | 1.09 |
| **21** | 4.6 | 52.0 | 90.5 | 1791.6 | 34.5 | 1.54 |
| **22** | 4.3 | 37.4 | 68.7 | 2792.3 | 74.6 | 1.87 |
| **23** | 5.8 | 54.5 | 63.9 | 1200.5 | 22.0 | 1.34 |
| **24** | 3.5 | 24.3 | 43.6 | 607.7 | 25.0 | 1.40 |
| **25** | 4.4 | 49.2 | 68.6 | 1189.0 | 24.2 | 1.38 |
| **26** | 2.3 | 31.6 | 24.1 | 698.4 | 22.1 | 1.34 |
| **27** | 6.3 | 84.5 | 287.6 | 4798.0 | 56.7 | 1.75 |
| **28** | 1.4 | 9.4 | 12.6 | 292.6 | 31.1 | 1.49 |
| **29** | 7.2 | 47.4 | 66.2 | 788.8 | 16.6 | 1.22 |
| **33** | 1.5 | 12.7 | ND | 232.0 | 18.2 | 1.26 |
| **34** | 0.3 | 10.1 | 5.6 | 161.3 | 15.9 | 1.20 |
| **36** | 1.8 | 8.5 | 11.9 | 179.7 | 21.2 | 1.33 |
| **37** | 2.1 | 41.4 | ND | 1181.1 | 28.5 | 1.45 |
| **38** | 1.9 | 12.6 | 28.0 | 1428.5 | 113.4 | 2.05 |
| **39** | 0.8 | 4.2 | 4.7 | 360.4 | 85.8 | 1.93 |
| **40** | 1.9 | 26.8 | 20.8 | 1007.5 | 37.5 | 1.57 |
| **41** | 0.8 | 5.5 | 4.8 | 120.6 | 21.9 | 1.34 |
| **42** | 0.9 | 5.5 | 7.2 | 243.1 | 44.2 | 1.65 |
| **43** | 1.4 | 5.8 | 8.4 | 509.6 | 88.5 | 1.95 |
| **44** | 1.4 | 9.6 | 11.5 | 275.7 | 28.6 | 1.46 |
| **45** | 3.0 | 15.8 | 54.4 | 1224.4 | 77.7 | 1.89 |
| **46** | 3.9 | 25.3 | 60.5 | 1160.8 | 45.8 | 1.66 |
| **47** | 1.3 | 15.9 | 7.0 | 222.0 | 14.0 | 1.15 |
| **48** | 1.1 | 8.3 | 20.8 | 952.1 | 114.1 | 2.06 |
| **49** | 1.7 | 14.1 | 40.5 | 1474.9 | 104.3 | 2.02 |
| **50** | 1.0 | 4.7 | 8.9 | 73.8 | 15.8 | 1.20 |
| **54** | 3.8 | 30.2 | 120.7 | 2074.0 | 68.7 | 1.84 |
| **55** | 5.5 | 18.5 | 133.1 | 1718.1 | 92.9 | 1.97 |
| **57** | 4.6 | 57.9 | 169.8 | 2114.9 | 36.5 | 1.56 |
| **58** | 3.6 | 33.1 | 131.0 | 1303.8 | 39.4 | 1.60 |
| **59** | 5.9 | 26.3 | 54.0 | 576.6 | 21.9 | 1.34 |
| **60** | 7.5 | 38.9 | 161.1 | 1114.1 | 28.6 | 1.46 |
| **61** | 4.2 | 77.6 | 118.9 | 2142.4 | 27.6 | 1.44 |
| **62** | 4.0 | 55.3 | 160.9 | 2153.1 | 38.9 | 1.59 |
| **65** | 7.8 | 64.9 | 109.5 | 1555.2 | 24.0 | 1.38 |
| **69** | 7.1 | 63.6 | 139.2 | 10574.5 | 166.3 | 2.22 |
| **70** | 6.8 | 41.4 | 274.7 | 5978.4 | 144.5 | 2.16 |
| **71** | 5.7 | 37.6 | 177.8 | 1080.5 | 28.7 | 1.46 |
| **72** | 5.4 | 30.1 | 274.3 | 10300.5 | 342.1 | 2.53 |
| **77** | 2.8 | 19.6 | 41.5 | 715.7 | 36.6 | 1.56 |
| **78** | 7.8 | 50.8 | 67.7 | 4438.4 | 87.4 | 1.94 |
| **79** | 6.8 | 51.2 | 80.1 | 914.4 | 17.8 | 1.25 |
| **90** | 1.0 | 5.8 | ND | 305.1 | 52.8 | 1.72 |
| **91** | 3.1 | 36.3 | 51.3 | 1335.9 | 36.8 | 1.57 |
| **104** | 0.6 | 2.3 | 4.2 | 89.3 | 38.1 | 1.58 |

### Functional expression of GABA_{A} receptors:

### Xenopus oocytes preparation

Xenopus laevis oocytes at maturation stages V-VI were used for the expression of cloned mRNA encoding GABA_{A} receptor subunits. Oocytes ready for RNA micro-injection were bought from Ecocyte, Castrop-Rauxel, Germany and stored in modified Barth's medium (composition in mM: NaCl 88, KCl 1, NaHCO₃ 2.4, HEPES 10, MgSO₄ 0.82, CaNO₃ 0.33, CaCl₂ 0.33, pH = 7.5) at 20 °C until the experiment.

### Xenopus oocytes microinjection

Oocytes were plated in 96-well plates for microinjection using the Roboinject automated instrument (MultiChannelSystems, Reutlingen, Germany). Approximately 50 nL of an aqueous solution containing the RNA transcripts for the subunits of the desired GABA_{A} receptor subtype was injected into each oocyte. RNA concentrations ranged between 20 and 200 pg/µL/subunit and were adjusted in pilot experiments to obtain GABA responses of a suitable size and a maximal effect of Flunitrazepam, Triazolam and Midazolam, reference benzodiazepine positive allosteric modulators (PAM) at the GABA_{A} receptor benzodiazepine (BZD) binding site. Oocytes were kept in modified Barth's medium (composition in mM: NaCl 88, KCl 1, NaHCO₃ 4, HEPES 10, MgSO₄ 0.82, CaNO₃ 0.33, CaCl₂ 0.33, pH = 7.5) at 20°C until the experiment.

### Electrophysiology

Electrophysiological experiments were performed using the Roboocyte instrument (MultiChannelSystems, Reutlingen, Germany) on days 3 to 5 after the micro-injection of mRNA. During the experiment the oocytes were constantly superfused by a solution containing (in mM) NaCl 90, KCl 1, HEPES 5, MgCl₂ 1, CaCl₂ 1 (pH 7.4). Oocytes were impaled by two glass microelectrodes (resistance: 0.5-0.8 MΩ) which were filled with a solution containing KCl 1M + K-acetate 1.5 M and voltage-clamped to -80 mV. The recordings were performed at room temperature using the Roboocyte two-electrode voltage clamp system (Multichannelsystem). After an initial equilibration period of 1.5 min GABA was added for 1.5 min at a concentration evoking approximately 20% of a maximal current response (EC₂₀). After another rest interval of 2.5 min GABA was again added evoking a response of similar amplitude and shape. 0.5 min after the onset of this second GABA application the test compound, at a concentration corresponding to approximatively 30-fold its Kᵢ α2β2γ1, was added while GABA was still present. Current traces were recorded at a digitization rate of 10 Hz during and shortly before and after the GABA application.

Each compound and concentration was tested on at least 3 oocytes. Different oocytes were used for different compound concentrations. The reference PAMs, Flunitrazepam, Triazolam and Midazolam, potentiated the GABA-induced current in α2β2γ1 GABA_{A} receptor subtype expressing oocytes by approximatively 60%.

### Data analysis

For the analysis, the digitized current traces of the first and second GABA response were superimposed and, if necessary, rescaled to equal maximal amplitudes. The ratio between the two responses during the time interval of test compound application was calculated point by point. The extremum of the resulting "ratio trace" was taken as the efficacy ("Fold increase") of the compound expressed as "% modulation of GABA EC₂₀" (100* (Fold increase-1)).

The results are shown in Table 2.

**Table 2**

| **Example** | **Ki h-GABA_{A} α2β2γ1 (nM)** | **Fold increase h-GABA-A α2β2γ1 oocyte @ 30-fold Ki** | **Efficacy (GABA)%** |
|---|---|---|---|
| **1** | 8.0 | 1.77 | 77 |
| **2** | 47.9 | 1.84 | 84 |
| **3** | 8.8 | 2.13 | 113 |
| **4** | 65.8 | 1.73 | 73 |
| **5** | 6.6 | 1.57 | 57 |
| **6** | 93.3 | 2.06 | 106 |
| **7** | 8.5 | 1.56 | 56 |
| **8** | 47.6 | 1.58 | 58 |
| **9** | 8.0 | 1.72 | 72 |
| **10** | 9.5 | 1.69 | 69 |
| **11** | 49.1 | 1.60 | 60 |
| **12** | 10.4 | 1.49 | 49 |
| **13** | 6.2 | 1.52 | 52 |
| **14** | 3.4 | 1.71 | 71 |
| **15** | 8.7 | 1.60 | 60 |
| **16** | 26.5 | 1.68 | 68 |
| **17** | 19.8 | 1.70 | 70 |
| **18** | 21.6 | 1.70 | 70 |
| **19** | 26.0 | 1.72 | 72 |
| **20** | 23.8 | 1.57 | 57 |
| **21** | 52.0 | 2.00 | 100 |
| **22** | 37.4 | 1.86 | 86 |
| **23** | 54.5 | 1.61 | 61 |
| **24** | 24.3 | 1.85 | 85 |
| **25** | 49.2 | 1.84 | 84 |
| **26** | 31.6 | 1.45 | 45 |
| **27** | 84.5 | 1.71 | 71 |
| **28** | 9.4 | 1.59 | 59 |
| **29** | 47.4 | 2.05 | 105 |
| **33** | 12.7 | 1.80 | 80 |
| **34** | 10.1 | 1.81 | 81 |
| **36** | 8.5 | 1.33 | 33 |
| **37** | 41.4 | 1.67 | 67 |
| **38** | 12.6 | 2.14 | 114 |
| **39** | 4.2 | 2.33 | 133 |
| **40** | 26.8 | 1.75 | 75 |
| **41** | 5.5 | 1.70 | 70 |
| **42** | 5.5 | 1.97 | 97 |
| **43** | 5.8 | 1.23 | 23 |
| **44** | 9.6 | 1.62 | 62 |
| **45** | 15.8 | 9.28 | 828 |
| **46** | 25.3 | 1.55 | 55 |
| **47** | 15.9 | 4.14 | 314 |
| **48** | 8.3 | 2.01 | 101 |
| **49** | 14.1 | 1.57 | 57 |
| **50** | 4.7 | 1.54 | 54 |
| **54** | 30.2 | 7.02 | 602 |
| **55** | 18.5 | 1.59 | 59 |
| **57** | 57.9 | 2.49 | 149 |
| **58** | 33.1 | 1.67 | 67 |
| **59** | 26.3 | 2.15 | 115 |
| **60** | 38.9 | 2.32 | 132 |
| **61** | 77.6 | 1.61 | 61 |
| **62** | 55.3 | 1.63 | 63 |
| **65** | 64.9 | 2.14 | 114 |
| **69** | 63.6 | 2.36 | 136 |
| **70** | 41.4 | 1.78 | 78 |
| **71** | 37.6 | 1.99 | 99 |
| **72** | 30.1 | 7.30 | 630 |
| **77** | 19.6 | 1.66 | 66 |
| **78** | 50.8 | 2.03 | 103 |
| **79** | 51.2 | 1.79 | 79 |
| **90** | 5.8 | 1.65 | 65 |
| **91** | 36.3 | 1.58 | 58 |
| **104** | 2.3 | 1.94 | 94 |

### Reference compounds

The reference compounds (classical marketed benzodiazepines) and their structural analogues listed below were also tested for their affinity towards the GABA_{A} receptor α.1β2γ1 and α2β2γ1subtypes as well as in the GABA_{A} receptor α1β3γ2 subtype. The results are shown in Table 3.

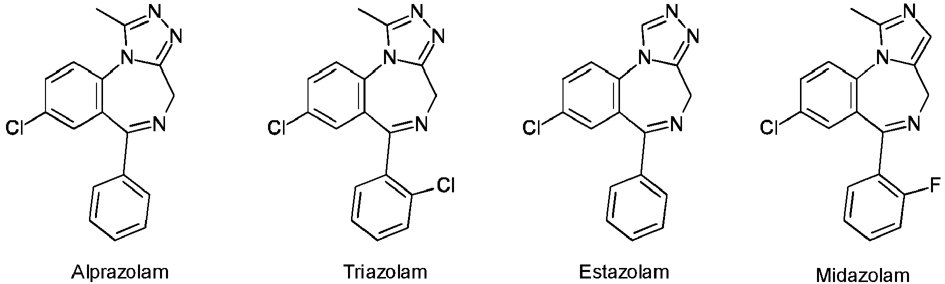

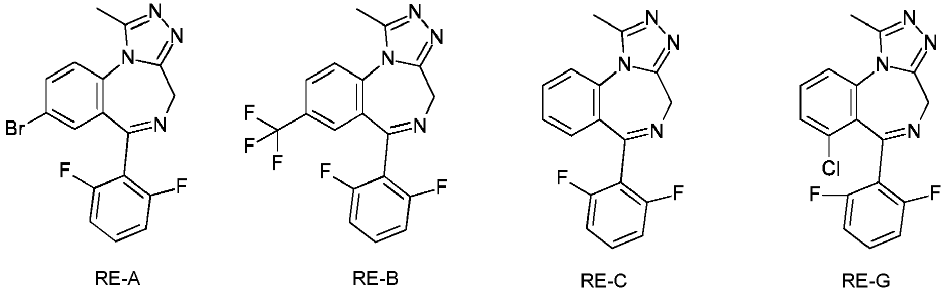

**Table 3**

| **Example** | **Ki H-GABA_{A} α1β2γ1 (nM)** | **Ki H-GABA_{A} α2β2γ1 (nM)** | **Ki H-GABA_{A} α1β3γ2 (nM)** | **γ2/γ1 Selectivity Ratio** | **LogSel** |
|---|---|---|---|---|---|
| **Alprazolam** | 5923 | 3945 | 19.6 | 0.0050 | -2.3 |
| **Triazolam** | 44.2 | 46.2 | 1.5 | 0.032 | -1.5 |
| **Estazolam** | ND | 3182 | 28.4 | 0.0089 | -2.0 |
| **Midazolam** | 1153.2 | 737.7 | 5.0 | 0.0068 | -2.2 |
| **RE-A** | ND | 32.1 | 5.6 | 0.18 | -0.74 |
| **RE-B** | ND | 68.4 | 15.6 | 0.23 | -0.64 |
| **RE-C** | ND | 626.7 | 5.8 | 0.0092 | -2.0 |
| **RE-G** | ND | 30.8 | 301.8 | 9.8 | 0.99 |
| **Example 1** | 11.3 | 8.0 | 146.2 | 18.3 | 1.3 |
| **Example 40** | 20.8 | 26.8 | 1007.5 | 37.5 | 1.6 |
| **Example 14** | 5.2 | 3.4 | 298.6 | 86.6 | 1.9 |

The compounds of formula (I) and their pharmaceutically acceptable salts can be used as medicaments (e.g. in the form of pharmaceutical preparations). The pharmaceutical preparations can be administered internally, such as orally (e.g. in the form of tablets, coated tablets, dragees, hard and soft gelatin capsules, solutions, emulsions or suspensions), nasally (e.g. in the form of nasal sprays), rectally (e.g. in the form of suppositories) or topical ocularly (e.g. in the form of solutions, ointments, gels or water soluble polymeric inserts). However, the administration can also be effected parenterally, such as intramuscularly, intravenously, or intraocularly (e.g. in the form of sterile injection solutions).

The compounds of formula (I) and their pharmaceutically acceptable salts can be processed with pharmaceutically inert, inorganic or organic adjuvants for the production of tablets, coated tablets, dragees, hard gelatin capsules, injection solutions or topical formulations Lactose, corn starch or derivatives thereof, talc, stearic acid or its salts etc. can be used, for example, as such adjuvants for tablets, dragees and hard gelatin capsules.

Suitable adjuvants for soft gelatin capsules, are, for example, vegetable oils, waxes, fats, semi-solid substances and liquid polyols, etc.

Suitable adjuvants for the production of solutions and syrups are, for example, water, polyols, saccharose, invert sugar, glucose, etc.

Suitable adjuvants for injection solutions are, for example, water, alcohols, polyols, glycerol, vegetable oils, etc.

Suitable adjuvants for suppositories are, for example, natural or hardened oils, waxes, fats, semi-solid or liquid polyols, etc.

Suitable adjuvants for topical ocular formulations are, for example, cyclodextrins, mannitol or many other carriers and excipients known in the art.

Moreover, the pharmaceutical preparations can contain preservatives, solubilizers, viscosity-increasing substances, stabilizers, wetting agents, emulsifiers, sweeteners, colorants, flavorants, salts for varying the osmotic pressure, buffers, masking agents or antioxidants. They can also contain still other therapeutically valuable substances.

The dosage can vary in wide limits and will, of course, be fitted to the individual requirements in each particular case. In general, in the case of oral administration a daily dosage of about 0.1 mg to 20 mg per kg body weight, preferably about 0.5 mg to 4 mg per kg body weight (e.g. about 300 mg per person), divided into preferably 1-3 individual doses, which can consist, for example, of the same amounts, should it be appropriate. In the case of topical administration, the formulation can contain 0.001% to 15% by weight of medicament and the required dose, which can be between 0.1 and 25 mg in can be administered either by single dose per day or per week, or by multiple doses (2 to 4) per day, or by multiple doses per week It will, however, be clear that the upper or lower limit given herein can be exceeded when this is shown to be indicated.

**Preparation of pharmaceutical compositions comprising compounds of the invention:**

**Tablets of the following composition are manufactured in the usual manner:**

| **Ingredient** | **mg/tablet** | | | |
|---|---|---|---|---|
| | 5 | 25 | 100 | 500 |
| Compound of formula I | 5 | 25 | 100 | 500 |
| Lactose Anhydrous DTG | 125 | 105 | 30 | 150 |
| Sta-Rx 1500 | 6 | 6 | 6 | 60 |
| Microcrystalline Cellulose | 30 | 30 | 30 | 450 |
| Magnesium Stearate | 1 | 1 | 1 | 1 |
| Total | 167 | 167 | 167 | 831 |

### Manufacturing Procedure

1. Mix ingredients 1, 2, 3 and 4 and granulate with purified water.
2. Dry the granules at 50°C.
3. Pass the granules through suitable milling equipment.
4. Add ingredient 5 and mix for three minutes; compress on a suitable press.

**Capsules of the following composition are manufactured:**

| **Ingredient** | **mg/capsule** | | | |
|---|---|---|---|---|
| | 5 | 25 | 100 | 500 |
| Compound of formula I | 5 | 25 | 100 | 500 |
| Hydrous Lactose | 159 | 123 | 148 | - |
| Corn Starch | 25 | 35 | 40 | 70 |
| Talk | 10 | 15 | 10 | 25 |
| Magnesium Stearate | 1 | 2 | 2 | 5 |
| Total | 200 | 200 | 300 | 600 |

### Manufacturing Procedure

1. Mix ingredients 1, 2 and 3 in a suitable mixer for 30 minutes.
2. Add ingredients 4 and 5 and mix for 3 minutes.
3. Fill into a suitable capsule.

A compound of formula I lactose and corn starch are firstly mixed in a mixer and then in a comminuting machine. The mixture is returned to the mixer; the talc is added thereto and mixed thoapproximatively. The mixture is filled by machine into suitable capsules, e.g. hard gelatin capsules.

**Injection solutions of the following composition are manufactured:**

| **Ingredient** | **mg/injection solution.** |
|---|---|
| Compound of formula I | 3 |
| Polyethylene Glycol 400 | 150 |
| acetic acid | q.s. ad pH 5.0 |
| water for injection solutions | ad 1.0 ml |

The invention is illustrated hereinafter by Examples, which have no limiting character.

In case the preparative examples are obtained as a mixture of enantiomers, the pure enantiomers can be obtained by methods described herein or by methods known to those skilled in the art, such as e.g. chiral chromatography or crystallization.

### Examples

### Building block A

### 7-bromo-6-chloro-5-(2,6-difluorophenyl)-1,3-dihydro-1,4-benzodiazepin-2-one

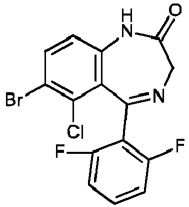

### a) 5-chloro-2-methyl-3,1-benzoxazin-4-one

A solution of 2-amino-6-chlorobenzoic acid (250 g, 1.46 mol) in acetic anhydride (1250 mL) was stirred at 140 °C for 2 h. The reaction mixture was concentrated *in vacuo.* The resulting crude residue was suspended in ethyl acetate (1000 mL), stirred for 30 min, filtered and dried *in vacuo* to afford the title compound (238 g, 84 %) as a grey solid. ¹H NMR (DMSO-*d₆*, 400 MHz): δ: 7.80 (app t*, J* = 8.0 Hz, 1H), 7.62 (d, *J* = 8.0 Hz, 1H), 7.49 (d, *J* = 7.6 Hz, 1H), 2.36 (s, 3H).

### b) N-[3-chloro-2-(2,6-difluorobenzoyl)phenyl]acetamide

To a solution of 5-chloro-2-methyl-3,1-benzoxazin-4-one (100 g, 511.2 mmol) and 2-bromo-1,3-difluorobenzene (118.4 g, 613.5 mmol) in tetrahydrofuran (1000 mL) was added dropwise *i-*PrMgCl·LiCl (1.3 M, 500 mL, 650 mmol) at -70 °C under nitrogen. The mixture was allowed to warm up to room temperature within 1 h, quenched with saturated aqueous ammonium chloride (1500 mL) and extracted with ethyl acetate (2 x 1500 mL). The organic phase was washed with brine (2000 mL), dried over sodium sulfate and concentrated *in vacuo.* The residue was suspended in ethyl acetate (150 mL). The resulting suspension was stirred at room temperature for 20 min, filtered and dried *in vacuo* to afford the title compound (113 g, 71 %) as an off-white solid. ¹H NMR (DMSO-*d₆*, 400 MHz): δ: 9.85 (s, 1H), 7.65-7.45 (m, 1H), 7.40 (t, *J* = 7.2 Hz, 1H), 7.38-7.34 (m, 2H), 7.16 (t, *J* = 8.8 Hz, 2H), 1.85 (s, 3H).

### c) (2-amino-6-chloro-phenyl)-(2,6-difluorophenyl)methanone

To a solution of *N*-[3-chloro-2-(2,6-difluorobenzoyl)phenyl]acetamide (113 g, 364.9 mmol) in ethanol (250 mL) was added aqueous hydrochloric acid (12 M, 200 mL). The reaction mixture was stirred at 100 °C for 1 h, then diluted with ethyl acetate (1100 mL). The organic phase was washed with water (1100 mL), saturated aqueous sodium bicarbonate (1100 mL) and brine (1100 mL), dried over sodium sulfate and concentrated *in vacuo.* Petroleum ether (120 mL) was added to the crude and the suspension was stirred at room temperature for 20 min. The solid was filtered and dried to afford the title compound (88 g, 90 %) as a yellow solid. ¹H NMR (DMSO-*d₆,* 400 MHz): δ: 7.62-7.56 (m, 1H), 7.21-7.15 (m, 3H), 6.83 (d, *J* = 7.6 Hz, 1H), 6.74 (s, 2H), 6.58 (d, *J* = 7.6 Hz, 1H).

### d) (6-amino-3-bromo-2-chloro-phenyl)-(2,6-difluorophenyl)methanone

To a solution of (2-amino-6-chloro-phenyl)-(2,6-difluorophenyl)methanone (88.0 g, 328.8 mmol) in dichloromethane (225 mL) and *N*,*N*-dimethylformamide (225 mL) was added 1-bromopyrrolidine-2,5-dione (64.4 g, 362 mmol) at 0 °C. The reaction mixture was stirred at 30 °C for 1 h. The mixture was diluted with dichloromethane (600 mL) and washed with water (500 mL) and brine (4 × 500 mL), dried over sodium sulfate and concentrated *in vacuo.* The residue was purified by chromatography (silica, petroleum ether / ethyl acetate, 1:0 to 2:1). The solid was suspended in petroleum ether (200 mL) and stirred at room temperature for 20 min. The suspension was filtered and the solid was dried *in vacuo* to afford the title compound (96.0 g, 84 %) as a yellow solid. MS: 345.9 ([{⁷⁹Br, ³⁵Cl}M+H]⁺), 347.8 ([{⁸¹Br, ³⁵Cl or ⁷⁹Br, ³⁷Cl}M+H]⁺), ESI pos.

### e) 7-bromo-6-chloro-5-(2,6-difluorophenyl)-1,3-dihydro-1.4-benzodiazepin-2-one

To a solution of (6-amino-3-bromo-2-chloro-phenyl)-(2,6-difluorophenyl)methanone (25.0 g, 72.1 mmol) in pyridine (625 mL) was added ethyl 2-aminoacetate hydrochloride (70.5 g, 505 mmol). The reaction mixture was stirred at 135 °C for 36 h. The reaction mixture was concentrated *in vacuo* to remove pyridine. The residue was diluted with ethyl acetate (2000 mL) and washed with aqueous HCl (1.0 M, 3 × 1500 mL), water (2000 mL) and brine (2 × 1000 mL), dried over sodium sulfate, filtered and concentrated *in vacuo.* The crude product was purified by flash column chromatography (silica, petroleum ether / ethyl acetate 10:1 to 2:1) to afford the title compound (10.1 g, 12 %) as an off-white solid. MS: 385.0 ([⁷⁹Br, ³⁵Cl}M+H]⁺), ESI pos.

### Building block B

### 7-bromo-6-chloro-5-(2-fluorophenyl)-1,3-dihydro-1,4-benzodiazepin-2-one

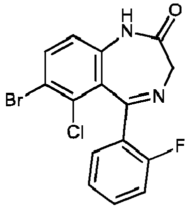

### a) N-[3-chloro-2-(2-fluorobenzoyl)phenyl]acetamide

To a solution of 5-chloro-2-methyl-3,1-benzoxazin-4-one (20.0 g, 102.3 mmol) and 1-bromo-2-fluorobenzene (17.9 g, 102.3 mmol) in tetrahydrofuran (600 mL) at -70 °C was added dropwise *n*-BuLi in tetrahydrofuran (2.5 M, 49 mL, 123 mmol). The reaction mixture was stirred at -60 °C for 1 h, then quenched with aqueous ammonium chloride (200 mL). The aqueous layer was extracted with tetrahydrofuran (2 × 250 mL) and ethyl acetate (2 × 250 mL). The combined organic phase was washed with brine (200 mL), dried over sodium sulfate and concentrated *in vacuo.* Purification by flash column chromatography (silica, petroleum ether / ethyl acetate 20:1 to 3:1) afforded the title compound (21 g, 70 %) as a white solid. MS: 292.3 ([M+H]⁺), ESI pos.

### b) (2-amino-6-chloro-phenyl)-(2-fluorophenyl)methanone

In analogy to experiment of building block A c, *N*-[3-chloro-2-(2-fluorobenzoyl)phenyl]acetamide was converted into the title compound (10 g, 58 %) which was obtained as a yellow solid. MS: 250.1 ([M+H]⁺), ESI pos.

### c) (6-amino-3-bromo-2-chloro-phenyl)-(2-fluorophenyl)methanone

In analogy to experiment of building block A d, (2-amino-6-chloro-phenyl)-(2-fluorophenyl)methanone was converted into the title compound (32.4 g, 70 %) which was obtained as a yellow solid. MS: 327.9 ⁷⁹Br, ³⁵Cl}M+H]⁺), 330.0 ([{⁸¹Br, ³⁵Cl or ⁷⁹Br, ³⁷Cl }M+H]⁺), ESI pos.

### d) 7-bromo-6-chloro-5-(2-fluorophenyl)-1,3-dihydro-1,4-benzodiazepin-2-one

To a solution of (6-amino-3-bromo-2-chloro-phenyl)-(2-fluorophenyl)methanone (35.0 g, 98.3 mmol) in pyridine (210 mL) was added ethyl 2-aminoacetate hydrochloride (96.0 g, 688 mmol) at 90 °C. The reaction mixture was stirred at 110 °C for 16 h. The reaction mixture was cooled down to room temperature and most of pyridine was removed *in vacuo.* The residue was diluted with ethyl acetate (1250 mL). The organic phase was washed with aqueous HCl (1.0 M, 1250 mL), water (500 mL) and brine (1000 mL), dried over sodium sulfate, filtered and concentrated *in vacuo.* The crude was purified by flash column chromatography (silica, petroleum ether / ethyl acetate 1:0, 25:1, 1:1). The product was dissolved in ethyl acetate (15 mL). Petroleum ether (45 mL) was added dropwise to get a white slurry. The solid was collected by filtration and dried *in vacuo* to afford the title compound (30.4 g, 39 %) as an off-white solid. MS: 367.0 ([⁷⁹Br, ³⁵Cl}M+H]⁺), 368.9 ([{⁸¹Br, ³⁵Cl or ⁷⁹Br, ³⁷Cl }M+H]⁺), ESI pos.

### Building block C

### 6,7-dichloro-5-(2-fluorophenyl)-1,3-dihydro-1,4-benzodiazepin-2-one

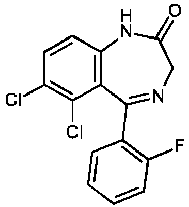

### a) (6-amino-2,3-dichloro-phenyl)-(2-fluorophenyl)methanone

In analogy to experiment of building block A d, (2-amino-6-chloro-phenyl)-(2-fluorophenyl)methanone using 1-chloropyrrolidine-2,5-dione was converted into the title compound (234 mg, 53 %) which was obtained as an orange yellow solid. MS: 284.0 ([{³⁵Cl, ³⁵Cl}M+H]⁺), ESI pos.

### b) 6,7-dichloro-5-(2-fluorophenyl)-1,3-dihydro-1,4-benzodiazepin-2-one

In analogy to experiment of building block B d, (6-amino-2,3-dichloro-phenyl)-(2-fluorophenyl)methanone was converted into the title compound (193 mg, 74 %) which was obtained as a grey solid. MS: 323.2 ([{³⁵Cl, ³⁵Cl}M+H]⁺), ESI pos.

### Building block D

### 6,7-dichloro-5-(2,6-difluorophenyl)-1,3-dihydro-1,4-benzodiazepin-2-one

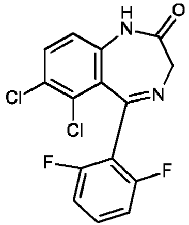

### a) (6-amino-2,3-dichloro-phenyl)-(2,6-difluorophenyl)methanone

In analogy to experiment of building block A d, (2-amino-6-chloro-phenyl)-(2,6-difluorophenyl)methanone using 1-chloropyrrolidine-2,5-dione was converted into the title compound (3.44 g, 47 %) which was obtained as a yellow solid. MS: 302.0 ([{³⁵Cl, ³⁵Cl}M+H]⁺), ESI pos.

### b) 6,7-dichloro-5-(2,6-difluorophenyl)-1,3-dihydro-1,4-benzodiazepin-2-one

In analogy to experiment of building block A e, (6-amino-2,3-dichloro-phenyl)-(2,6-difluorophenyl)methanone was converted into the title compound (2.6 g, 53 %) which was obtained as a yellow solid. MS: 341.0 ([{³⁵Cl, ³⁵Cl}M+H]⁺), ESI pos.

### Building block E

### 7-bromo-6-chloro-5-(3-fluoro-2-pyridyl)-1,3-dihydro-1,4-benzodiazepin-2-one

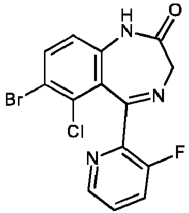

### a) N-[3-chloro-2-(3-fluoropyridine-2-carbonyl)phenyl]acetamide

To a solution of 2-bromo-3-fluoropyridine (18.0 g, 102 mmol) in methyl tert-butyl ether (720 mL) was added *n*-BuLi (2.5 m in tetrahydrofuran, 36.8 mL, 92.02 mmol) slowly at -60 °C. The mixture was stirred for 1 h, then a solution of 5-chloro-2-methyl-3,1-benzoxazin-4-one (10.0 g, 51.1 mmol) in tetrahydrofuran (600 mL) was added dropwise. The mixture was stirred at -60 °C for 1 h, then quenched by addition of saturated aqueous ammonium chloride (50 mL). The mixture was diluted with water (100 mL) and extracted with ethyl acetate (3 × 100 mL). The organic layer was washed with brine (100 mL), dried over sodium sulfate and concentrated *in vacuo.* Purification by flash column chromatography (dichloromethane / methanol 50:1 to 20:1) afforded the title compound (5.20 g, 23.2%) as a light yellow solid. MS: 293.1 ([M+H]⁺), ESI pos.

### b) (2-amino-6-chloro-phenyl)-(3-fluoro-2-pyridyl)methanone

A suspension of *N*-[3-chloro-2-(3-fluoropyridine-2-carbonyl)phenyl]acetamide (4.00 g, 13.7 mmol) in hydrochloric acid (4 m in methanol, 68 mL, 273 mmol) was stirred at 40 °C for 24 h. After cooling to room temperature a saturated aqueous solution of sodium bicarbonate was added dropwise to pH 8-9. The mixture was extracted with ethyl acetate (3 × 150 mL). The combined organic layer was washed with brine (100 mL), dried over sodium sulfate and concentrated *in vacuo.* Purification by flash column chromatography (petroleum ether / ethyl acetate 3:1 to 20:1) afforded the title compound (3.00 g, 86%) as a yellow solid. MS: 251.1 ([M+H]⁺), ESI pos.

### c) (6-amino-3-bromo-2-chloro-phenyl)-(3-fluoro-2-pyridyl)methanone

In analogy to experiment of building block A d, (2-amino-6-chloro-phenyl)-(3-fluoro-2-pyridyl)methanone was converted into the title compound (4.50 g, 74%) which was obtained as a yellow solid. MS: 328.9 ([{⁷⁹Br, ³⁵Cl}M+H]⁺), ESI pos.

### d) 7-bromo-6-chloro-5-(3-fluoro-2-pyridyl)-13-dihydro-1,4-benzodiazepin-2-one

In analogy to experiment of building block B d, (6-amino-3-bromo-2-chloro-phenyl)-(3-fluoro-2-pyridyl)methanone was converted into the title compound (550 mg, 24%) which was obtained as a pink solid. MS: 368.0 ([{⁷⁹Br, ³⁵Cl}M+H]⁺), ESI pos.

### Building block F

### 6-chloro-5-(2-fluorophenyl)-7-iodo-1,3-dihydro-1,4-benzodiazepin-2-one

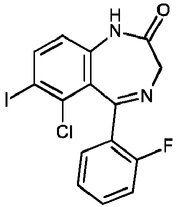

### a) (6-amino-2-chloro-3-iodo-phenyl)-(2-fluorophenyl)methanone

In analogy to experiment of building block A d, (2-amino-6-chloro-phenyl)-(2-fluorophenyl)methanone using 1-iodopyrrolidine-2,5-dione was converted into the title compound (3.2 g, 76 %) which was obtained as a brown solid. The crude was used as such in the following step without further characterization.

### b) 6-chloro-5-(2-fluorophenyl)-7-iodo-1,3-dihydro-1,4-benzodiazepin-2-one

In analogy to experiment of building block B d, (6-amino-2-chloro-3-iodo-phenyl)-(2-fluorophenyl)methanone was converted into the title compound (1.1 g, 45 %) which was obtained as a yellow solid. MS: 415.0 ([M+H]⁺), ESI pos.

### Building block H

### 6-chloro-5-(2-fluorophenyl)-7-iodo-1,3-dihydro-1,4-benzodiazepin-2-one

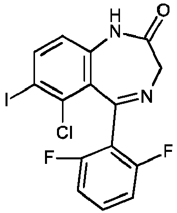

### a) (6-amino-2-chloro-3-iodo-phenyl)-(2,6-difluorophenyl)methanone

In analogy to experiment of building block A d, (2-amino-6-chloro-phenyl)-(2,6-difluorophenyl)methanone using 1-iodopyrrolidine-2,5-dione was converted into the title compound (7.1 g, 85 %) which was obtained as a yellow solid. MS: 393.8 ([M+H]⁺), ESI pos.

### b) 6-chloro-5-(2,6-difluorophenyl)-7-iodo-1,3-dihydro-1,4-benzodiazepin-2-one

In analogy to experiment of building block A e, (6-amino-2-chloro-3-iodo-phenyl)-(2,6-difluorophenyl)methanone was converted into the title compound (2.7 g, 68 %) which was obtained as a yellow solid. MS: 432.8 ([M+H]⁺), ESI pos.

### Building block I

### 6,7-dibromo-5-(2,6-difluorophenyl)-1,3-dihydro-1,4-benzodiazepin-2-one

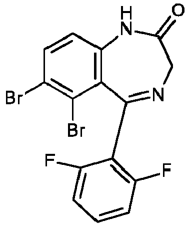

### a) 5-bromo-2-methyl-3,1-benzoxazin-4-one

In analogy to experiment of building block A a, 2-amino-6-bromobenzoic acid was converted into the title compound (15.4 g, 92 %) which was obtained as a light brown powder. MS: 240.1 ([{⁷⁹Br}M+H]⁺), ESI pos.

### b) N-[3-bromo-2-(2,6-difluorobenzoyl)phenyl]acetamide

In analogy to experiment of building block A b, 5-bromo-2-methyl-3,1-benzoxazin-4-one was converted into the title compound (8.3 g, 62 %) which was obtained as a brown solid. MS: 354.0 ([{⁷⁹Br}M+H]⁺), ESI pos.

### c) N-[3,4-dibromo-2-(2,6-difluorobenzoyl)phenyl]acetamide

To a solution of *N*-[3-bromo-2-(2,6-difluorobenzoyl)phenyl]acetamide (5.43 g, 15.3 mmol) in *N*,*N*-dimethylformamide (100 mL) and acetic acid (44 mL, 767 mmol) was added 1-bromopyrrolidine-2,5-dione (4.09 g, 23 mmol) at 0 °C. The reaction mixture was stirred at 60 °C for 22 h. After the addition of a further amount of 1-bromopyrrolidine-2,5-dione (1.36 g, 7.67 mmol), the mixture was stirred at 60 °C for 22 h. The reaction mixture was cooled to room temperature then diluted with ethyl acetate (500 mL). The organic phase was washed with water (500 mL) and brine (500 mL). The aqueous layers were back-extracted with ethyl acetate (500 mL). The combined organic extracts were dried over sodium sulfate and concentrated *in vacuo.* The residue was purified by flash column chromatography (silica, heptane / ethyl acetate, 1:0 to 1:1) to afford the title compound (6.18 g, 93 %) as a yellow solid. MS: 431.9 ([⁷⁹Br, ⁷⁹Br}M+H]⁺), 433.9 ([{⁷⁹Br, ⁸¹Br}M+H]⁺), ESI pos.

### d) (6-amino-2.3-dibromo-phenyl)-(2.6-difluorophenyl)methanone

In analogy to experiment of building block A c, *N*-[3,4-dibromo-2-(2,6-difluorobenzoyl)phenyl]acetamide was converted into the title compound (3.93 g, 70 %) which was obtained as a yellow solid. MS: 389.9 ([{⁷⁹Br, ⁷⁹Br}M+H]⁺), 391.8 ⁷⁹Br, ⁸¹Br}M+H]⁺), ESI pos.

### e) 6,7-dibromo-5-(2,6-difluorophenyl)-1,3 -dihydro-1,4-benzodiazepin-2-one

In analogy to experiment of building block A e, (6-amino-2,3-dibromo-phenyl)-(2,6-difluorophenyl)methanone was converted into the title compound (270 mg, 37 %) which was obtained as a yellow solid. MS: 429.0 ([{⁷⁹Br, ⁷⁹Br}M+H]⁺), 430.9 ([{⁷⁹Br, ⁸¹Br}M+H]⁺), ESI pos.

### Building block J

### 7-bromo-6-chloro-5-(3-chloro-2-pyridyl)-1,3-dihydro-1,4-benzodiazepin-2-one

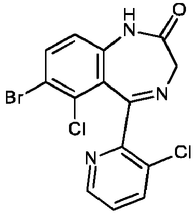

### a) N-[3-chloro-2-(3-chloropyridine-2-carbonyl)phenyl]acetamide

In analogy to experiment of building block E a, 5-chloro-2-methyl-3,1-benzoxazin-4-one using 2-bromo-3-fluoro-pyridine was converted into the title compound (3.6 g, 40 %) which was obtained as a light yellow solid. MS: 309.0 ([M+H]⁺), ESI pos.

### b) (2-amino-6-chloro-phenyl)-(3-chloro-2-pyridyl)methanone

In analogy to experiment of building block A c, *N*-[3-chloro-2-(3-chloropyridine-2-carbonyl)phenyl]acetamide was converted into the title compound (2.2 g, 71 %) which was obtained as a yellow solid. MS: 267.1 ([M+H]⁺), ESI pos.

### c) (6-amino-3-bromo-2-chloro-phenyl)-(3-chloro-2-pyridyl)methanone

In analogy to experiment of building block B c, (2-amino-6-chloro-phenyl)-(3-chloro-2-pyridyl)methanone was converted into the title compound (2.9 g, 66 %) which was obtained as a yellow solid. MS: 344.9 ([{⁷⁹Br, ³⁵Cl, ³⁵Cl}M+H]⁺), ESI pos.

### d) 7-bromo-6-chloro-5-(3-chloro-2-pyridyl)-1,3-dihydro-1,4-benzodiazepin-2-one

In analogy to experiment of building block B d, (6-amino-3-bromo-2-chloro-phenyl)-(3-chloro-2-pyridyl)methanone was converted into the title compound (380 mg, 24.%) which was obtained as a white solid. MS: 383.9 ([{⁷⁹Br, ³⁵Cl, ³⁵Cl}M+H]⁺), ESI pos.

### Building block K

### 7-bromo-6-chloro-5-(2-chloro-6-fluoro-phenyl)-1,3-dihydro-1,4-benzodiazepin-2-one

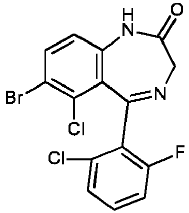

### a) N-[3-chloro-2-(2-chloro-6-fluoro-benzoyl)phenyl]acetamide

To a mixture of 1-chloro-3-fluoro-benzene (7.27 g, 55.66 mmol), diisopropylamine (512 mg, 5.06 mmol) and *N*,*N*,*N'*,*N'*-tetramethylethylenediamine (7.06 g, 60.72 mmol) in tetrahydrofuran (100 mL) was added dropwise n-BuLi in tetrahydrofuran (2.5 M, 24.3 mL, 60.7 mmol). The reaction mixture was stirred at -65 °C for 1 h. A solution of 5-chloro-2-methyl-3,1-benzoxazin-4-one (10.0 g, 50.6 mmol) in tetrahydrofuran (300 mL) was added dropwise and the reaction mixture was stirred at -65 °C for 0.5 h before being quenched by addition of saturated aqueous ammonium chloride (150 mL). The resulting mixture was diluted with ethyl acetate (200 mL). The organic phase was washed with water (100 mL) and brine (100 mL), dried over sodium sulfate and concentrated in vacuo. The residue was purified by flash column chromatography (silica, petroleum ether / ethyl acetate 10:1 to 5:1 to 3:1) to afford the title compound (2.4 g, 15 %) as a light yellow solid. MS: 325.9 ([M+H]⁺), ESI pos.

### b) (2-amino-6-chloro-phenyl)-(2-chloro-6-fluoro-phenyl)methanone

In analogy to experiment of building block A c, *N*-[3-chloro-2-(2-chloro-6-fluorobenzoyl)phenyl]acetamide was converted into the title compound (1.9 g, 91 %) which was obtained as a yellow solid. MS: 284.1 ([M+H]⁺), ESI pos.

### c) (6-amino-3-bromo-2-chloro-phenyl)-(2-chloro-6-fluoro-phenyl)methanone

In analogy to experiment of building block A d, (2-amino-6-chloro-phenyl)-(2-chloro-6-fluorophenyl)methanone was converted into the title compound (1.74 g, 72 %) which was obtained as a yellow solid. The crude was used as such in the following step without further characterization.

### d) 7-bromo-6-chloro-5-(2-chloro-6-fluoro-phenyl)-1,3-dihydro-1,4-benzodiazepin-2-one

In analogy to experiment of building block A e, (6-amino-3-bromo-2-chloro-phenyl)-(2-chloro-6-fluoro-phenyl)methanone was converted into the title compound (32.5 mg, 15 %) which was obtained as a light yellow solid. MS: 400.9 ⁷⁹Br, ³⁵Cl, ³⁵Cl}M+H]⁺), 402.8 ([{⁸¹Br, ³⁵Cl, ³⁵Cl or ⁷⁹Br, ¹⁷Cl, ³⁵Cl }M+H]⁺), ESI pos.

### Building block N

### 6,7-dichloro-5-(3-fluoro-2-pyridyl)-1,3-dihydro-1,4-benzodiazepin-2-one

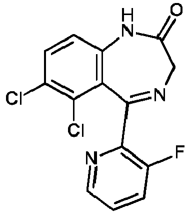

### a) N-[3,4-dichloro-2-(3-fluoropyridine-2-carbonyl)phenyl]acetamide

In analogy to experiment of building block A d, *N*-[3-chloro-2-(3-fluoropyridine-2-carbonyl)phenyl]acetamide using 1-chloropyrrolidine-2,5-dione was converted into the title compound (36 g, 84 %) which was obtained as a white solid. MS: 327.0 ([{³⁵Cl,³⁵Cl}M+H]⁺), ESI pos.

### b) (6-amino-2,3-dichloro-phenyl)-(3-fluoro-2-pyridyl)methanone

In analogy to experiment of building block E b, *N*-[3,4-dichloro-2-(3-fluoropyridine-2-carbonyl)phenyl]acetamide was converted into the title compound (11.0 g, 85 %) which was obtained as an orange solid. MS: 285.0 ([{³⁵Cl, ³⁵Cl}M+H]⁺), ESI pos.

### c) 6,7-dichloro-5-(3-fluoro-2-pyridyl)-1,3-dihydro-1,4-benzodiazepin-2-one

In analogy to experiment of building block B d, *N*-[3,4-dichloro-2-(3-fluoropyridine-2-carbonyl)phenyl]acetamide was converted into the title compound (3.76 g, 28 %) which was obtained as a white solid. MS: 323.7 ([{³⁵Cl, ³⁵Cl}M+H]⁺), ESI pos.

### Building block P

### 6-chloro-5-(3-fluoro-2-pyridyl)-7-iodo-1,3-dihydro-1,4-benzodiazepin-2-one

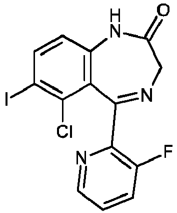

### a) (6-amino-2-chloro-3-iodo-phenyl)-(3-fluoro-2-pyridyl)methanone

In analogy to experiment of building block A d, (2-amino-6-chloro-phenyl)-(3-fluoro-2-pyridyl)methanone using 1-iodopyrrolidine-2,5-dione was converted into the title compound (28 g, 93 %) which was obtained as a yellow solid. MS: 376.9 ([M+H]⁺), ESI pos.

### b) 6-chloro-5-(3-fluoro-2-pyridyl)-7-iodo-1,3-dihydro-1,4-benzodiazepin-2-one

In analogy to experiment of building block B d, (6-amino-2-chloro-3-iodo-phenyl)-(3-fluoro-2-pyridyl)methanone was converted into the title compound (9.0 g, 58 %) which was obtained as a pink solid. MS: 416.0 ([M+H]⁺), ESI pos.

### Example 1

### 8-bromo-7-chloro-6-(2,6-difluorophenyl)-1-methyl-4H-[1,2,4]triazolo[4,3-a][1,4]benzodiazepine

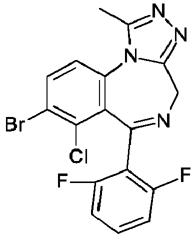

### a) 7-bromo-6-chloro-5-(2,6-difluorophenyl)-1,3-dihydro-1,4-benzodiazepin-2-thione

To a suspension of 7-bromo-6-chloro-5-(2,6-difluorophenyl)-1,3-dihydro-1,4-benzodiazepin-2-one (building block A, 6.7 g, 17.4 mmol) in toluene (167 mL) at room temperature was added Lawesson's reagent (8.43 g, 20.9 mmol). The reaction mixture was stirred at 120 °C for 1.5 h, before being diluted with ethyl acetate (400 mL). The organic layer was washed with water (300 mL) and brine (300 mL), dried over sodium sulfate, filtered and concentrated *in vacuo.* The crude material was purified by flash column chromatography (silica, 15-30 % ethyl acetate in heptane) to afford the title compound (6.98 g, 100 %) as a yellow solid. MS: 400.8 ⁷⁹Br, ³⁵Cl}M+H]⁺), 402.9 ([{⁸¹Br, ³⁵Cl or ⁷⁹Br, ³⁷Cl }M+H]⁺), ESI pos.

### b) 8-bromo-7-chloro-6-(2,6-difluorophenyl)-1-methyl-4H-[1,2,4]triazolo[4,3-a][1,4]benzodiazepine

A microwave vial was charged with 7-bromo-6-chloro-5-(2,6-difluorophenyl)-1,3-dihydro-1,4-benzodiazepin-2-thione (4.53 g, 11.3 mmol), acetohydrazide (1.67 g, 22.6 mmol) and butan-1-ol (45 mL). The vial was capped and heated in a microwave oven to 150 °C for 1 h. The reaction mixture was purified directly by flash column chromatography (silica, 0-5 % methanol in dichloromethane) to afford the title compound (3.01 g, 63 %) as a yellow foam. MS: 423.1 ([{⁷⁹Br, ³⁵Cl}M+H]⁺), 425.0 ([{⁸¹Br, ³⁵Cl or ⁷⁹Br, ³⁷Cl }M+H]⁺), ESI pos.

### Example 2

### 8-bromo-7-chloro-1-cyclopropyl-6-(2-fluorophenyl)-4H-[1,2,4]triazolo[4,3-a] [1,4]benzodiazepine

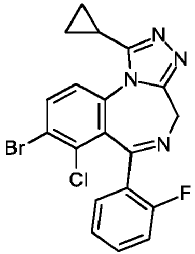

### a) 7-bromo-6-chloro-5-(2-fluorophenyl)-1-dihydro-1,4-benzodiazepine-2-thione

In analogy to experiment of example 1 a, 7-bromo-6-chloro-5-(2-fluorophenyl)-1,3-dihydro-1,4-benzodiazepin-2-one (building block B) was converted into the title compound (390 mg, 54 %) which was obtained as a yellow solid. The crude was used as such in the following step without further characterization.

### b) 8-bromo-7-chloro-1-cyclopropyl-6-(2-fluorophenyl)-4H-[1,2,4]triazolo[4,3-a][1,4]benzodiazepine

To a solution of 7-bromo-6-chloro-5-(2-fluorophenyl)-1,3-dihydro-1,4-benzodiazepine-2-thione (80 mg, 0.210 mmol) in butan-1-ol (1.5 mL) was added cyclopropanecarbohydrazide (41 mg, 0.420 mmol). The reaction mixture was stirred at 130 °C for 12 h. The mixture was diluted with ethyl acetate (100 mL), washed with water (30 mL), brine (30 mL), dried over sodium sulfate and concentrated *in vacuo.* The residue was purified by preparative HPLC (Phenomenex Gemini, 0.05 % ammonia hydroxide in water / acetonitrile) and lyophilized to afford the title compound (5.5 mg, 6 %) as a white solid. MS: 431.1 ([{⁷⁹Br, ³⁵Cl}M+H]⁺), 433.0 ([{⁸¹Br, ³⁵Cl or ⁷⁹Br, ³⁷Cl }M+H]⁺), ESI pos.

### Example 3

### 8-bromo-7-chloro-6-(2-fluorophenyl)-1-pyridazin-3-yl-4H-[1,2,4]triazolo[4,3-a] [1,4]benzodiazepine

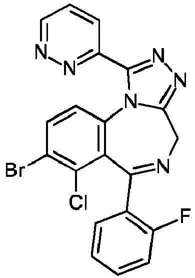

In analogy to experiment of example 1 b, 7-bromo-6-chloro-5-(2-fluorophenyl)-1,3-dihydro-1,4-benzodiazepine-2-thione using pyridazine-3-carbohydrazide was converted into the title compound (294 mg, 73 %) which was obtained as a white solid. MS: 469.1 ([⁷⁹Br, ³⁵Cl}M+H]⁺), 471.1 ([{⁸¹Br, ³⁵Cl or ⁷⁹Br, ³⁷Cl }M+H]⁺), ESI pos.

### Example 4

### 7,8-dichloro-6-(2-fluorophenyl)-1-methyl-4H-[1,2,4]triazolo[4,3-a][1,4]benzodiazepine

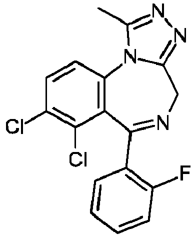

To a suspension of 6,7-dichloro-5-(2-fluorophenyl)-1,3-dihydro-1,4-benzodiazepin-2-one (building block C, 70 mg, 0.217 mmol) in 1,4-dioxane (1 mL) was added Lawesson's reagent (114 mg, 0.282 mmol). The reaction mixture was heated to 80 °C for 4 h, before being cooled down to room temperature. A solution of acetohydrazide (32.1 mg, 0.433 mmol) in 1,4-dioxane (0.5 mL) was added and the mixture was heated to 140 °C in a microwave oven for 1 h. A further amount of acetohydrazide (64.2 mg, 0.866 mmol) was added and the mixture was heated to 140 °C in a microwave oven for 1 h. The reaction mixture was diluted with ethyl acetate (10 mL) and the organic phase was washed with water (10 mL) and brine (10 mL). The aqueous layers were back-extracted with ethyl acetate (10 mL). The combined organic layers were dried over sodium sulfate and concentrated *in vacuo.* The residue was purified by flash column chromatography (silica, 0-20 % methanol in ethyl acetate) to afford the title compound (17.2 mg, 22 %) as an off-white solid. MS: 361.2 ([{³⁵Cl, ³⁵Cl}M+H]⁺), ESI pos.

### Example 5

### 7,8-dichloro-6-(2,6-difluorophenyl)-1-methyl-4H-[1,2,4]triazolo[4,3-a][1,4]benzodiazepine

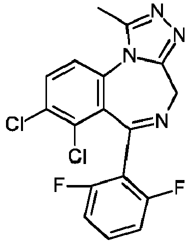

In analogy to experiment of example 4, 6,7-dichloro-5-(2,6-difluorophenyl)-1,3-dihydro-1,4-benzodiazepin-2-one (building block D) was converted into the title compound (21 mg, 32 %) which was obtained as an off-white solid. MS: 379.1 ([{³⁵Cl, ³⁵Cl}M+H]⁺), ESI pos.

### Example 6

### 7-chloro-8-cyclopropyl-6-(2,6-difluorophenyl)-1-methyl-4H-[1,2,4]triazolo[4,3-a][1,4]benzodiazepine

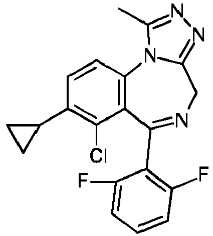

To a solution of 8-bromo-7-chloro-6-(2,6-difluorophenyl)-1-methyl-4H-[1,2,4]triazolo[4,3-a][1,4]benzodiazepine (5.29 g, 12.5 mmol) in degassed toluene (70 mL) and water (14 mL), was added cyclopropylboronic acid (1.29 g, 15 mmol), palladium (II) acetate (280 mg, 1.25 mmol), tricyclohexylphosphine (350 mg, 1.25 mmol) and potassium phosphate tribasic (7.95 g, 37.5 mmol). The mixture was heated to 100 °C for 16 h, before being diluted with ethyl acetate and water. The phases were separated then the organic layer was washed with brine, dried over sodium sulfate, filtered and concentrated *in vacuo.* The crude material was purified by flash column chromatography (silica, 0-10 % methanol in dichloromethane). The solid was recrystallized in ethyl acetate to afford the title compound (1.78 g, 35 %) as a white solid. MS: 385.2 ([M+H]⁺), ESI pos.

### Example 7

### 8-bromo-7-chloro-6-(2,6-difluorophenyl)-4H-[1,2,4]triazolo[4,3-a][1,4]benzodiazepine

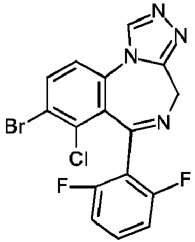

In analogy to experiment of example 2 b, 7-bromo-6-chloro-5-(2,6-difluorophenyl)-1,3-dihydro-1,4-benzodiazepin-2-thione using formohydrazide was converted into the title compound (2.4 g, 32 %) which was obtained as a white solid. MS: 409.1 ([{⁷⁹Br, ³⁵Cl}M+H]⁺), 411.1 ([{⁸¹Br, ³⁵Cl or ⁷⁹Br, ³⁷Cl }M+H]⁺), ESI pos.

### Example 8

### 8-bromo-7-chloro-6-(2-fluorophenyl)-4H-[1,2,4]triazolo[4,3-a][1,4]benzodiazepine

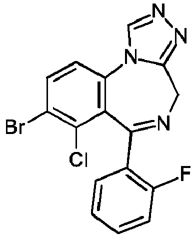

In analogy to experiment of example 2 b, 7-bromo-6-chloro-5-(2-fluorophenyl)-1,3-dihydro-1,4-benzodiazepine-2-thione using formohydrazide was converted into the title compound (35.9 mg, 20 %) which was obtained as a white solid. MS: 391.0 ([{⁷⁹Br, ³⁵Cl}M+H]⁺), 393.1 ([{⁸¹Br, ³⁵Cl or ⁷⁹Br, ³⁷Cl }M+H]⁺), ESI pos.

### Example 9

### 7,8-dichloro-6-(2,6-difluorophenyl)-2,4-dihydro-[1,2,4]triazolo[4,3-a][1,4]benzodiazepin-1-one

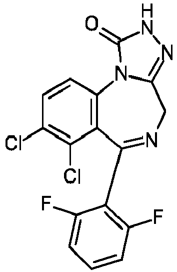

### a) 6,7-dichloro-5-(2,6-difluorophenyl)-1,3-dihydro-2H-benzo[e][1,4]diazepine-2-thione

To a solution of 6,7-dichloro-5-(2,6-difluorophenyl)-1,3-dihydro-2H-benzo[e][1,4]diazepin-2-one (building block D, 409 mg, 1.2 mmol) in diglyme (3.7 mL) was added at room temperature phosphorus pentasulfide (300 mg, 1.35 mmol), followed by sodium bicarbonate (300 mg, 3.58 mmol). The reaction mixture was heated to 80 °C for 17 h, before being cooled down to room temperature. The mixture was poured into ice (20 mL) and stirred for further 1 h, before being extracted with ethyl acetate (2 × 20 mL). The combined organic layers were washed with saturated aqueous sodium bicarbonate (1 × 20 mL), dried over sodium sulfate, filtered and concentrated *in vacuo.* The resulting dark red solution in diglyme was purified by flash column chromatography (silica, ethyl acetate in heptane 0-40 %). The final product was dried at high vacuum at 70 °C for 1 h, to afford the title compound (334 mg, 78 %) as a light red solid. MS: 357.0 ([{³⁵Cl, ³⁵Cl}M+H]⁺), ESI pos.

### b) 6,7-dichloro-5-(2,6-difluorophenyl)-1,3-dihydro-1,4-benzodiazepin-2-one hydrazone

To a solution of 6,7-dichloro-5-(2,6-difluorophenyl)-1,3-dihydro-2H-benzo[e][1,4]diazepine-2-thione (200 mg, 0.560 mmol) in propan-2-ol (0.88 mL) and tetrahydrofuran (4.4 mL) was added at room temperature hydrazine monohydrate (54.3 µL, 1.12 mmol) and the mixture was stirred at room temperature for 3 h under argon. The suspension was concentrated *in vacuo* to give a light yellow solid. The residue was taken with methyl tert-butyl ether (1 mL) and pentane (2 mL) to provide a suspension that was stirred for 10 min. The solid was collected by filtration, washed with pentane (2 × 3 mL) and dried at high vacuum to afford the title compound (199 mg, 100 %) as an orange solid. MS: 355.1 ([{³⁵Cl, ³⁵Cl}M+H]⁺), ESI pos.

### c) 7,8-dichloro-6-(2,6-difluorophenyl)-2,4-dihydro-[1,2,4]triazolo[4,3-a][1,4]benzodiazepin-1-one

To a solution of 6,7-dichloro-5-(2,6-difluorophenyl)-1,3-dihydro-1,4-benzodiazepin-2-one hydrazone (64.5 mg, 0.182 mmol) in tetrahydrofuran (1.25 mL) was added at room temperature 1,1'-carbonyldiimidazole (35.3 mg, 0.218 mmol). The reaction mixture was heated to 70 °C for 2.5 h, before being cooled to room temperature and left standing overnight. Saturated aqueous sodium bicarbonate (10 mL) was added and the mixture was extracted with ethyl acetate (2 × 10 mL). The combined organic layers were dried over sodium sulfate, filtered and concentrated *in vacuo.* The residue was purified by flash column chromatography (silica, ethyl acetate in heptane 20-100 %) to afford the title compound (33 mg, 48 %) as an off-white solid. MS: 381.1 ([{³⁵Cl, ³⁵Cl}M+H]⁺), ESI pos.

### Example 10

### 7,8-dichloro-6-(2-fluorophenyl)-1-pyridazin-3-yl-4H-[1,2,4]triazolo[4,3-a] [1,4]benzodiazepine

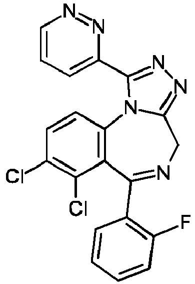

### a) 6,7-dichloro-5-(2-fluorophenyl)-1.3-dihydro-1,4-benzodiazepine-2-thione

In analogy to experiment of example 9 a, 6,7-dichloro-5-(2-fluorophenyl)-1,3-dihydro-1,4-benzodiazepin-2-one (building block C) was converted into the title compound (2.62 g, 78 %) which was obtained as an orange solid. MS: 339.0 ([{³⁵Cl,³⁵Cl}M+H]⁺), ESI pos.

### b) 7,8-dichloro-6-(2-fluorophenyl)-1-pyridazin-3-yl-4H-[1,2,4]triazolo[4,3-a][1,4]benzodiazepine

In analogy to experiment of example 1 b, 6,7-dichloro-5-(2-fluorophenyl)-1,3-dihydro-1,4-benzodiazepine-2-thione using pyridazine-3-carbohydrazide was converted into the title compound (312 mg, 70 %) which was obtained as an off-white solid. MS: 425.2 ([{³⁵Cl, ³⁵Cl}M+H]⁺), ESI pos.

### Example 11

### 7,8-dichloro-6-(2,6-difluorophenyl)-1-tetrahydropyran-4-yl-4H-[1,2,4]triazolo[4,3-a] [1,4]benzodiazepine

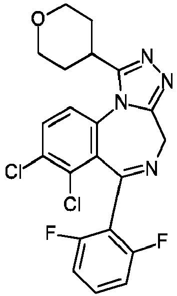

In analogy to experiment of example 1 b, 6,7-dichloro-5-(2,6-difluorophenyl)-1,3-dihydro-1,4-benzodiazepine-2-thione using tetrahydropyran-4-carbohydrazide was converted into the title compound (29 mg, 46 %) which was obtained as a white solid. MS: 449.1 ([{³⁵Cl,³⁵Cl}M+H]⁺), ESI pos.

### Example 12

### 7,8-dichloro-6-(2,6-difluorophenyl)-1-pyrimidin-4-yl-4H-[1,2,4]triazolo[4,3-a][1,4]benzodiazepine

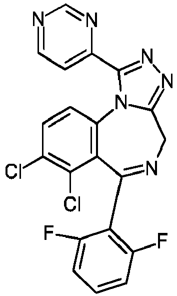

In analogy to experiment of example 1 b, 6,7-dichloro-5-(2,6-difluorophenyl)-1,3-dihydro-1,4-benzodiazepine-2-thione using pyrimidine-4-carbohydrazide was converted into the title compound (21 mg, 34 %) which was obtained as an off-white solid. MS: 443.1 ([{³⁵Cl, ³⁵Cl}M+H]⁺), ESI pos.

### Example 13

### 8-bromo-7-chloro-6-(2,6-difluorophenyl)-2,4-dihydro-[1,2,4]triazolo[4,3-a][1,4[benzodiazepin-1-one

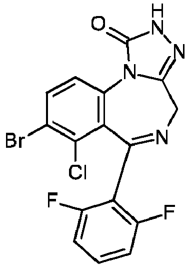

### a) 7-bromo-6-chloro-5-(2,6-difluorophenyl)-1,3-dihydro-1,4-benzodiazepin-2-one hydrazone

In analogy to experiment of example 9 b, 7-bromo-6-chloro-5-(2,6-difluorophenyl)-1,3-dihydro-1,4-benzodiazepine-2-thione was converted into the title compound (127 mg, 80 %) which was obtained as a light brown solid. MS: 399.0 ([{⁷⁹Br, ³⁵Cl}M+H]⁺), 401.1 ([{⁸¹Br, ³⁵Cl or ⁷⁹Br, ³⁷Cl }M+H]⁺), ESI pos.

### b) 8-bromo-7-chloro-6-(2,6-difluorophenyl)-2,4-dihydro-[1,2,41triazolo[4,3-a][1,4]benzodiazepin-1-one

In analogy to experiment of example 9 c, 7-bromo-6-chloro-5-(2,6-difluorophenyl)-1,3-dihydro-1,4-benzodiazepin-2-one hydrazone was converted into the title compound (51 mg, 38 %) which was obtained as a light yellow solid. MS: 425.0 ([{⁷⁹Br, ³⁵Cl}M+H]⁺), 427.0 ([{⁸¹Br, ³⁵Cl or ⁷⁹Br, ³⁷Cl }M+H]⁺), ESI pos.

### Example 14

### 7,8-dichloro-6-(2,6-difluorophenyl)-1-pyridazin-3-yl-4H-[1,2,4]triazolo[4,3-a] [1,4]benzodiazepine

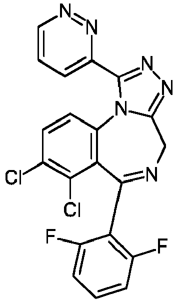

In analogy to experiment of example 1 b, 6,7-dichloro-5-(2,6-difluorophenyl)-1,3-dihydro-1,4-benzodiazepine-2-thione using pyridazine-3-carbohydrazide was converted into the title compound (33 mg, 53 %) which was obtained as an off-white solid. MS: 443.1 ([³⁵Cl, ³⁵Cl}M+H]⁺), ESI pos.

### Example 15

### 7,8-dichloro-6-(2,6-difluorophenyl)-4H-[1,2,4]triazolo[4,3-a][1,4]benzodiazepine

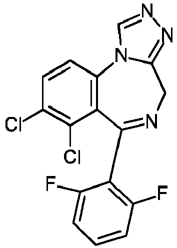

In analogy to experiment of example 1 b, 6,7-dichloro-5-(2,6-difluorophenyl)-1,3-dihydro-1,4-benzodiazepine-2-thione using formohydrazide was converted into the title compound (512 mg, 67 %) which was obtained as a light yellow solid. MS: 365.1 ([{³⁵Cl,³⁵Cl}M+H]⁺), ESI pos.

### Example 16

### 7-chloro-6-(2,6-difluorophenyl)-8-methyl-2,4-dihydro-[1,2,4]triazolo[4,3-a][1,4[benzodiazepin-1-one

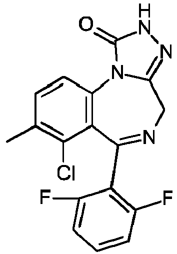

### a) 6-chloro-5-(2,6-difluorophenyl)-7-methyl-1,3-dihydro-2H-benzo[e][1,4]diazepin-2-one

A microwave vial was charged with 7-bromo-6-chloro-5-(2,6-difluorophenyl)-1,3-dihydro-1,4-benzodiazepin-2-one (building block A, 50 mg, 0.130 mmol), trimethylboroxine (19.5 mg, 21.8 µl, 0.156 mmol), potassium carbonate (26.9 mg, 0.195 mmol) and tetrakis(triphenylphosphine)palladium(0) (7.49 mg, 6.48 µmol). Degassed 1,4-dioxane (0.9 mL) and water (0.3 mL) were added. The vial was capped and heated to 130 °C for 30 min in a microwave oven. The mixture was purified directly by flash column chromatography (silica, 10-70 % ethyl acetate in heptane) to afford the title compound (24 mg, 58 %) as an off-white solid. MS: 321.1 ([M+H]⁺), ESI pos.

### b) 6-chloro-5-(2,6-difluorophenyl)-7-methyl-1,3-dihydro-2H-benzo[e][1,4]diazepine-2-thione

To a solution of 6-chloro-5-(2,6-difluorophenyl)-7-methyl-1,3-dihydro-2H-benzo[e][1,4]diazepin-2-one (96.2 mg, 0.3 mmol) in diglyme (0.9 mL) was added at room temperature phosphorus pentasulfide (75 mg, 0.337 mmol), followed by sodium bicarbonate (75.1 mg, 0.894 mmol). The reaction mixture was heated to 80 °C for 16 h, before being cooled to room temperature. The mixture was poured into ice (10 mL) and stirred for further 1 h. The resulting precipitate was filtered through a sintered funnel. The collected residue was washed with water (4 × 3 mL) and dried at high vacuum to afford the title compound (94 mg, 93 %) as a light brown solid. MS: 337.1 ([M+H]⁺), ESI pos.

### c) 6-chloro-5-(2,6-difluorophenyl)-7-methyl-1,3-dihydro-1,4-benzodiazepin-2-one hydrazone

In analogy to experiment of example 9 b, 6-chloro-5-(2,6-difluorophenyl)-7-methyl-1,3-dihydro-2H-benzo[e][1,4]diazepine-2-thione was converted into the title compound (25 mg, 63 %) which was obtained as a light yellow solid. MS: 335.1 ([M+H]⁺), ESI pos.

### d) 7-chloro-6-(2,6-difluorophenyl)-8-methyl-2,4-dihydro-[1,2,4]triazolo[4,3-a][1,4]benzodiazepin-1-one

In analogy to experiment of example 9 c, 6-chloro-5-(2,6-difluorophenyl)-7-methyl-1,3-dihydro-1,4-benzodiazepin-2-one hydrazone was converted into the title compound (17 mg, 63 %) which was obtained as a white solid. MS: 361.1 ([M+H]⁺), ESI pos.

### Example 17

### 7-chloro-6-(2,6-difluorophenyl)-8-methyl-4H-[1,2,4]triazolo[4,3-a][1,4]benzodiazepine

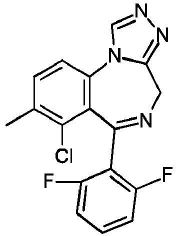

A mixture of 8-bromo-7-chloro-6-(2,6-difluorophenyl)-4H-[1,2,4]triazolo[4,3-a][1,4]benzodiazepine (110 mg, 0.270 mmol), tetrakis (triphenylphosphine) palladium (0) (31 mg, 0.030 mmol), trimethylaluminum (2.0 m in toluene, 0.2 mL, 0.410 mmol) in *N,N-*dimethylformamide (1.5 mL) was heated to 70 °C for 16 h. The reaction mixture was diluted with ethyl acetate (100 mL), washed with water (30 mL), brine (2 × 30 mL), dried over sodium sulfate and concentrated *in vacuo.* The residue was purified by preparative HPLC (Boston Prime C18, 0.1 % trifluoroacetic acid in water / acetonitrile) and lyophilized to afford the title compound (51.6 mg, 56 %) as a white solid. MS: 345.2 ([M+H]⁺), ESI pos.

### Example 18

### 7,8-dichloro-6-(2-fluorophenyl)-1-pyrimidin-4-yl-4H-[1,2,4]triazolo[4,3-a] [1,4]benzodiazepine

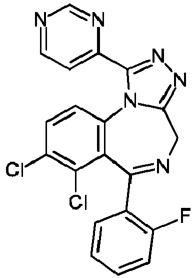

In analogy to experiment of example 1 b, 6,7-dichloro-5-(2-fluorophenyl)-1,3-dihydro-1,4-benzodiazepine-2-thione using pyrimidine-4-carbohydrazide was converted into the title compound (42 mg, 67 %) which was obtained as a light yellow solid. MS: 425.1 ([{³⁵Cl, ³⁵Cl}M+H]⁺), ESI pos.

### Example 19

### 7,8-dichloro-6-(2,6-difluorophenyl)-1-(2-methoxyethyl)-4H-[1,2,4]triazolo[4,3-a][1,4]benzodiazepine

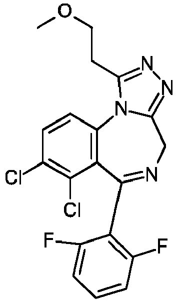

In analogy to experiment of example 1 b, 6,7-dichloro-5-(2,6-difluorophenyl)-1,3-dihydro-1,4-benzodiazepine-2-thione using 3-methoxypropanehydrazide was converted into the title compound (20 mg, 38 %) which was obtained as a white solid. MS: 423.1 ([{³⁵Cl,³⁵Cl}M+H]⁺), ESI pos.

### Example 20

### 7-chloro-6-(2,6-difluorophenyl)-1,8-dimethyl-4H-[1,2,4]triazolo[4,3-a][1,4]benzodiazepine

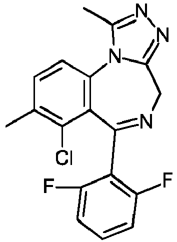

In analogy to experiment of example 17, 8-bromo-7-chloro-6-(2,6-difluorophenyl)-1-methyl-4H-[1,2,4]triazolo[4,3-a][1,4]benzodiazepine was converted into the title compound (25 mg, 29 %) which was obtained as a white solid. MS: 359.1 ([M+H]⁺), ESI pos.

### Example 21

### 7-chloro-8-(1,1-difluoroethyl)-6-(2,6-difluorophenyl)-4H-[1,2,4]triazolo[4,3-a][1,4]benzodiazepine

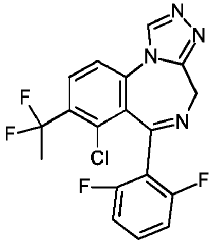

### a) 7-chloro-6-(2,6-difluorophenyl)-8-(1-ethoxyvinyl)-4H-[1,2,41triazolo[4,3-a][1,4]benzodiazepine

To a suspension of 8-bromo-7-chloro-6-(2,6-difluorophenyl)-4H-[1,2,4]triazolo[4,3-a][1,4]benzodiazepine (200 mg, 0.490 mmol) and tributyl(1-ethoxyvinyl)tin (352 mg, 0.980 mmol) in *N*,*N*-dimethylformamide (4 mL) was added tetrakis(triphenylphosphine)palladium(0) (56.42 mg, 0.050 mmol). The reaction mixture was heated to 100 °C for 16 h. The mixture was diluted with ethyl acetate (100 mL), washed with water (30 mL) and brine (30 mL), dried over sodium sulfate and concentrated *in vacuo.* The residue was purified by flash column chromatography (silica, petroleum ether / ethyl acetate 10:1, dichloromethane / methanol 80:1) to afford the title compound (160 mg, 82 %) as a yellow gum. The crude was used as such in the following step without further characterization.

### b) 1-[7-chloro-6-(2,6-difluorophenyl)-4H-[1,2,4]triazolo[4,3-a][1,4]benzodiazepin-8-yl]ethanone

To a solution of 7-chloro-6-(2,6-difluorophenyl)-8-(1-ethoxyvinyl)-4H-[1,2,4]triazolo[4,3-a][1,4]benzodiazepine (160 mg, 0.400 mmol) in 1,4-dioxane (10 mL) was added aqueous hydrochloric acid (2.0 m, 0.99 mL, 1.98 mmol). The mixture was stirred at room temperature for 0.5 h. The mixture was diluted with ethyl acetate (100 mL), washed with water (30 mL) and brine (30 mL), dried over sodium sulfate and concentrated *in vacuo.* The residue was purified by preparative HPLC (Boston Prime C18, 0.1 % trifluoroacetic acid in water / acetonitrile) and lyophilized to afford the title compound (70 mg, 47 %) as a white solid. MS: 372.9 ([M+H]⁺), ESI pos.

### c) 7-chloro-8-(1,1-difluoroethyl)-6-(2,6-difluorophenyl)-4H-[1,2,4]triazolo[4,3-a][1,4]benzodiazepine

To a solution of 1-[7-chloro-6-(2,6-difluorophenyl)-4H-[1,2,4]triazolo[4,3-a][1,4]benzodiazepin-8-yl]ethanone (60 mg, 0.160 mmol) in dichloromethane (0.2 mL) was added diethylaminosulfur trifluoride (0.8 mL). The mixture was stirred at room temperature for 120 h, before being poured into saturated aqueous sodium bicarbonate (20 mL) and extracted with dichloromethane (2 × 50 mL). The organic layer was separated and washed with brine (30 mL), dried over sodium sulfate and concentrated *in vacuo.* The residue was purified by preparative HPLC (Phenomenex Synergi C18, 0.1 % trifluoroacetic acid in water / acetonitrile) and lyophilized to afford the title compound (10.5 mg, 16 %) as a white solid. MS: 395.1 ([M+H]⁺), ESI pos.

### Example 22

### 7-chloro-8-(1,1-difluoroethyl)-6-(2,6-difluorophenyl)-1-methyl-4H-[1,2,4]triazolo[4,3-a][1,4]benzodiazepine

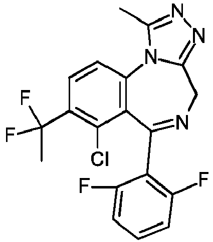

### a) 7-chloro-6-(2,6-difluorophenyl)-8-(1-ethoxyvinyl)-1-methyl-4H-[1,2,4]triazolo[4,3-a][1,4]benzodiazepine

In analogy to experiment of example 21 a, 8-bromo-7-chloro-6-(2,6-difluorophenyl)-1-methyl-4H-[1,2,4]triazolo[4,3-a][1,4]benzodiazepine was converted into the title compound (250 mg, 85 %) which was obtained as a grey solid. MS: 414.9 ([M+H]⁺), ESI pos.

### b) 1-[7-chloro-6-(2,6-difluorophenyl)-1-methyl-4H-[1,2,4]triazolo[4,3-a][1,4]benzodiazepin-8-yl]ethanone

In analogy to experiment of example 21 b, 7-chloro-6-(2,6-difluorophenyl)-8-(1-ethoxyvinyl)-1-methyl-4H-[1,2,4]triazolo[4,3-a][1,4]benzodiazepine was converted into the title compound (110 mg, 47 %) which was obtained as a white solid. MS: 386.9 ([M+H]⁺), ESI pos.

### c) 7-chloro-8-(1,1-difluoroethyl)-6-(2,6-difluorophenyl)-1-methyl-4H-[1,2,4]triazolo[4,3-a][1,4]benzodiazepine

In analogy to experiment of example 21 c, 1-[7-chloro-6-(2,6-difluorophenyl)-1-methyl-4H-[1,2,4]triazolo[4,3-a][1,4]benzodiazepin-8-yl]ethanone was converted into the title compound (33.6 mg, 31 %) which was obtained as a white solid. MS: 409.1 ([M+H]⁺), ESI pos.

### Example 23

### 7-chloro-8-(difluoromethyl)-6-(2,6-difluorophenyl)-4H-[1,2,4]triazolo[4,3-a] [1,4]benzodiazepine

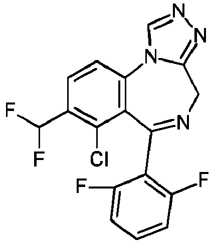

### a) 7-chloro-6-(2,6-difluorophenyl)-8-vinyl-4H-[1,2,4]triazolo[4,3-a][1,4]benzodiazepine

To a solution of 8-bromo-7-chloro-6-(2,6-difluorophenyl)-4H-[1,2,4]triazolo[4,3-a][1,4]benzodiazepine (300.0 mg, 0.730 mmol) in ethanol (6 mL) was added potassium vinyltrifluoroborate (196 mg, 0.15 mmol), triethylamine (222 mg, 2.2 mmol) and [1,1'-bis (diphenylphosphino)ferrocene]dichloropalladium(II) (54 mg, 0.07 mmol). The reaction mixture was stirred at 8 °C for 12 h, followed by addition of water (60 mL). The reaction mixture was extracted with ethyl acetate (100 mL). The organic layer was separated and washed with brine (60 mL), dried over sodium sulfate, filtered and concentrated *in vacuo.* The residue was purified by flash column chromatography (petrolether / ethyl acetate / ethanol 20:3:1 to 8:3:1) to afford the title compound (180 mg, 69 %) as a light yellow solid. MS: 357.1 ([M+H]⁺), ESI pos.

### b) 7-chloro-6-(2,6-difluorophenyl)-4H-[1,2,4]triazolo[4,3-a][1,4]benzodiazepine-8-carbaldehyde

To a solution of 7-chloro-6-(2,6-difluorophenyl)-8-vinyl-4H-[1,2,4]triazolo[4,3-a][1,4]benzodiazepine (170 mg, 0.48 mmol) in acetone (6 mL) and water (1 mL) was added osmium tetroxide (12 mg, 0.05 mmol). After 10 min stirring at room temperature, sodium periodate (203 mg, 0.95 mmol) was added, and the mixture was stirred for further 2 h. The reaction mixture was diluted with water (10 mL) and ethyl acetate (30 mL). The organic layer was washed with brine (10 mL), dried over sodium sulfate, filtered and concentrated *in vacuo.* The residue was purified by flash column chromatography (silica, dichloromethane / methanol 200:1 to 60:1) to afford the title compound (120 mg, 70 %) as a black solid. MS: 359.0 ([M+H]⁺), ESI pos.

### c) 7-chloro-8-(difluoromethyl)-6-(2,6-difluorophenyl)-4H-[1,2,4]triazolo[4,3-a][1,4]benzodiazepine

To a solution of 7-chloro-6-(2,6-difluorophenyl)-4H-[1,2,4]triazolo[4,3-a][1,4]benzodiazepine-8-carbaldehyde (160 mg, 0.45 mmol) in dichloromethane (3 mL) was added diethylaminosulfur trifluoride (359 mg, 2.23 mmol) at -78 °C. Upon addition, the reaction mixture was warmed up to room temperature and stirred for further 12 h. The reaction mixture was quenched by addition of saturated aqueous sodium bicarbonate (10 mL), then extracted with dichloromethane (30 mL). The organic layer was washed with brine (10 mL), filtered and concentrated *in vacuo.* The residue was purified by preparative HPLC (Boston Prime C18, 0.1% trifluoroacetic acid in water / acetonitrile) and lyophilized to afford the title compound (8.8 mg, 5 %) as a white solid. MS: 381.0 ([M+H]⁺), ESI pos.

### Example 24

### 8-bromo-7-chloro-6-(3-fluoro-2-pyridyl)-1-methyl-4H-[1,2,4]triazolo [4,3-a][1,4]benzodiazepine

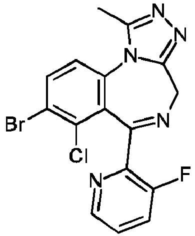

### a) 7-bromo-6-chloro-5-(3-fluoro-2-pyridyl)-1,3-dihydro-1,4-benzodiazepine-2-thione

In analogy to experiment of example 1 a, 7-bromo-6-chloro-5-(3-fluoro-2-pyridyl)-1,3-dihydro-1,4-benzodiazepin-2-one (building block E) was converted into the title compound (110 mg, 53 %) which was obtained as a brown solid. MS: 384.0 ([{⁷⁹Br, ³⁵Cl}M+H]⁺), 386.1 ([{⁸¹Br, ³⁵Cl or ⁷⁹Br, ³⁷Cl }M+H]⁺), ESI pos.

### b) 8-bromo-7-chloro-6-(3-fluoro-2-pyridyl)-1-methyl-4H-[1,2,4]triazolo[4,3-a][1,4]benzodiazepine

In analogy to experiment of example 1 b, 7-bromo-6-chloro-5-(3-fluoro-2-pyridyl)-1,3-dihydro-1,4-benzodiazepine-2-thione was converted into the title compound (9.5 mg, 8.5 %) which was obtained as a white solid. MS: 406.0 ([{⁷⁹Br, ³⁵Cl}M+H]⁺), 408.0 ([{⁸¹Br, ³⁵Cl or ⁷⁹Br, ³⁷Cl }M+H]⁺), ESI pos.

### Example 25

### 7-chloro-8-(difluoromethyl)-6-(2,6-difluorophenyl)-1-methyl-4H-[1,2,4]triazolo[4,3-a] [1,4]benzodiazepine

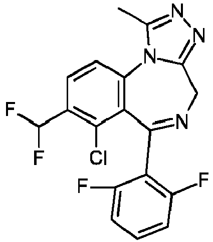

### a) 7-chloro-6-(2,6-difluorophenyl)-1-methyl-8-vinyl-4H-[1,2,4]triazolo[4,3-a][1,4]benzodiazepine

In analogy to experiment of example 23 a, 8-bromo-7-chloro-6-(2,6-difluorophenyl)-1-methyl-4H-[1,2,4]triazolo[4,3-a][1,4]benzodiazepine was converted into the title compound (120 mg, 69 %) which was obtained as a light yellow solid. MS: 371.0 ([M+H]⁺), ESI pos.

### b) 1-[7-chloro-6-(2,6-difluorophenyl)-1-methyl-4H-[1,2,4]triazolo[4,3-a][1,4]benzodiazepin-8-yl]ethanone

In analogy to experiment of example 23 b, 7-chloro-6-(2,6-difluorophenyl)-1-methyl-8-vinyl-4H-[1,2,4]triazolo[4,3-a][1,4]benzodiazepine was converted into the title compound (100 mg, 83 %) which was obtained as an off-white solid. MS: 387.0 ([M+H]⁺), ESI pos.

### c) 7-chloro-8-(difluoromethyl)-6-(2,6-difluorophenyl)-1-methyl-4H-[1,2,4]triazolo[4,3-a][1,4]benzodiazepine

In analogy to experiment of example 23 c, 1-[7-chloro-6-(2,6-difluorophenyl)-1-methyl-4H-[1,2,4]triazolo[4,3-a][1,4]benzodiazepin-8-yl]ethanone was converted into the title compound (10.1 mg, 19 %) which was obtained as a white solid. MS: 395.1 ([M+H]⁺), ESI pos.

### Example 26

### 8-bromo-7-chloro-6-(2-fluorophenyl)-2,4-dihydro-[1,2,4]triazolo[4,3-a][1,4]benzodiazepin-1-one

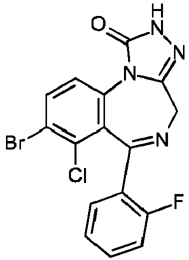

### a) 7-bromo-6-chloro-5-(2-fluorophenyl)-1,3-dihydro-1,4-benzodiazepin-2-one hydrazone

In analogy to experiment of example 9 b, 7-bromo-6-chloro-5-(2-fluorophenyl)-1,3-dihydro-1,4-benzodiazepine-2-thione was converted into the title compound (798 mg, 91 %) which was obtained as a light yellow solid. MS: 381.0 ([{⁷⁹Br, ³⁵Cl}M+H]⁺), 383.1 ([{⁸¹Br, ³⁵Cl or ⁷⁹Br, ³⁷Cl }M+H]⁺), ESI pos.

### b) 8-bromo-7-chloro-6-(2-fluorophenyl)-2,4-dihydro-[1,2,4]triazolo[4,3-a][1,4]benzodiazepin-1-one

In analogy to experiment of example 9 c, 7-bromo-6-chloro-5-(2-fluorophenyl)-1,3-dihydro-1,4-benzodiazepin-2-one hydrazone was converted into the title compound (38 mg, 31 %) which was obtained as a light yellow solid. MS: 407.0 ([{⁷⁹Br, ³⁵Cl}M+H]⁺), 409.0 ([{⁸¹Br, ³⁵Cl or ⁷⁹Br, ³⁷Cl }M+H]⁺), ESI pos.

### Example 27

### 7-chloro-6-(2-fluorophenyl)-1-pyridazin-3-yl-8-(trifluoromethyl)-4H-[1,2,4]triazolo[4,3-a][1,4]benzodiazepine

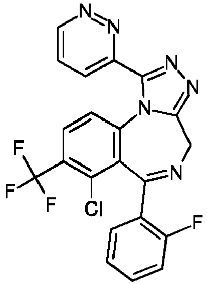

### a) 6-chloro-5-(2-fluorophenyl)-7-(trifluoromethyl)-1,3-dihydro-1,4-benzodiazepin-2-one

A solution of 6-chloro-5-(2-fluorophenyl)-7-iodo-1,3-dihydro-1,4-benzodiazepin-2-one (building block F, 250 mg, 0.600 mmol), iodocopper (229 mg, 1.21 mmol), methyl 2,2-difluoro-2-fluorosulfonyl-acetate (289 mg, 1.51 mmol) in *N*,*N*-dimethylformamide (7.5 mL) was heated to 70 °C for 16 h. The mixture was diluted with ethyl acetate (50 mL). The organic layer was filtered, washed with water (30 mL) and brine (30 mL), dried over sodium sulfate and concentrated *in vacuo.* The crude was purified by preparative HPLC (Phenomenex Synergi C18, 0.1 % trifluoroacetic acid in water / acetonitrile) and lyophilized to afford the title compound (150 mg, 70 %) as a white solid. MS: 356.8 ([M+H]⁺), ESI pos.

### b) 6-chloro-5-(2-fluorophenyl)-7-(trifluoromethyl)-1,3-dihydro-1,4-benzodiazepine-2-thione

In analogy to experiment of example 1 a, 6-chloro-5-(2-fluorophenyl)-7-(trifluoromethyl)-1,3-dihydro-1,4-benzodiazepin-2-one was converted into the title compound (246 mg, 89 %) which was obtained as a yellow solid. MS: 372.9 ([M+H]⁺), ESI pos.

### c) 7-chloro-6-(2-fluorophenyl)-1-pyridazin-3-yl-8-(trifluoromethyl)-4H-[1,2,4]triazolo[4,3-a][1,4]benzodiazepine

In analogy to experiment of example 2 b, 6-chloro-5-(2-fluorophenyl)-7-(trifluoromethyl)-1,3-dihydro-1,4-benzodiazepine-2-thione using pyridazine-3-carbohydrazide was converted into the title compound (6.9 mg, 4 %) which was obtained as a white solid. MS: 458.8([M+H]⁺), ESI pos.

### Example 28

### 7,8-dichloro-6-(2,6-difluorophenyl)-1-(1-methylpyrazol-4-yl)-4H-[1,2,4]triazolo[4,3-a][1,4]benzodiazepine

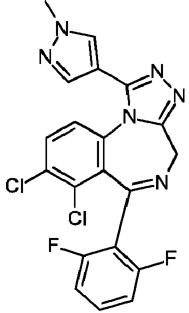

In analogy to experiment of example 1 b, 6,7-dichloro-5-(2,6-difluorophenyl)-1,3-dihydro-1,4-benzodiazepine-2-thione using 1-methylpyrazole-4-carbohydrazide was converted into the title compound (31 mg, 46 %) which was obtained as an off-white solid. MS: 445.2 ([{³⁵Cl, ³⁵Cl}M+H]⁺), ESI pos.

### Example 29

### 8-bromo-7-chloro-6-(3-fluoro-2-pyridyl)-4H-[1,2,4]triazolo[4,3-a][1,4]benzodiazepine

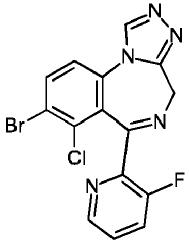

In analogy to experiment of example 1 b, 7-bromo-6-chloro-5-(3-fluoro-2-pyridyl)-1,3-dihydro-1,4-benzodiazepine-2-thione using formohydrazide was converted into the title compound (8.0 mg, 7.0 %) which was obtained as a light yellow solid. MS: 392.0 ([{⁷⁹Br, ³⁵Cl}M+H]⁺), 394.0 ([{⁸¹Br, ³⁵Cl or ⁷⁹Br, ³⁷Cl}M+H]⁺), ESI pos.

### Example 33

### [8-bromo-7-chloro-6-(2,6-difluorophenyl)-4H-[1,2,4]triazolo[4,3-a][1,4]benzodiazepin-1-yl]methanol

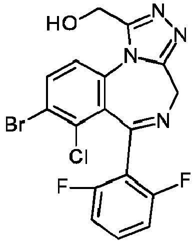

In analogy to experiment of example 1 b, 7-bromo-6-chloro-5-(2,6-difluorophenyl)-1,3-dihydro-1,4-benzodiazepin-2-thione using 2-hydroxyacetohydrazide was converted into the title compound (348 mg, 77 %) as a white powder. MS: 439.1 ([{⁷⁹Br, ³⁵Cl}M+H]⁺), 441.2 ([{⁸¹Br, ³⁵Cl or ⁷⁹Br, ³⁷Cl }M+H]⁺), ESI pos.

### Example 34

### 7-chloro-6-(2,6-difluorophenyl)-8-iodo-4H-[1,2,4]triazolo[4,3-a][1,4]benzodiazepine

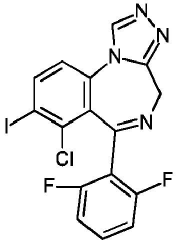

### a) 6-chloro-5-(2,6-difluorophenyl)-7-iodo-1,3-dihydro-1,4-benzodiazepine-2-thione

In analogy to experiment of example 1 a, 6-chloro-5-(2,6-difluorophenyl)-7-iodo-1,3-dihydro-1,4-benzodiazepin-2-one (building block H) was converted into the title compound (460 mg) which was obtained as a dark green solid. The crude was used as such in the following step without further characterization.

### b) 7-chloro-6-(2,6-difluorophenyl)-8-iodo-4H-[1,2,4]triazolo[4,3-a][1,4]benzodiazepine

In analogy to experiment of example 2 b, 6-chloro-5-(2,6-difluorophenyl)-7-iodo-1,3-dihydro-1,4-benzodiazepine-2-thione using formohydrazide was converted into the title compound (134 mg, 28 %) which was obtained as a white solid. MS: 456.8 ([M+H]⁺), ESI pos.

### Example 36

### [8-bromo-7-chloro-6-(2,6-difluorophenyl)-4H-[1,2,4]triazolo[4,3-a][1,4]benzodiazepin-1-yl]methanamine hydrochloride

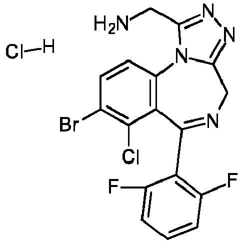

### a) tert-butyl N-[[8-bromo-7-chloro-6-(2,6-difluorophenyl)-4H-[1,2,4]triazolo[4,3-a][1,4]benzodiazepin-1-yl]methyl]carbamate

In analogy to experiment of example 1 b, 7-bromo-6-chloro-5-(2,6-difluorophenyl)-1,3-dihydro-1,4-benzodiazepin-2-thione using *tert*-butyl *N*-(2-hydrazino-2-oxo-ethyl)carbamate was converted into the title compound (126 mg, 63 %) as a white powder. MS: 538.0 ([{⁷⁹Br, ³⁵Cl}M+H]⁺), 540.0 ([{⁸¹Br, ³⁵Cl or ⁷⁹Br, ³⁷Cl }M+H]⁺), ESI pos.

### b) [8-bromo-7-chloro-6-(2,6-difluorophenyl)-4H-[1,2,4]triazolo[4,3-a][1,4]benzodiazepin-1-yl]methanamine hydrochloride

To a suspension of tert-butyl *N*-[[8-bromo-7-chloro-6-(2,6-difluorophenyl)-4H-[1,2,4]triazolo[4,3-a][1,4]benzodiazepin-1-yl]methyl]carbamate (126 mg, 0.234 mmol) in 1,4-dioxane (2 mL) was added dropwise hydrochloric acid (4.0 m in 1,4-dioxane, 585 µL, 2.34 mmol). The reaction mixture was stirred at room temperature for 4 h. The solid was filtered and dried *in vacuo* to afford the title compound (100 mg, 90 %) as a light orange solid. MS: 438.1 ([{⁷⁹Br, ³⁵Cl}M+H]⁺), 440.2 ([{⁸¹Br, ³⁵Cl or ⁷⁹Br, ³⁷Cl }M+H]⁺), ESI pos.

### Example 37

### 7-chloro-6-(2,6-difluorophenyl)-8-(trifluoromethyl)-4H-[1,2,4]triazolo[4,3-a] [1,4]benzodiazepine

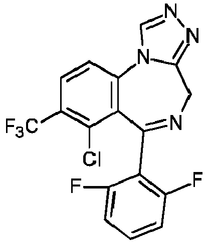

### a) 6-chloro-5-(2,6-difluorophenyl)-7-(trifluoromethyl)-1,3-dihydro-1,4-benzodiazepin-2-one

In analogy to experiment of example 27 a, 6-chloro-5-(2-fluorophenyl)-7-iodo-1,3-dihydro-1,4-benzodiazepin-2-one (building block H) was converted into the title compound (120 mg, 69 %) which was obtained as a dark red oil. MS: 375.0 ([M+H]⁺), ESI pos.

### b) 6-chloro-5-(2,6-difluorophenyl)-7-(trifluoromethyl)-1,3-dihydro-1,4-benzodiazepine-2-thione

In analogy to experiment of example 1 a, 6-chloro-5-(2,6-difluorophenyl)-7-(trifluoromethyl)-1,3-dihydro-1,4-benzodiazepin-2-one was converted into the title compound (72 mg, 57 %) which was obtained as a yellow solid. The crude was used as such in the following step without further characterization.

### c) 7-chloro-6-(2,6-difluorophenyl)-8-(trifluoromethyl)-4H-[1,2,4]triazolo[4,3-a][1,4]benzodiazepine

In analogy to experiment of example 2 b, 6-chloro-5-(2,6-difluorophenyl)-7-(trifluoromethyl)-1,3-dihydro-1,4-benzodiazepine-2-thione using formohydrazide was converted into the title compound (8.0 mg, 10 %) which was obtained as a white solid. MS: 399.0 ([M+H]⁺), ESI pos.

### Example 38

### 7-chloro-6-(2,6-difluorophenyl)-1-pyridazin-3-yl-8-(trifluoromethyl)-4H-[1,2,4]triazolo[4,3-a][1,4]benzodiazepine

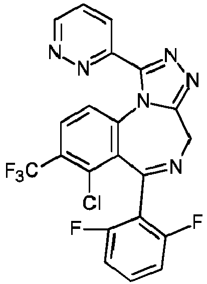

In analogy to experiment of example 2 b, 6-chloro-5-(2,6-difluorophenyl)-7-(trifluoromethyl)-1,3-dihydro-1,4-benzodiazepine-2-thione using pyridazine-3-carbohydrazide was converted into the title compound (31 mg, 24 %) which was obtained as a pink solid. MS: 477.1 ([M+H]⁺), ESI pos.

### Example 39

### 7-chloro-6-(2,6-difluorophenyl)-8-iodo-1-pyridazin-3-yl-4H-[1,2,4]triazolo[4,3-a][1,4]benzodiazepine

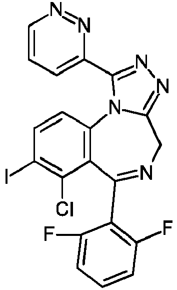

In analogy to experiment of example 2 b, 6-chloro-5-(2,6-difluorophenyl)-7-iodo-1,3-dihydro-1,4-benzodiazepine-2-thione (example 34 a) using pyridazine-3-carbohydrazide was converted into the title compound (19.4 mg, 13 %) which was obtained as a pink solid. MS: 535.1 ([M+H]⁺), ESI pos.

### Example 40

### 7-chloro-6-(2,6-difluorophenyl)-1-methyl-8-(trifluoromethyl)-4H-[1,2,4]triazolo[4,3-a][1,4]benzodiazepine

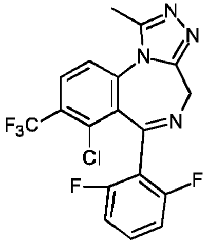

In analogy to experiment of example 2 b, 6-chloro-5-(2,6-difluorophenyl)-7-(trifluoromethyl)-1,3-dihydro-1,4-benzodiazepine-2-thione using acetohydrazide was converted into the title compound (39.6 mg, 31 %) which was obtained as a white solid. MS: 413.1 ([M+H]⁺), ESI pos.

### Example 41

### 7-chloro-6-(2,6-difluorophenyl)-8-iodo-1-methyl-4H-[1,2,4]triazolo[4,3-a][1,4]benzodiazepine

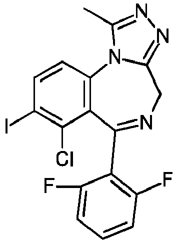

In analogy to experiment of example 2 b, 6-chloro-5-(2,6-difluorophenyl)-7-iodo-1,3-dihydro-1,4-benzodiazepine-2-thione using acetohydrazide was converted into the title compound (18.0 mg, 12 %) which was obtained as a white solid. MS: 471.0 ([M+H]⁺), ESI pos.

### Example 42

### 7,8-dibromo-6-(2,6-difluorophenyl)-1-pyridazin-3-yl-4H-[1,2,4]triazolo[4,3-a][1,4]benzodiazepine

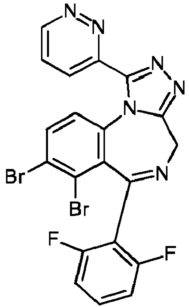

### a) 6,7-dibromo-5-(2,6-difluorophenyl)-1,3-dihydro-1,4-benzodiazepine-2-thione

In analogy to experiment of example 1 a, 6,7-dibromo-5-(2,6-difluorophenyl)-1,3-dihydro-1,4-benzodiazepin-2-one (building block I) was converted into the title compound (170 mg, 49 %) which was obtained as an off-white solid. MS: 444.9 ([{⁷⁹Br, ⁷⁹Brl}M+H]⁺), 446.8 ([{⁸¹Br, ⁷⁹Br}M+H]⁺), 448.9 ([{⁸¹Br, ⁸¹Br}M+H]⁺), ESI pos.

### b) 7,8-dibromo-6-(2,6-difluorophenyl)-1-pyridazin-3-yl-4H-[1,2,4]triazolo[4,3-a][1,4]benzodiazepine

In analogy to experiment of example 1 b, 6,7-dibromo-5-(2,6-difluorophenyl)-1,3-dihydro-1,4-benzodiazepine-2-thione using pyridazine-3-carbohydrazide was converted into the title compound (57 mg, 56 %) which was obtained as an off-white solid. MS: 531.1 ([{⁷⁹Br, ⁷⁹Brl}M+H]⁺), 533.0 ([{⁸¹Br, ⁷⁹Br}M+H]⁺), 535.1 ([{⁸¹Br, ⁸¹Br}M+H]⁺), ESI pos.

### Example 43

### 7,8-dibromo-6-(2,6-difluorophenyl)-1-pyrimidin-4-yl-4H-[1,2,4]triazolo[4,3-a] [1,4]benzodiazepine

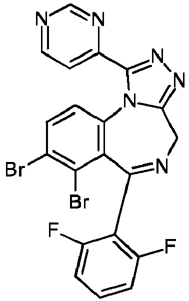

In analogy to experiment of example 1 b, 6,7-dibromo-5-(2,6-difluorophenyl)-1,3-dihydro-1,4-benzodiazepine-2-thione using pyrimidine-4-carbohydrazide was converted into the title compound (51 mg, 50 %) which was obtained as an off-white solid. MS: 531.1 ([⁷⁹Br, ⁷⁹Brl}M+H]⁺), 533.1 ([{⁸¹Br, ⁷⁹Br}M+H]⁺), 535.1 ([{⁸¹Br, ⁸¹Br}M+H]⁺), ESI pos.

### Example 44

### 8-bromo-7-chloro-6-(3-chloro-2-pyridyl)-1-pyrimidin-4-yl-4H-[1,2,4]triazolo[4,3-a][1,4]benzodiazepine

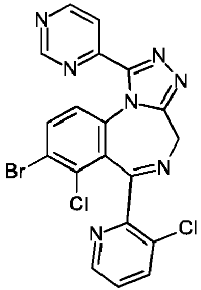

### a) 7-bromo-6-chloro-5-(3-chloro-2-pyridyl)-1,3-dihydro-1,4-benzodiazepine-2-thione

In analogy to experiment of example 1 a, 7-bromo-6-chloro-5-(3-chloro-2-pyridyl)-1,3-dihydro-1,4-benzodiazepin-2-one (building block J) was converted into the title compound (490 mg, 97 %) which was obtained as a brown solid. MS: 399.9 ([⁷⁹Br, ³⁵Cl, ³⁵Cl }M+H]⁺), 401.9 ([{⁸¹Br, ³⁵Cl, ³⁵Cl or ⁷⁹Br, ³⁷Cl, ³⁵Cl}M+H]⁺), ESI pos.

### b) 8-bromo-7-chloro-6-(3-chloro-2-pyridyl)-1-pyrimidin-4-yl-4H-[1,2,4]triazolo[4,3-a][1,4]benzodiazepine

In analogy to experiment of example 1 b, 7-bromo-6-chloro-5-(3-chloro-2-pyridyl)-1,3-dihydro-1,4-benzodiazepine-2-thione using pyrimidine-4-carbohydrazide was converted into the title compound (6.6 mg, 5.3 %) which was obtained as a white solid. MS: 486.0 ⁷⁹Br, ³⁵Cl, ³⁵Cl }M+H]⁺), 488.0 ([{⁸¹Br, ³⁵Cl, ³⁵Cl or ⁷⁹Br, ³⁷Cl, ³⁵Cl}M+H]⁺), ESI pos.

### Example 45

### 8-bromo-7-chloro-6-(3-fluoro-2-pyridyl)-1-pyridazin-3-yl-4H-[1,2,4]triazolo[4,3-a] [1,4]benzodiazepine

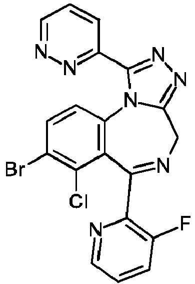

In analogy to experiment of example 1 b, 7-bromo-6-chloro-5-(3-fluoro-2-pyridyl)-1,3-dihydro-1,4-benzodiazepine-2-thione using pyridazine-3-carbohydrazide was converted into the title compound (8.6 mg, 7 %) which was obtained as a light yellow solid. MS: 470.0 ([{⁷⁹Br, ³⁵Cl}M+H]⁺), 472.0 ([{⁸¹Br, ³⁵Cl or ⁷⁹Br, ³⁷Cl }M+H]⁺), ESI pos.

### Example 46

### 8-bromo-7-chloro-6-(3-fluoro-2-pyridyl)-1-pyrimidin-4-yl-4H-[1,2,4]triazolo[4,3-a][1,4]benzodiazepine

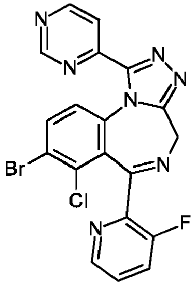

In analogy to experiment of example 1 b, 7-bromo-6-chloro-5-(3-fluoro-2-pyridyl)-1,3-dihydro-1,4-benzodiazepine-2-thione using pyrimidine-4-carbohydrazide was converted into the title compound (6.7 mg, 5.4 %) which was obtained as a white solid. MS: 470.0 ([{⁷⁹Br, ³⁵Cl}M+H]⁺), 472.1 ([{⁸¹Br, ³⁵Cl or ⁷⁹Br, ³⁷Cl }M+H]⁺), ESI pos.

### Example 47

### 8-bromo-7-chloro-6-(3-chloro-2-pyridyl)-1-pyridazin-3-yl-4H-[1,2,4]triazolo[4,3-a][1,4]benzodiazepine

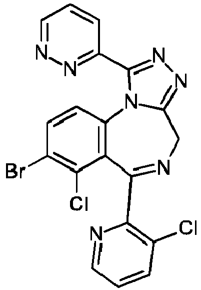

In analogy to experiment of example 1 b, 7-bromo-6-chloro-5-(3-chloro-2-pyridyl)-1,3-dihydro-1,4-benzodiazepine-2-thione using pyridazine-3-carbohydrazide was converted into the title compound (18 mg, 25 %) which was obtained as a white solid. MS: 485.8 ([{⁷⁹Br,³⁵Cl, ³⁵Cl }M+H]⁺), 487.9 ([{⁸¹Br, ³⁵Cl, ³⁵Cl or ⁷⁹Br, ³⁷Cl, ³⁵Cl}M+H]⁺), ESI pos.

### Example 48

### 7-chloro-6-(2,6-difluorophenyl)-8-methyl-1-pyridazin-3-yl-4H-[1,2,4]triazolo[4,3-a][1,4]benzodiazepine

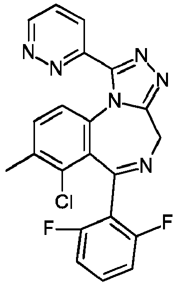

In analogy to experiment of example 1 b, 6-chloro-5-(2,6-difluorophenyl)-7-methyl-1,3-dihydro-1,4-benzodiazepine-2-thione using pyridazine-3-carbohydrazide was converted into the title compound (15.5 mg, 12 %) which was obtained as a pink solid. MS: 423.1 ([M+H]⁺), ESI pos.

### Example 49

### 7-chloro-6-(2,6-difluorophenyl)-8-methyl-1-pyrimidin-4-yl-4H-[1,2,4]triazolo[4,3-a][1,4]benzodiazepine

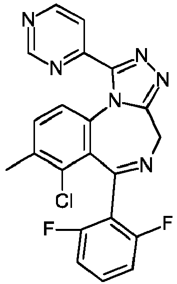

In analogy to experiment of example 1 b, 6-chloro-5-(2,6-difluorophenyl)-7-methyl-1,3-dihydro-1,4-benzodiazepine-2-thione using pyrimidine-4-carbohydrazide was converted into the title compound (6.3 mg, 5 %) which was obtained as a light yellow solid. MS: 423.1 ([M+H]⁺), ESI pos.

### Example 50

### 8-bromo-7-chloro-6-(2-chloro-6-fluoro-phenyl)-1-methyl-4H-[1,2,4]triazolo [4,3-a][1,4]benzodiazepine

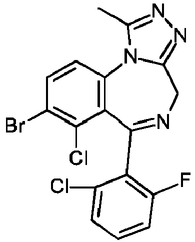

### a) 7-bromo-6-chloro-5-(2-chloro-6-fluoro-phenyl)-1,3-dihydro-1,4-benzodiazepine-2-thione

In analogy to experiment of example 1 a, 7-bromo-6-chloro-5-(2-chloro-6-fluoro-phenyl)-1,3-dihydro-1,4-benzodiazepine-2-one (building block K) was converted into the title compound (88 mg, 95 %) which was obtained as a yellow solid. The crude was used as such in the following step without further characterization.

### b) 8-bromo-7-chloro-6-(2-chloro-6-fluoro-phenyl)-1-methyl-4H-[1,2,4]triazolo[4,3-a][1,4]benzodiazepine

In analogy to experiment of example 1 b, 7-bromo-6-chloro-5-(2-chloro-6-fluoro-phenyl)-1,3-dihydro-1,4-benzodiazepine-2-thione was converted into the title compound (22.5 mg, 24 %) which was obtained as a yellow solid. MS: 439.0 ⁷⁹Br, ³⁵Cl, ³⁵Cl}M+H]⁺), 441.0 ([{⁸¹Br, ³⁵Cl, ³⁵Clor ⁷⁹Br, ³⁷Cl, ¹⁵Cl}M+H]⁺), ESI pos.

### Example 54

### 7,8-dichloro-6-(3-fluoro-2-pyridyl)-1-pyridazin-3-yl-4H-[1,2,4]triazolo[4,3-a][1,4]benzodiazepine

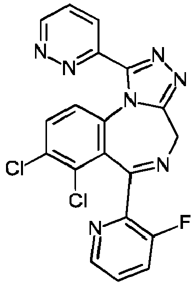

### a) 6,7-dichloro-5-(3-fluoro-2-pyridyl)-1,3-dihydro-1,4-benzodiazepine-2-thione

In analogy to experiment of example 1 a, 6,7-dichloro-5-(3-fluoro-2-pyridyl)-1,3-dihydro-1,4-benzodiazepin-2-one (building block N) was converted into the title compound (2.56 g, 67 %) which was obtained as a light yellow solid. MS: 340.0 ([{³⁵Cl, ³⁵Cl}M+H]⁺), ESI pos.

### b) 7,8-dichloro-6-(3-fluoro-2-pyridyl)-1-pyridazin-3-yl-4H-[1,2,4]triazolo[4,3-a][1,4]benzodiazepine

In analogy to experiment of example 1 b, 6,7-dichloro-5-(3-fluoro-2-pyridyl)-1,3-dihydro-1,4-benzodiazepine-2-thione using pyridazine-3-carbohydrazide was converted into the title compound (111 mg, 44 %) which was obtained as an off-white solid. MS: 426.0 ([{³⁵Cl, ³⁵Cl}M+H]⁺), ESI pos.

### Example 55

### 7,8-dichloro-6-(3-fluoro-2-pyridyl)-1-pyrimidin-4-yl-4H-[1,2,4]triazolo[4,3-a][1,4]benzodiazepine

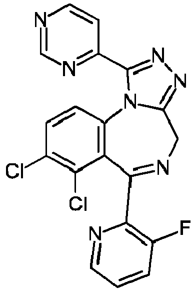

In analogy to experiment of example 1b, 6,7-dichloro-5-(3-fluoro-2-pyridyl)-1,3-dihydro-1,4-benzodiazepine-2-thione (example 54 a) using pyrimidine-4-carbohydrazide was converted into the title compound (111 mg, 44 %) which was obtained as an off-white solid. MS: 426.1 ([{³⁵Cl, ³⁵Cl}M+H)⁺), ESI pos.

### Example 57

### 7-chloro-6-(2,6-difluorophenyl)-8-methoxy-1-pyridazin-3-yl-4H-[1,2,4]triazolo[4,3-a][1,4]benzodiazepine

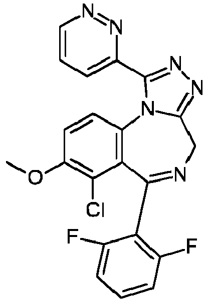

A solution of 8-bromo-7-chloro-6-(2,6-difluorophenyl)-1-pyridazin-3-yl-4H-[1,2,4]triazolo[4,3-a][1,4]benzodiazepine (110 mg, 0.230 mmol), [(2-di-*tert*-butylphosphino-2',4',6'-triisopropyl-1,1'-biphenyl)-2-(2'-amino-1,1'-biphenyl)] palladium(II) methanesulfonate (40 mg, 0.050 mmol) and triethylamine (0.16 mL, 1.13 mmol) in methanol (15 mL) was heated to 80 °C for 16 h. The reaction mixture was filtered through a plug of celite, concentrated *in vacuo* and purified directly by preparative HPLC (Phenomenex Luna C18, 0.075 % trifluoroacetic acid in water / acetonitrile) to afford the title compound (47 mg, 46 %) as a pink solid. MS: 439.0 ([M+H]⁺), ESI pos.

### Example 58

### 7,8-dichloro-6-(3-fluoro-2-pyridyl)-1-(1-methylpyrazol-4-yl)-4H-[1,2,4]triazolo [4,3-a][1,4]benzodiazepine

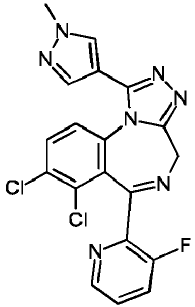

In analogy to experiment of example 1 b, 6,7-dichloro-5-(3-fluoro-2-pyridyl)-1,3-dihydro-1,4-benzodiazepine-2-thione using 1-methylpyrazole-4-carbohydrazide was converted into the title compound (74 mg, 29 %) which was obtained as a white solid. MS: 428.1 ([{³⁵Cl, ³⁵Cl}M+H]⁺), ESI pos.

### Example 59

### 7,8-dichloro-6-(3-fluoro-2-pyridyl)-2,4-dihydro-[1,2,4]triazolo[4,3-a][1,4]benzodiazepin-1-one

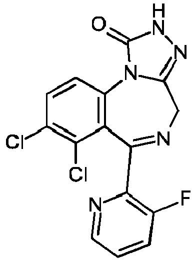

### a) 6,7-dichloro-5-(3-fluoro-2-pyridyl)-1,3-dihydro-1,4-benzodiazepin-2-one hydrazone

In analogy to experiment of example 9 b, 6,7-dichloro-5-(3-fluoro-2-pyridyl)-1,3-dihydro-1,4-benzodiazepine-2-thione was converted into the title compound (400 mg, 80 %) which was obtained as a yellow solid. MS: 338.0 ([{³⁵Cl, ³⁵Cl}M+H]⁺), ESI pos.

### b) 7,8-dichloro-6-(3-fluoro-2-pyridyl)-2,4-dihydro-[1,2,4]triazolo[4,3-a][1,4]benzodiazepin-1-one

In analogy to experiment of example 9 c, 6,7-dichloro-5-(3-fluoro-2-pyridyl)-1,3-dihydro-1,4-benzodiazepin-2-one hydrazone was converted into the title compound (98 mg, 60 %) which was obtained as a yellow solid. MS: 364.0 ([{³⁵Cl, ³⁵Cl}M+H]⁺), ESI pos.

### Example 60

### 7,8-dichloro-6-(3-fluoro-2-pyridyl)-4H-[1,2,4]triazolo[4,3-a][1,4]benzodiazepine

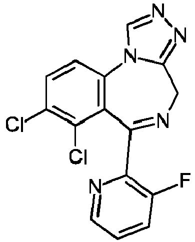

In analogy to experiment of example 1 b, 6,7-dichloro-5-(3-fluoro-2-pyridyl)-1,3-dihydro-1,4-benzodiazepine-2-thione using formohydrazide was converted into the title compound (54 mg, 35 %) which was obtained as a light yellow solid. MS: 348.0 ([{³⁵Cl, ³⁵Cl}M+H]⁺), ESI pos.

### Example 61

### 7-chloro-6-(2,6-difluorophenyl)-8-methoxy-1-pyrimidin-4-yl-4H-[1,2,4]triazolo[4,3-a][1,4]benzodiazepine

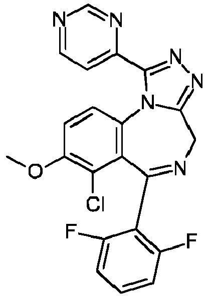

### a)8-bromo-7-chloro-6-(2,6-difluorophenyl)-1-pyrimidin-4-yl-4H-[1,2,4]triazolo[4,3-a][1,4]benzodiazepine

In analogy to experiment of example 2 b, 7-bromo-6-chloro-5-(2-fluorophenyl)-1,3-dihydro-1,4-benzodiazepine-2-thione using pyrimidine-4-carbohydrazide was converted into the title compound (133 mg, 33 %) which was obtained as a light yellow solid. MS: 486.9 ({⁷⁹Br, ¹⁵Cl}M+H]⁺), 488.9 ([{⁸¹Br, ³⁵Cl or ⁷⁹Br, ³⁷Cl}M+H]⁺), ESI pos.

### b) 7-chloro-6-(2,6-difluorophenyl)-8-methoxy-1-pyrimidin-4-yl-4H-[1,2,4]triazolo[4,3-a][1,4]benzodiazepine

In analogy to experiment of example 57, 8-bromo-7-chloro-6-(2,6-difluorophenyl)-l-pyrimidin-4-yl-4H-[1,2,4]triazolo[4,3-a][1,4]benzodiazepine was converted into the title compound (24 mg, 20 %) which was obtained as an off-white solid. MS: 439.0 ([M+H]⁺), ESI pos.

### Example 62

### 7-chloro-6-(2,6-difluorophenyl)-8-methoxy-1-(3-pyridyl)-4H-[1,2,4]triazolo[4,3-a][1,4]benzodiazepine

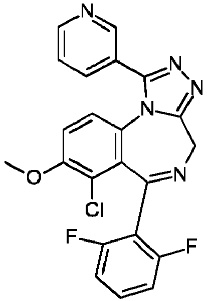

### a) 8-bromo-7-chloro-6-(2,6-difluorophenyl)-1-(3-pyridyl)-4H-[1,2,4]triazolo[4,3-a][1,4]benzodiazepine

In analogy to experiment of example 2 b, 7-bromo-6-chloro-5-(2,6-difluorophenyl)-1,3-dihydro-1,4-benzodiazepine-2-thione using nicotinic acid hydrazide was converted into the title compound (188 mg, 31 %) which was obtained as a brown solid. MS: 485.9 ({⁷⁹Br, ¹⁵Cl}M+H]⁺), 487.9 ([{⁸¹Br, ³⁵Clor ⁷⁹Br, ³⁷Cl}M+H]⁺), ESI pos.

### b) 7-chloro-6-(2,6-difluorophenyl)-8-methoxy-1-(3-pyridyl)-4H-[1,2,4]triazolo[4,3-a][1,4]benzodiazepine

In analogy to experiment of example 57, 8-bromo-7-chloro-6-(2,6-difluorophenyl)-1-(3-pyridyl)-4H-[1,2,4]triazolo[4,3-a][1,4]benzodiazepine was converted into the title compound (29 mg, 17 %) which was obtained as a white solid. MS: 438.1 ([M+H]⁺), ESI pos.

### Example 65

### 7,8-dichloro-1-cyclopropyl-6-(3-fluoro-2-pyridyl)-4H-[1,2,4]triazolo[4,3-a][1,4]benzodiazepine

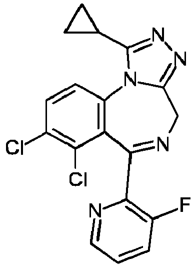

In analogy to experiment of example 1 b, 6,7-dichloro-5-(3-fluoro-2-pyridyl)-1,3-dihydro-1,4-benzodiazepine-2-thione using cyclopropanecarbohydrazide was converted into the title compound (77 mg, 44 %) which was obtained as a white solid. MS: 387.9 ([{³⁵Cl, ³⁵Cl}M+H]⁺), 389.8 ([{³⁵Cl, ³⁷Cl}M+H]⁺), ESI pos.

### Example 69

### 7-chloro-6-(3-fluoro-2-pyridyl)-1-pyrimidin-4-yl-8-(trifluoromethyl)-4H-[1,2,4]triazolo[4,3-a][1,4]benzodiazepine

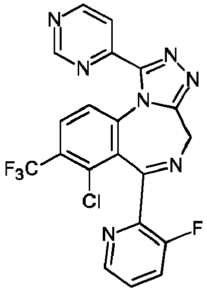

### a) 6-chloro-5-(3-fluoro-2-pyridyl)-7-(trifluoromethyl)-1,3-dihydro-1,4-benzodiazepin-2-one

In analogy to experiment of example 27 a, 6-chloro-5-(3-fluoro-2-pyridyl)-7-iodo-1,3-dihydro-1,4-benzodiazepin-2-one (building block P) was converted into the title compound (1.0 g, 26 %) which was obtained as a brown solid. MS: 358.0 ([M+H]⁺), ESI pos.

### b) 6-chloro-5-(3-fluoro-2-pyridyl)-7-(trifluoromethyl)-1,3-dihydro-1,4-benzodiazepine-2-thione

In analogy to experiment of example 1 a, 6-chloro-5-(3-fluoro-2-pyridyl)-7-(trifluoromethyl)-1,3-dihydro-1,4-benzodiazepin-2-one was converted into the title compound (430 mg, 91 %) which was obtained as a brown solid. MS: 373.8 ([M+H]⁺), ESI pos.

### c) 7-chloro-6-(3-fluoro-2-pyridyl)-1-pyrimidin-4-yl-8-(trifluoromethyl)-4H-[1,2,4]triazolo[4,3-a][1,4]benzodiazepine

In analogy to experiment of example 1 b, 6-chloro-5-(3-fluoro-2-pyridyl)-7-(trifluoromethyl)-1,3-dihydro-1,4-benzodiazepine-2-thione using pyrimidine-4-carbohydrazide was converted into the title compound (20 mg, 27 %) which was obtained as a white solid. MS: 460.1 ([M+H]⁺), ESI pos.

### Example 70

### 7-chloro-6-(3-fluoro-2-pyridyl)-1-(1-methylpyrazol-4-yl)-8-(trifluoromethyl)-4H-[1,2,4]triazolo[4,3-a][1,4]benzodiazepine

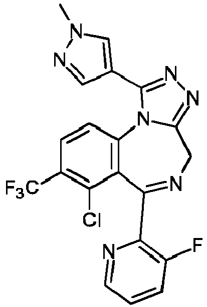

In analogy to experiment of example 1 b, 6-chloro-5-(3-fluoro-2-pyridyl)-7-(trifluoromethyl)-1,3-dihydro-1,4-benzodiazepin-2-thione using 1-methylpyrazole-4-carbohydrazide was converted into the title compound (14 mg, 18 %) which was obtained as a white solid. MS: 462.2 ([M+H]⁺), ESI pos.

### Example 71

### 7,8-dichloro-6-(3-fluoro-2-pyridyl)-1-methyl-4H-[1,2,4]triazolo[4,3-a] [1,4]benzodiazepine

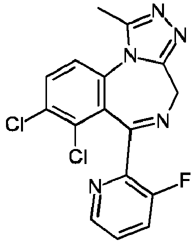

In analogy to experiment of example 1 b, 6,7-dichloro-5-(3-fluoro-2-pyridyl)-1,3-dihydro-1,4-benzodiazepine-2-thione using acetohydrazide was converted into the title compound (60 mg, 47 %) which was obtained as a white solid. MS: 362.1 ([{³⁵Cl, ³⁵Cl}M+H]⁺), ESI pos.

### Example 72

### 7-chloro-6-(3-fluoro-2-pyridyl)-1-pyridazin-3-yl-8-(trifluoromethyl)-4H-[1,2,4]triazolo[4,3-a][1,4]benzodiazepine

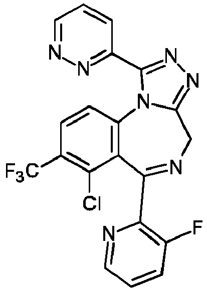

In analogy to experiment of example 1 b, 6-chloro-5-(3-fluoro-2-pyridyl)-7-(trifluoromethyl)-1,3-dihydro-1,4-benzodiazepin-2-thione using pyridazine-3-carbohydrazide was converted into the title compound (15 mg, 32 %) which was obtained as a white solid. MS: 460.2 ([M+H]⁺), ESI pos.

### Example 77

### 8-bromo-7-chloro-1-cyclopropyl-6-(3-fluoro-2-pyridyl)-4H-[1,2,4]triazolo[4,3-a][1,4]benzodiazepine

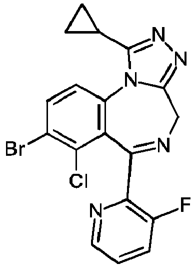

In analogy to experiment of example 1 b, 7-bromo-6-chloro-5-(3-fluoro-2-pyridyl)-1,3-dihydro-1,4-benzodiazepine-2-thione using cyclopropanecarbohydrazide was converted into the title compound (283 mg, 31 %) which was obtained as a white solid. MS: 432.0 ([{⁷⁹Br, ³⁵Cl}M+H]⁺), 434.1 ([{⁸¹Br, ³⁵Clor ⁷⁹Br, ³⁷Cl}M+H]⁺), ESI pos.

### Example 78

### 7-chloro-6-(3-fluoro-2-pyridyl)-1-methyl-8-(trifluoromethyl)-4H-[1,2,4]triazolo[4,3-a][1,4]benzodiazepine

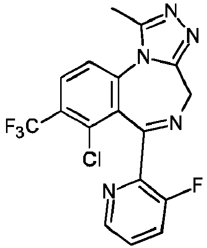

In analogy to experiment of example 1 b, 6-chloro-5-(3-fluoro-2-pyridyl)-7-(trifluoromethyl)-1,3-dihydro-1,4-benzodiazepin-2-thione using acetohydrazide was converted into the title compound (38 mg, 52 %) which was obtained as a white solid. MS: 396.1 ([M+H]⁺), ESI pos.

### Example 79

### 7-chloro-1-cyclopropyl-6-(2,6-difluorophenyl)-8-methoxy-4H-[1,2,4]triazolo[4,3-a][1,4]benzodiazepine

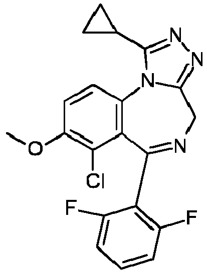

### a) 8-bromo-7-chloro-1-cyclopropyl-6-(2,6-difluorophenyl)-4H-[1,2,4]triazolo[4,3-a][1,4]benzodiazepine

In analogy to experiment of example 2 b, 7-bromo-6-chloro-5-(2,6-difluorophenyl)-1,3-dihydro-1,4-benzodiazepine-2-thione using cyclopropane carbohydrazide was converted into the title compound (180 mg, 54 %) which was obtained as a brown solid. MS: 448.9 ([{⁷⁹Br, ¹⁵Cl}M+H]⁺), 450.9 ([{⁸¹Br, ³⁵Clor ⁷⁹Br, ³⁷Cl}M+H]⁺), ESI pos.

### b) 7-chloro-1-cyclopropyl-6-(2,6-difluorophenyl)-8-methoxy-4H-[1,2,4]triazolo[4,3-a][1,4]benzodiazepine

In analogy to experiment of example 57, 8-bromo-7-chloro-1-cyclopropyl-6-(2,6-difluorophenyl)-4H-[1,2,4]triazolo[4,3-a][1,4]benzodiazepine was converted into the title compound (6 mg, 5 %) which was obtained as a white solid. MS: 401.1 ([M+H]⁺), ESI pos.

### Example 90

### 7,8-dichloro-6-(2,6-difluorophenyl)-1-methoxy-4H-[1,2,4]triazolo[4,3-a][1,4]benzodiazepine

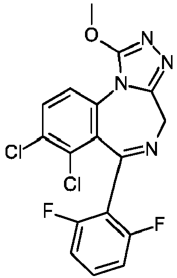

### a) 1-bromo-7,8-dichloro-6-(2,6-difluorophenyl)-4H-[1,2,4]triazolo[4,3-a][1,4]benzodiazepine

To a mixture of 7,8-dichloro-6-(2,6-difluorophenyl)-4H-[l,2,4]triazolo[4,3-a][1,4]benzodiazepine (400 mg, 1.1 mmol) in toluene (25 mL) was added 1-bromopyrrolidine-2,5-dione (214 mg, 1.2 mmol) at room temperature. The reaction mixture was stirred for 2.5 hours, before being heated to 60 °C for 2 h. A further amount of 1-bromopyrrolidine-2,5-dione (214 mg, 1.2 mmol) was added and the reaction mixture was kept at 60 °C for further 30 min. The reaction mixture was poured into saturated aqueous sodium bicarbonate (100 mL) and extracted with ethyl acetate (2 × 100 mL). The combined organic layers were dried over sodium sulfate and concentrated *in vacuo.* The residue was purified by flash column chromatography (silica, ethyl acetate / ethanol 3:1 in heptane 0-60 %) to afford the title compound (496 mg, 86 %) as an off-white solid. MS: 443.0 ⁷⁹Br, ³⁵Cl, ³⁵Cl}M+H]⁺), 445.1 ([{⁸¹Br, ³⁵Cl, ³⁵Cl or ⁷⁹Br, ³⁷Cl, ³⁵Cl}M+H]⁺), ESI pos.

### b) 7,8-dichloro-6-(2,6-difluorophenyl)-1-methoxy-4H-[1,2,4]triazolo[4,3-a][1,4]benzodiazepine

A Schlenk tube was charged with -bromo-7,8-dichloro-6-(2,6-difluorophenyl)-4H-[1,2,4]triazolo[4,3-a][1,4]benzodiazepine (30 mg, 0.068 mmol), *rac*-BINAP (8.4 mg, 13.5 µmol), cesium carbonate (66 mg, 203 µmol) and toluene (0.9 mL). The tube was degassed by bubbling argon through the reaction mixture for 5 min. Palladium (II) acetate (3.03 mg, 0.0135 mmol) was added then the tube was sealed and the reaction mixture was heated to 80 °C for 30 min. The reaction mixture was cooled down to room temperature, before being quenched with aqueous saturated sodium bicarbonate (5 mL) and extracted with ethyl acetate (3 × 5 mL). The combined organic layers were washed with saturated aqueous sodium bicarbonate (1 × 5 mL), dried over sodium sulfate, filtered and concentrated *in vacuo.* The residue was purified by flash column chromatography (silica, ethyl acetate / ethanol (3:1) in heptane 0-100 %) and, subsequently, by preparative HPLC (YMC-Triart C18, 0.1 % HCOOH in water / acetonitrile) to afford the title compound (6.4 mg, 24 %) as an off-white solid. MS: 395.1 ([{³⁵Cl, ³⁵Cl}M+H]⁺), ESI pos.

### Example 91

### trans-7,8-dichloro-6-(2,6-difluorophenyl)-1-(3-methoxycyclobutyl)-4H-[1,2,4]triazolo[4,3-a][1,4]benzodiazepine

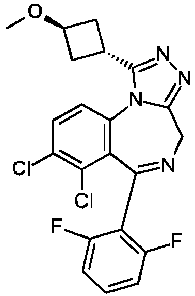

In analogy to experiment of example 2 b, 6,7-dichloro-5-(2,6-difluorophenyl)-1,3-dihydro-1,4-benzodiazepine-2-thione (example 9 a) using 3-methoxycyclobutanecarbohydrazide was converted into the title compound (69 mg, 60 %) which was obtained as a white solid. MS: 449.3 ([{³⁵Cl, ³⁵Cl}M+H]⁺), ESI pos.

### Example 104

### 8-bromo-7-chloro-6-(2,6-difluorophenyl)-1-pyridazin-3-yl-4H-[1,2,4]triazolo [4,3-a][1,4]benzodiazepine

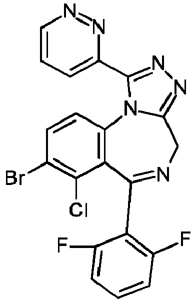

In analogy to experiment of example 1 b, 7-bromo-6-chloro-5-(2,6-difluorophenyl)-1,3-dihydro-1,4-benzodiazepine-2-thione using pyridazine-3-carbohydrazide was converted into the title compound (20.7 mg, 17 %) which was obtained as a white solid. MS: 487.0 ([{⁷⁹Br, ¹⁵Cl}M+H]⁺), 489.1 ([{⁸¹Br, ³⁵Cl or ⁷⁹Br, ³⁷Cl}M+H]⁺), ESI pos.

### Reference Compounds

### RE-A

### 8-bromo-6-(2,6-difluorophenyl)-1-methyl-4H-[1,2,4]triazolo [4,3-a][1,4]benzodiazepine

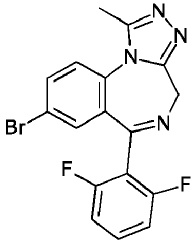

### a) N-[2-(2,6-difluorobenzoyl)phenyl]acetamide

In analogy to experiment of building block A b, 2-methyl-3,1-benzoxazin-4-one (CAS# 525-76-8) was converted into the title compound (40 g, 80 %) which was obtained as a light yellow solid. MS: 276.2 ([M+H]⁺), ESI pos.

### b) (2-aminophenyl)-(2,6-difluorophenyl)methanone

In analogy to experiment of building block A c, *N*-[2-(2,6-difluorobenzoyl)phenyl]acetamide was converted into the title compound (19.5 g, 75 %) which was obtained as a yellow solid. MS: 234.1 ([M+H]⁺), ESI pos.

### c) (2-amino-5-bromo-phenyl)-(2,6-difluorophenyl)methanone

To a solution of (2-aminophenyl)-(2,6-difluorophenyl)methanone (5.00 g, 21.4 mmol) in dichloromethane (50 mL) was added portionwise *N*-bromosuccinimide (4.02 g, 22.51 mmol) at - 15 °C. The reaction mixture was stirred at -15 °C for 1 h, till complete consumption of starting material (as judged by LCMS analysis). The mixture was concentrated under reduced pressure and the resulting residue purified by preparative HPLC (Shim-pack C18, 0.225% trifluoroacetic acid in water / acetonitrile). The combined fractions were diluted with saturate aqueous sodium bicarbonate and extracted with ethyl acetate (3 × 200 mL). The organic phase was washed with brine (2 × 100 mL), dried (Na₂SO₄) and concentrated *in vacuo* to afford the title compound (4.44 g, 66 %) as a yellow solid. MS: 311.9 ([{⁷⁹Br}M+H]⁺), 314.0 ([{⁸¹Br}M+H]⁺), ESI pos.

### d) 7-bromo-5-(2,6-difluorophenyl)-1,3-dihydro-1,4-benzodiazepin-2-one

In analogy to experiment of building block A e, (2-amino-5-bromo-phenyl)-(2,6-difluorophenyl)methanone was converted into the title compound (300 mg, 13 %) which was obtained as a yellow solid. MS: 351.0 ([{⁷⁹Br}M+H]⁺), 353.0 ([{⁸¹Br}M+H]⁺), ESI pos.

### e) 7-bromo-5-(2,6-difluorophenyl)-1,3-dihydro-1,4-benzodiazepine-2-thione

In analogy to experiment of example 30 a, 7-bromo-5-(2,6-difluorophenyl)-1,3-dihydro-1,4-benzodiazepin-2-one was converted into the title compound (310 mg, 92 %) which was obtained as a yellow solid. The crude was used as such in the following step without further characterization.

### f) 8-bromo-6-(2,6-difluorophenyl)-1-methyl-4H-[1,2,4]triazolo[4,3-a][1,4]benzodiazepine

In analogy to experiment of example 30 b, 7-bromo-5-(2,6-difluorophenyl)-1,3-dihydro-1,4-benzodiazepine-2-thione using acetohydrazide was converted into the title compound (5.1 mg, 4 %) which was obtained as a white solid. MS: 389.0 ([{⁷⁹Br}M+H]⁺), 391.0 ([{⁸¹Br}M+H]⁺), ESI pos.

### RE-B

### 6-(2,6-difluorophenyl)-1-methyl-8-(trifluoromethyl)-4H-[1,2,4]triazolo[4,3-a][1,4]benzodiazepine

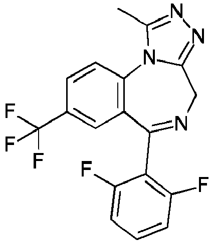

### a) (2-amino-5-iodo-phenyl)-(2,6-difluorophenyl)methanone

To a solution of (2-aminophenyl)-(2,6-difluorophenyl)methanone (1.6 g, 6.86 mmol) in DMF (15 mL) was added portionwise *N*-iodosuccinimide (1.62 g, 7.2 mmol). The reaction mixture was stirred at 20 °C for 16 h, before being diluted with water (20 mL). The mixture was extracted with ethyl acetate (3 × 20 mL), then the combined organic extracts were washed with brine (2 × 10 mL), dried (Na₂SO₄) and concentrated *in vacuo.* The residue was purified by flash column chromatography (petroleum ether / ethyl acetate, 10:1 to 5:1) to afford the title compound (2.0 g, 81 %) as a yellow solid. MS: 360.0 ([M+H]⁺), ESI pos.

### b) 5-(2,6-difluorophenyl)-7-iodo-1,3-dihydro-1,4-benzodiazepin-2-one

In analogy to experiment of building block A e, (2-amino-5-iodo-phenyl)-(2,6-difluorophenyl)methanone was converted into the title compound (650 mg, 12 %) which was obtained as a yellow solid. MS: 399.0 ([M+H]⁺), ESI pos.

### c) 5-(2,6-difluorophenyl)-7-iodo-1,3-dihydro-1,4-benzodiazepine-2-thione

In analogy to experiment of example 30 a, 5-(2,6-difluorophenyl)-7-iodo-1,3-dihydro-1,4-benzodiazepin-2-one was converted into the title compound (200 mg, 82 %) which was obtained as a yellow solid. MS: 414.9 ([M+H]⁺), ESI pos.

### d) 5-(2,6-difluorophenyl)-7-iodo-1,3-dihydro-1,4-benzodiazepin-2-one hydrazone

In analogy to experiment of example 64 a, 5-(2,6-difluorophenyl)-7-iodo-1,3-dihydro-1,4-benzodiazepine-2-thione was converted into the title compound (220 mg, 98 %) which was obtained as a yellow solid. MS: 413.0 ([M+H]⁺), ESI pos.

### e) 6-(2,6-difluorophenyl)-8-iodo-1-methyl-4H-[1,2,4]triazolo[4,3-a][1,4]benzodiazepine

In analogy to experiment of example 98 c, 5-(2,6-difluorophenyl)-7-iodo-1,3-dihydro-1,4-benzodiazepin-2-one hydrazone was converted into the title compound (150 mg, 64 %) which was obtained as a yellow solid. MS: 437.0 ([M+H]⁺), ESI pos.

### f) 6-(2,6-difluorophenyl)-1-methyl-8-(trifluoromethyl)-4H-[1,2,4]triazolo[4,3-a][1,4]benzodiazepine

In analogy to experiment of example 92 a, 6-(2,6-difluorophenyl)-8-iodo-1-methyl-4H-[1,2,4]triazolo[4,3-a][1,4]benzodiazepine was converted into the title compound (5 mg, 6 %) which was obtained as a white solid. MS: 379.0 ([M+H]⁺), ESI pos.

### RE-C

### 6-(2,6-difluorophenyl)-1-methyl-4H-[1,2,4]triazolo[4,3-a][1,4]benzodiazepine

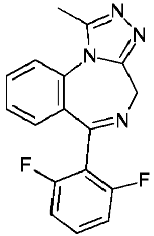

To a stirred solution of 8-bromo-6-(2,6-difluorophenyl)-1-methyl-4H-[1,2,4]triazolo[4,3-a][1,4]benzodiazepine (30 mg, 0.08 mmol) in methanol (0.5 mL) was added 10 wt.% Pd/C (2.5 mg, 2.4 µmol) and the resulting black suspension was purged by evacuation and then back filled with a stream of hydrogen (balloon) for three times. The mixture was stirred for 16 hours at room temperature under hydrogen atmosphere then filtered through a pad of dicalite. The filter cake was rinsed with methanol and the filtrate was concentrated *in vacuo.* The residue was purified by preparative HPLC (Phenomenex Gemini-NX C18, 0.1 % trifluoroacetic acid in water / acetonitrile) followed by preparative TLC (silica, dichloromethane / methanol, 20:1) to obtain the title compound (5 mg, 20 %) as a white solid. MS: 276.2 ([M+H]⁺), ESI pos.

### RE-G

### 7-chloro-6-(2,6-difluorophenyl)-1-methyl-4H-[1,2,4]triazolo[4,3-a][1,4]benzodiazepine

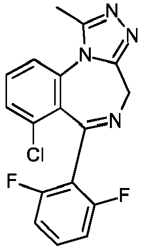

### a) 6-chloro-5-(2,6-difluorophenyl)-1,3-dihydro-1,4-benzodiazepin-2-one

In analogy to experiment of building block A e, (2-amino-6-chloro-phenyl)-(2,6-difluorophenyl)methanone was converted into the title compound (3.0 g, 44 %) which was obtained as a yellow solid. MS: 307.0 ([M+H]⁺), ESI pos.

### b) 6-chloro-5-(2,6-difluorophenyl)-1,3-dihydro-1,4-benzodiazepine-2-thione

In analogy to experiment of example 1 a, 6-chloro-5-(2,6-difluorophenyl)-1,3-dihydro-1,4-benzodiazepin-2-one was converted into the title compound (2.8 g, 89 %) which was obtained as a yellow solid. MS: 323.0 ([M+H]⁺), ESI pos.

### c) 6-chloro-5-(2,6-difluorophenyl)-1,3-dihydro-1,4-benzodiazepin-2-one hydrazone

In analogy to experiment of example 9 b, 6-chloro-5-(2,6-difluorophenyl)-1,3-dihydro-1,4-benzodiazepine-2-thione was converted into the title compound (220 mg, 98 %) which was used as a such without further purification. MS: 321.1 ([M+H]⁺), ESI pos.

### d) 7-chloro-6-(2,6-difluorophenyl)-1-methyl-4H-[1,2,4]triazolo[4,3-a][1,4]benzodiazepine

A solution of 6-chloro-5-(2,6-difluorophenyl)-1,3-dihydro-1,4-benzodiazepin-2-one hydrazone (220 mg, 0.690 mmol) and triethyl orthoacetate (556 mg, 3.43 mmol) in toluene (3 mL) was heated to 120 °C. After 1 h, the solvent was removed under reduced pressure and the resulting residue was partitioned between ethyl acetate (100 mL) and water (2 × 20 mL). The combined organic extracts were dried (Na₂SO4), filtered and concentrated *in vacuo.* The residue was purified directly by preparative HPLC (Boston Prime C18, 0.1 % trifluoroacetic acid in water / acetonitrile) and lyophilized to afford the title compound (73 mg, 31 %) as a white solid. MS: 344.9 ([M+H]⁺), ESI pos.

## Claims

1. A compound of formula (I) 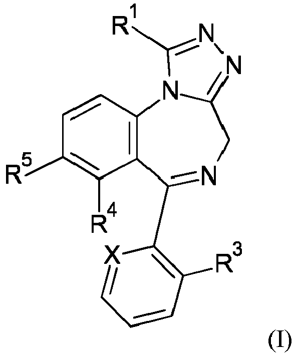 wherein
R¹ is selected from
i) H,
ii) C₁₋₆-alkyl,
iii) C₁₋₆-alkoxy,
iv) C₁₋₆-alkoxy-C₁₋₆-alkyl,
v) hydroxy,
vi) hydroxy-C₁₋₆-alkyl,
vii) C₃₋₈-cycloalkyl optionally substituted by R⁷, R⁸ and R⁹,
viii) amino-C₁₋₆-alkyl
ix) heteroaryl optionally substituted by R⁷, R⁸ and R⁹, and
x) heterocycloalkyl optionally substituted by R⁷, R⁸ and R⁹;
R³ is selected from
i) Cl, and
ii) F;
X is selected from
i) CR⁶, and
ii) N;
R⁶ is selected from
i) H,
ii) Cl, and
iii) F;
R⁴ is selected from
i) Br, and
ii) Cl;
R⁵ is selected from
i) C₁₋₆-alkyl,
ii) C₁₋₆-alkoxy,
iii) halogen,
iv) halo-C₁₋₆-alkyl,
v) cyano, and
vi) C₃₋₈-cycloalkyl;
R⁷, R⁸ and R⁹ are independently selected from
i) C₁₋₆-alkyl, and
ii) C₁₋₆-alkoxy;
or pharmaceutically acceptable salts.

2. A compound of formula (I) according to claim 1, wherein
R¹ is selected from
i) H,
ii) C₁₋₆-alkyl,
iii) C₁₋₆-alkoxy,
iv) C₁₋₆-alkoxy-C₁₋₆-alkyl,
v) hydroxy,
vi) hydroxy-C₁₋₆-alkyl,
vii) C₃₋₈-cycloalkyl optionally substituted by R⁷, R⁸ and R⁹,
viii) amino-C₁₋₆-alkyl
ix) pyrazolyl optionally substituted by R⁷, R⁸ and R⁹,
x) pyridinyl optionally substituted by R⁷, R⁸ and R⁹,
xi) pyrimidinyl optionally substituted by R⁷, R⁸ and R⁹,
xii) pyridazinyl optionally substituted by R⁷, R⁸ and R⁹, and
xiii) tetrahydropyranyl optionally substituted by R⁷, R⁸ and R⁹;
R³ is selected from
i) Cl, and
ii) F;
X is selected from
i) CR⁶, and
ii) N;
R⁶ is selected from
i) H,
ii) Cl, and
iii) F;
R⁴ is selected from
i) Br, and
ii) Cl;
R⁵ is selected from
i) C₁₋₆-alkyl,
ii) C₁₋₆-alkoxy,
iii) halogen,
iv) halo-C₁₋₆-alkyl,
v) C₃₋₈-cycloalkyl;
R⁷, R⁸ and R⁹ are independently selected from
i) C₁₋₆-alkyl, and
ii) C₁₋₆-alkoxy;
or pharmaceutically acceptable salts.

3. A compound of formula (I) according to claim 1 or 2, wherein
R¹ is selected from
i) H,
ii) C₁₋₆-alkyl,
iii) hydroxy,
iv) hydroxy-C₁₋₆-alkyl,
v) C₃₋₈-cycloalkyl optionally substituted by R⁷, R⁸ and R⁹,
vi) pyrazolyl optionally substituted by R⁷, R⁸ and R⁹,
vii) pyrimidinyl optionally substituted by R⁷, R⁸ and R⁹,
viii) pyridazinyl optionally substituted by R⁷, R⁸ and R⁹, and
ix) tetrahydropyranyl optionally substituted by R⁷, R⁸ and R⁹;
R³ is F;
X is selected from
i) CR⁶, and
ii) N;
R⁶ is selected from
i) H, and
ii) F;
R⁴ is selected from
i) Br, and
ii) Cl;
R⁵ is selected from
i) C₁₋₆-alkyl,
ii) halogen, and
iii) halo-C₁₋₆-alkyl;
R⁷, R⁸ and R⁹ are independently selected from C₁₋₆-alkyl,
or pharmaceutically acceptable salts.

4. A compound according to any one of claims 1 to 3, wherein
R¹ is C₁₋₆-alkyl;
R³ is F;
X is CR⁶;
R⁶ is F;
R⁴ is Cl;
R⁵ is halo-C₁₋₆-alkyl;
or pharmaceutically acceptable salts.

5. A compound according to any one of claims 1 to 4, selected from
8-bromo-7-chloro-6-(2,6-difluorophenyl)-1-methyl-4H-[1,2,4]triazolo[4,3-a][1,4]benzodiazepine;
8-bromo-7-chloro-1-cyclopropyl-6-(2-fluorophenyl)-4H-[1,2,4]triazolo[4,3-a][1,4]benzodiazepine;
8-bromo-7-chloro-6-(2-fluorophenyl)-1-pyridazin-3-yl-4H-[1,2,4]triazolo[4,3-a][1,4]benzodiazepine;
7,8-dichloro-6-(2-fluorophenyl)-1-methyl-4H-[1,2,4]triazolo[4,3-a][1,4]benzodiazepine;
7,8-dichloro-6-(2,6-difluorophenyl)-1-methyl-4H-[1,2,4]triazolo[4,3-a][1,4]benzodiazepine;
7-chloro-8-cyclopropyl-6-(2,6-difluorophenyl)-1-methyl-4H-[1,2,4]triazolo[4,3-a][1,4]benzodiazepine;
8-bromo-7-chloro-6-(2,6-difluorophenyl)-4H-[1,2,4]triazolo[4,3-a][1,4]benzodiazepine;
8-bromo-7-chloro-6-(2-fluorophenyl)-4H-[1,2,4]triazolo[4,3-a][1,4]benzodiazepine;
7,8-dichloro-6-(2,6-difluorophenyl)-2,4-dihydro-[1,2,4]triazolo[4,3-a][1,4]benzodiazepin-1-one;
7,8-dichloro-6-(2-fluorophenyl)-1-pyridazin-3-yl-4H-[1,2,4]triazolo[4,3-a][1,4]benzodiazepine;
7,8-dichloro-6-(2,6-difluorophenyl)-1-tetrahydropyran-4-yl-4H-[1,2,4]triazolo[4,3-a][1,4]benzodiazepine;
7,8-dichloro-6-(2,6-difluorophenyl)-1-pyrimidin-4-yl-4H-[1,2,4]triazolo[4,3-a][1,4]benzodiazepine;
8-bromo-7-chloro-6-(2,6-difluorophenyl)-2,4-dihydro-[1,2,4]triazolo[4,3-a][1,4]benzodiazepin-1-one;
7,8-dichloro-6-(2,6-difluorophenyl)-1-pyridazin-3-yl-4H-[1,2,4]triazolo[4,3-a][1,4]benzodiazepine;
7,8-dichloro-6-(2,6-difluorophenyl)-4H-[1,2,4]triazolo[4,3-a][1,4]benzodiazepine;
7-chloro-6-(2,6-difluorophenyl)-8-methyl-2,4-dihydro-[1,2,4]triazolo[4,3-a][1,4]benzodiazepin-1-one;
7-chloro-6-(2,6-difluorophenyl)-8-methyl-4H-[1,2,4]triazolo[4,3-a][1,4]benzodiazepine;
7,8-dichloro-6-(2-fluorophenyl)-1-pyrimidin-4-yl-4H-[1,2,4]triazolo[4,3-a][1,4]benzodiazepine;
7,8-dichloro-6-(2,6-difluorophenyl)-1-(2-methoxyethyl)-4H-[1,2,4]triazolo[4,3-a][1,4]benzodiazepine;
7-chloro-6-(2,6-difluorophenyl)-1,8-dimethyl-4H-[1,2,4]triazolo[4,3-a][1,4]benzodiazepine;
7-chloro-8-(1,1-difluoroethyl)-6-(2,6-difluorophenyl)-4H-[1,2,4]triazolo[4,3-a][1,4]benzodiazepine;
7-chloro-8-(1,1-difluoroethyl)-6-(2,6-difluorophenyl)-1-methyl-4H-[1,2,4]triazolo[4,3-a][1,4]benzodiazepine;
7-chloro-8-(difluoromethyl)-6-(2,6-difluorophenyl)-4H-[1,2,4]triazolo[4,3-a] [1,4]benzodiazepine;
8-bromo-7-chloro-6-(3-fluoro-2-pyridyl)-1-methyl-4H-[1,2,4]triazolo[4,3-a][1,4]benzodiazepine;
7-chloro-8-(difluoromethyl)-6-(2,6-difluorophenyl)-1-methyl-4H-[1,2,4]triazolo[4,3-a][1,4]benzodiazepine;
8-bromo-7-chloro-6-(2-fluorophenyl)-2,4-dihydro-[1,2,4]triazolo[4,3-a][1,4]benzodiazepin-1-one;
7-chloro-6-(2-fluorophenyl)-1-pyridazin-3-yl-8-(trifluoromethyl)-4H-[1,2,4]triazolo[4,3-a][1,4]benzodiazepine;
7,8-dichloro-6-(2,6-difluorophenyl)-1-(1-methylpyrazol-4-yl)-4H-[1,2,4]triazolo[4,3-a][1,4]benzodiazepine;
8-bromo-7-chloro-6-(3-fluoro-2-pyridyl)-4H-[1,2,4]triazolo[4,3-a][1,4]benzodiazepine;
[8-bromo-7-chloro-6-(2,6-difluorophenyl)-4H-[1,2,4]triazolo[4,3-a][1,4]benzodiazepin-1-yl]methanol;
7-chloro-6-(2,6-difluorophenyl)-8-iodo-4H-[1,2,4]triazolo[4,3-a][1,4]benzodiazepine;
[8-bromo-7-chloro-6-(2,6-difluorophenyl)-4H-[1,2,4]triazolo[4,3-a][1,4]benzodiazepin-1-yl]methanamine hydrochloride;
7-chloro-6-(2,6-difluorophenyl)-8-(trifluoromethyl)-4H-[1,2,4]triazolo[4,3-a][1,4]benzodiazepine;
7-chloro-6-(2,6-difluorophenyl)-1-pyridazin-3-yl-8-(trifluoromethyl)-4H-[1,2,4]triazolo[4,3-a][1,4]benzodiazepine;
7-chloro-6-(2,6-difluorophenyl)-8-iodo-1-pyridazin-3-yl-4H-[1,2,4]triazolo[4,3-a][1,4]benzodiazepine;
7-chloro-6-(2,6-difluorophenyl)-1-methyl-8-(trifluoromethyl)-4H-[1,2,4]triazolo[4,3-a][1,4]benzodiazepine;
7-chloro-6-(2,6-difluorophenyl)-8-iodo-1-methyl-4H-[1,2,4]triazolo[4,3-a][1,4]benzodiazepine;
7,8-dibromo-6-(2,6-difluorophenyl)-1-pyridazin-3-yl-4H-[1,2,4]triazolo[4,3-a][1,4]benzodiazepine;
7,8-dibromo-6-(2,6-difluorophenyl)-1-pyrimidin-4-yl-4H-[1,2,4]triazolo[4,3-a][1,4]benzodiazepine;
8-bromo-7-chloro-6-(3-chloro-2-pyridyl)-1-pyrimidin-4-yl-4H-[1,2,4]triazolo[4,3-a][1,4]benzodiazepine;
8-bromo-7-chloro-6-(3-fluoro-2-pyridyl)-1-pyridazin-3-yl-4H-[1,2,4]triazolo[4,3-a][1,4]benzodiazepine;
8-bromo-7-chloro-6-(3-fluoro-2-pyridyl)-1-pyrimidin-4-yl-4H-[1,2,4]triazolo[4,3-a][1,4]benzodiazepine;
8-bromo-7-chloro-6-(3-chloro-2-pyridyl)-1-pyridazin-3-yl-4H-[1,2,4]triazolo[4,3-a][1,4]benzodiazepine;
7-chloro-6-(2,6-difluorophenyl)-8-methyl-1-pyridazin-3-yl-4H-[1,2,4]triazolo[4,3-a][1,4]benzodiazepine;
7-chloro-6-(2,6-difluorophenyl)-8-methyl-1-pyrimidin-4-yl-4H-[1,2,4]triazolo[4,3-a][1,4]benzodiazepine;
8-bromo-7-chloro-6-(2-chloro-6-fluoro-phenyl)-1-methyl-4H-[1,2,4]triazolo[4,3-a][1,4]benzodiazepine;
7,8-dichloro-6-(3-fluoro-2-pyridyl)-1-pyridazin-3-yl-4H-[1,2,4]triazolo[4,3-a][1,4]benzodiazepine;
7,8-dichloro-6-(3-fluoro-2-pyridyl)-1-pyrimidin-4-yl-4H-[1,2,4]triazolo[4,3-a][1,4]benzodiazepine;
7-chloro-6-(2,6-difluorophenyl)-8-methoxy-1-pyridazin-3-yl-4H-[1,2,4]triazolo[4,3-a][1,4]benzodiazepine;
7,8-dichloro-6-(3-fluoro-2-pyridyl)-1-(1-methylpyrazol-4-yl)-4H-[1,2,4]triazolo[4,3-a][1,4]benzodiazepine;
7,8-dichloro-6-(3-fluoro-2-pyiidyl)-2,4-dihydro-[1,2,4]triazolo[4,3-a][1,4]benzodiazepin-1-one;
7,8-dichloro-6-(3-fluoro-2-pyridyl)-4H-[1,2,4]triazolo[4,3-a][1,4]benzodiazepine;
7-chloro-6-(2,6-difluorophenyl)-8-methoxy-1-pyrimidin-4-yl-4H-[1,2,4]triazolo[4,3-a][1,4]benzodiazepine;
7-chloro-6-(2,6-difluorophenyl)-8-methoxy-1-(3-pyridyl)-4H-[1,2,4]triazolo[4,3-a][1,4]benzodiazepine;
7,8-dichloro-1-cyclopropyl-6-(3-fluoro-2-pyridyl)-4H-[1,2,4]triazolo[4,3-a][1,4]benzodiazepine;
7-chloro-6-(3-fluoro-2-pyridyl)-1-pyrimidin-4-yl-8-(trifluoromethyl)-4H-[1,2,4]triazolo[4,3-a][1,4]benzodiazepine;
7-chloro-6-(3-fluoro-2-pyridyl)-1-(1-methylpyrazol-4-yl)-8-(trifluoromethyl)-4H-[1,2,4]triazolo[4,3-a][1,4]benzodiazepine;
7,8-dichloro-6-(3-fluoro-2-pyridyl)-1-methyl-4H-[1,2,4]triazolo[4,3-a][1,4]benzodiazepine;
7-chloro-6-(3-fluoro-2-pyridyl)-1-pyridazin-3-yl-8-(trifluoromethyl)-4H-[1,2,4]triazolo[4,3-a][1,4]benzodiazepine;
8-bromo-7-chloro-1-cyclopropyl-6-(3-fluoro-2-pyridyl)-4H-[1,2,4]triazolo[4,3-a][1,4]benzodiazepine;
7-chloro-6-(3-fluoro-2-pyridyl)-1-methyl-8-(trifluoromethyl)-4H-[1,2,4]triazolo[4,3-a][1,4]benzodiazepine;
7-chloro-1-cyclopropyl-6-(2,6-difluorophenyl)-8-methoxy-4H-[1,2,4]triazolo[4,3-a][1,4]benzodiazepine;
7,8-dichloro-6-(2,6-difluorophenyl)-1-methoxy-4H-[1,2,4]triazolo[4,3-a][1,4]benzodiazepine;
*trans*-7,8-dichloro-6-(2,6-difluorophenyl)-1-(3-methoxycyclobutyl)-4H-[1,2,4]tiiazolo[4,3-a][1,4]benzodiazepine;
8-bromo-7-chloro-6-(2,6-difluorophenyl)-1-pyridazin-3-yl-4H-[1,2,4]triazolo[4,3-a][1,4]benzodiazepine;
or pharmaceutically acceptable salts thereof.

6. A compound according to any one of claims 1 to 5, wherein the compound is selected from
7-chloro-6-(2,6-difluorophenyl)-1-methyl-8-(trifluoromethyl)-4H-[1,2,4]triazolo[4,3-a][1,4]benzodiazepine;
7-chloro-8-(1,1-difluoroethyl)-6-(2,6-difluorophenyl)-1-methyl-4H-[1,2,4]triazolo[4,3-a][1,4]benzodiazepine;
or pharmaceutically acceptable salts.

7. A compound according to any one of claims 1 to 6, wherein the compound is
7-chloro-6-(2,6-difluorophenyl)-1-methyl-8-(trifluoromethyl)-4H-[1,2,4]triazolo[4,3-a][1,4]benzodiazepine.
or pharmaceutically acceptable salts.

8. A process for the preparation of a compound of formula (I) according to any one of claims 1 to 7 comprising the reaction of a compound of formula (III) with a compound of formula (IV) wherein R¹, R³, R⁴ and R⁵ are as defined in anyone of the claims 1 to 4.

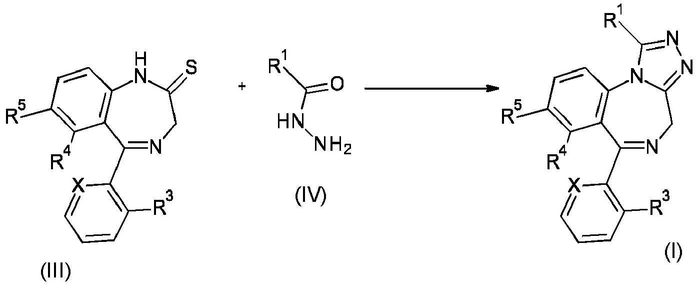

9. A compound according to any one of claims 1 to 7 for use as therapeutically active substance.

10. A pharmaceutical composition comprising a compound according to any one of claims 1 to 7 and a therapeutically inert carrier.

11. A compound according to any one of claims 1 to 7 for the treatment or prophylaxis of autism spectrum disorder, Rett syndrome, post-traumatic stress disorder and fragile-X disorder.
